# EUROPEAN PATENT SPECIFICATION

(11) **EP 4 294 210 B1**
(45) Date of publication and mention of the grant of the patent: **24.12.2025**
(21) Application number: 22706966.3
(22) Date of filing: 14.02.2022
(51) Int. Cl.: A23L 29/269, A23L 33/135, A23L 33/21, A61K 35/747

(54) **METHODS AND COMPOSITIONS USING COMBINATIONS OF LACTOBACILLUS MUCOSAE AND SOLUBLE DIETARY FIBER**
VERFAHREN UND ZUSAMMENSETZUNGEN UNTER VERWENDUNG VON KOMBINATIONEN VON LACTOBACILLUS MUCOSAE UND LÖSLICHEN NAHRUNGSFASERN
PROCÉDÉS ET COMPOSITIONS UTILISANT DES COMBINAISONS DE LACTOBACILLUS MUCOSAE ET DE FIBRES DIÉTÉTIQUES SOLUBLES

(30) Priority: 22.02.2021 US 202163152180 P; 27.05.2021 EP 21176330
(43) Date of publication of application: 27.12.2023
(73) Proprietor: Tate & Lyle Solutions USA LLC, Hoffman Estates, IL 60192 (US); University College Cork - National University of Ireland, Cork, Cork City (IE); Agriculture and Food Development Authority (TEAGASC), Co. Carlow (IE)
(72) Inventor: CAPLICE, Noel, Cork Western Road Cork City (IE); STANTON, Catherine, Carlow (IE); ROSS, Paul, Cork Western Road Cork City (IE); HERISSON, Florence, Cork Western Road Cork City (IE); KARNIK, Kavita, Hoffman Estates, IL 60192 (US); DE SOUZA, Mervyn, Hoffman Estates, IL 60192 (US); LAURIE, Ieva, Hoffman Estates, IL 60192 (US); CANENE-ADAMS, Kirstie, Hoffman Estates, IL 60192 (US)
(74) Representative: Marks & Clerk LLP
(86) International application number: PCT/US2022/016319
(87) International publication number: WO 2022/177856

(56) References cited:
- WO-A1-2021/081305
- US-A1- 2015 329 923
- US-A1- 2019 224 254
- US-A1- 2020 069 746
- KIM HYE-IN ET AL: "IL-10 Expression-Inducing Gut Bacteria Alleviate High-Fat Diet-Induced Obesity and Hyperlipidemia in Mice", JOURNAL OF MICROBIOLOGY AND BIOTECHNOLOGY, vol. 30, no. 4, 28 April 2020 (2020-04-28), pages 599 - 603, XP055845850, ISSN: 1017-7825, DOI: 10.4014/jmb.1912.12014
- KIM JEON-KYUNG ET AL: "Interplay Between Human Gut Bacteria Escherichia coli and Lactobacillus mucosae in the Occurrence of Neuropsychiatric Disorders in Mice", FRONTIERS IN IMMUNOLOGY, vol. 11, 1 January 2020 (2020-01-01), pages 27300273, XP055845848, DOI: 10.3389/fimmu.2020.00273
- ANONYMOUS: "Promitor Soluble Fibre and Synbiotics", 1 January 2021 (2021-01-01), XP055846166, Retrieved from the Internet <URL:https://www.tateandlyle.com/sites/default/files/2021-01/tlsynbioticsleavebehindfinal.pdf> [retrieved on 20210930]
- ANONYMOUS: "Tate & Lyle announces the publication of a gut health claim for PROMITOR Soluble Fibre by FSANZ | Tate & Lyle", 31 March 2020 (2020-03-31), XP055846172, Retrieved from the Internet <URL:https://www.tateandlyle.com/news/tate-lyle-announces-publication-gut-health-claim-promitor-soluble-fibre-fsanz> [retrieved on 20210930]
- ADELE COSTABILE ET AL: "Effects of Soluble Corn Fiber Alone or in Synbiotic Combination with Lactobacillus rhamnosus GG and the Pilus-Deficient Derivative GG-PB12 on Fecal Microbiota, Metabolism, and Markers of Immune Function: A Randomized, Double-Blind, Placebo-Controlled, Crossover Study in Healthy Elderly (Saimes Study", FRONTIERS IN IMMUNOLOGY, vol. 8, 12 December 2017 (2017-12-12), XP055738236, DOI: 10.3389/fimmu.2017.01443
- LONDON LIS E E ET AL: "Use ofLactobacillus mucosaeDPC 6426, an exopolysaccharide-producing strain, positively influences the techno-functional properties of yoghurt", INTERNATIONAL DAIRY JOURNAL, ELSEVIER APPLIED SCIENCE, BARKING, GB, vol. 40, 21 August 2014 (2014-08-21), pages 33 - 38, XP029095099, ISSN: 0958-6946, DOI: 10.1016/J.IDAIRYJ.2014.08.011
- SHAH BAKHT RAMIN ET AL: "Effects of prebiotic dietary fibers and probiotics on human health: With special focus on recent advancement in their encapsulated formulations", TRENDS IN FOOD SCIENCE AND TECHNOLOGY, ELSEVIER SCIENCE PUBLISHERS, GB, vol. 102, 23 June 2020 (2020-06-23), pages 178 - 192, XP086220169, ISSN: 0924-2244, [retrieved on 20200623], DOI: 10.1016/J.TIFS.2020.06.010

## Description

### CROSS-REFERENCE TO RELATED APPLICATIONS

This application claims the benefit of U.S. Provisional Patent Application no. 63/152180, filed February 22, 2021, and of European Patent Application no. 21176330.5, filed May 27, 2021.

### SEQUENCE LISTING STATEMENT

This application includes an electronically submitted Sequence Listing in text format. The text file contains a sequence listing entitled "21-0227-WO.txt" having a creation date of February 8, 2022 and a filesize of 1 kb. The Sequence Listing contained in this text file is part of the specification and is hereby incorporated by reference herein in its entirety.

### BACKGROUND OF THE DISCLOSURE

### 1. Field of the Disclosure

The present disclosure relates generally to compositions and methods of treatment utilizing soluble dietary fiber and *Lactobacillus mucosae* bacteria.

### 2. Technical Background

Metabolic syndrome (MetS; also known as syndrome X or dysmetabolic syndrome), a condition including obesity, dyslipidemia, hypertension and insulin resistance, is associated with and/or leads to numerous morbidities, including, e.g., non-alcoholic fatty liver disease and diastolic heart failure and cardiovascular disease. Metabolic syndrome and associated morbidities are some of the major global health challenges for 21st century scientists. In the past decade the gut microbiome has been shown to be a major player in alterations attributed to diet-induced dysregulation of lipid and glucose metabolism, cardinal features of the metabolic syndrome.

As such, there is a need for compositions and method for their use that can safely and effectively treat or prevent metabolic syndrome and/or its associated morbidities, as well as physiological markers and symptoms thereof.

US 2019/224254 A1 relates to the use of compositions to increase output of acetate and lactate while reducing pH and the levels of pathogenic bacteria and inflammation in the gut of a nursing infant mammal including humans. These compositions comprise one or more bacterial strains selected for their growth on mammalian milk oligosaccharides, a source of mammalian milk oligosaccharides, and, optionally, nutritive components required for the growth of that infant mammal.

US 2020/069746 A1 relates to a Bifidobacteria strain and its uses, to food products, feed products, dietary supplements and pharmaceutical formulations containing it, and to methods of making and using these compositions.

Kim Hye-In et al., "IL-10 Expression-Inducing Gut Bacteria Alleviate High-Fat Diet-Induced Obesity and Hyperlipidemia in Mice", J. Microbiol. Biotechnol. 2020; 30(4): 599-603, relates to effects of interleukin (IL)-10 expression-inducing bacteria Bifidobacterium adolescentis HP1, Lactobacillus mucosae HP2, and Weissella cibaria HP3 on high-fat diet (HFD)-induced obesity and liver steatosis in mice.

Kim Jeon-Kyung et al., "Interplay Between Human Gut Bacteria Escherichia coli and Lactobacillus mucosae in the Occurrence of Neuropsychiatric Disorders in Mice", Front. Immunol., 2020; 11: 27300273, relates to the roles of human gut bacteria in the occurrence of neuropsychiatric disorders.

### SUMMARY OF THE DISCLOSURE

One aspect of the disclosure is a composition that includes one or more strains of *Lactobacillus mucosae* and one or more soluble dietary fibers. The *L. mucosae* can be one of more specific strains, can express certain exopolysaccharides, be present in certain concentrations and dosages, be in certain physical forms, possess certain associated physical characteristics, and the like. One of the one or more strains of *Lactobacillus mucosae* is *L. mucosae* DPC6426. Similarly, the one or more soluble dietary fibers can possess certain physical characteristics and properties, be present in certain concentrations, and the like. The composition can also be in certain physical forms, especially in the form of a food or beverage product. Other forms are possible, such as powders, capsules and the like that include one or more pharmaceutically acceptable excipients, carriers, vehicles and/or diluents. The soluble dietary fiber has a linkage pattern comprising:
29-45% terminally-linked glucopyranosyl residues;
10-22% 6-linked glucopyranosyl residues;
13-27% 4-linked glucopyranosyl residues;
2-11% 3-linked glucopyranosyl residues;
3-13% 4,6-linked glucopyranosyl residues;
1-5% 3,6-linked glucopyranosyl residues;
0.5-4% 2,4-linked glucopyranosyl residues.

Another aspect of the disclosure is a method of reducing weight gain in a mammal, comprising administering to the mammal effective amounts of one or more strains of Lactobacillus mucosae and one or more soluble dietary fibers.

Another aspect of the disclosure is a method of treating or preventing cardiovascular disease in a mammal, comprising administering to the mammal effective amounts of one or more strains of Lactobacillus mucosae and one or more soluble dietary fibers.

Another aspect of the disclosure is a method of treating or preventing heart failure in a mammal, comprising administering to the mammal effective amounts of one or more strains of Lactobacillus mucosae and one or more soluble dietary fibers.

Another aspect of the disclosure is a method of reducing left ventricle wall thickness in a mammal, comprising administering to the mammal effective amounts of one or more strains of Lactobacillus mucosae and one or more soluble dietary fibers.

Another aspect of the disclosure is a method of reducing left atrial area in a mammal, comprising administering to the mammal effective amounts of one or more strains of Lactobacillus mucosae and one or more soluble dietary fibers.

Another aspect of the disclosure is a method of reducing systemic inflammation in a mammal, comprising administering to the mammal effective amounts of one or more strains of Lactobacillus mucosae and one or more soluble dietary fibers.

Another aspect of the disclosure is a method of reducing TNF-alpha in a mammal, comprising administering to the mammal effective amounts of one or more strains of Lactobacillus mucosae and one or more soluble dietary fibers.

Another aspect of the disclosure is a method of increasing FGF19 in a mammal, comprising administering to the mammal effective amounts of one or more strains of Lactobacillus mucosae and one or more soluble dietary fibers.

Another aspect of the disclosure is a method of reducing hsCRP in a mammal, comprising administering to the mammal effective amounts of one or more strains of Lactobacillus mucosae and one or more soluble dietary fibers.

Another aspect of the disclosure is a method of reducing IL1beta in a mammal, comprising administering to the mammal effective amounts of one or more strains of Lactobacillus mucosae and one or more soluble dietary fibers.

Another aspect of the disclosure is a method of reducing PAI1 in a mammal, comprising administering to the mammal effective amounts of one or more strains of Lactobacillus mucosae and one or more soluble dietary fibers.

Another aspect of the disclosure is a method of reducing IFN-gamma in a mammal, comprising administering to the mammal effective amounts of one or more strains of Lactobacillus mucosae and one or more soluble dietary fibers.

Another aspect of the disclosure is a method of reducing CD163 in a mammal, comprising administering to the mammal effective amounts of one or more strains of Lactobacillus mucosae and one or more soluble dietary fibers.

Another aspect of the disclosure is a method of reducing macrophage infiltration in a tissue of mammal, comprising administering to the mammal effective amounts of one or more strains of Lactobacillus mucosae and one or more soluble dietary fibers.

Another aspect of the disclosure is a method of increasing IL17A in a mammal, comprising administering to the mammal effective amounts of one or more strains of Lactobacillus mucosae and one or more soluble dietary fibers.

Another aspect of the disclosure is a method of reducing NF-Kb in a mammal, comprising administering to the mammal effective amounts of one or more strains of Lactobacillus mucosae and one or more soluble dietary fibers.

Another aspect of the disclosure is a method of reducing TLR4 in a mammal, comprising administering to the mammal effective amounts of one or more strains of Lactobacillus mucosae and one or more soluble dietary fibers.

Another aspect of the disclosure is a method of treating or preventing non-alcoholic fatty liver disease (NAFLD/NASH) in a mammal, comprising administering to the mammal effective amounts of one or more strains of Lactobacillus mucosae and one or more soluble dietary fibers.

Another aspect of the disclosure is a method of reducing liver fibrosis in a mammal, comprising administering to the mammal effective amounts of one or more strains of Lactobacillus mucosae and one or more soluble dietary fibers.

Another aspect of the disclosure is a method of reducing aSMA in a mammal, comprising administering to the mammal effective amounts of one or more strains of Lactobacillus mucosae and one or more soluble dietary fibers.

Another aspect of the disclosure is a method of reducing fat deposition in the liver in a mammal, comprising administering to the mammal effective amounts of one or more strains of Lactobacillus mucosae and one or more soluble dietary fibers.

Another aspect of the disclosure is a method of increasing the abundance of Porphyromonadaceae in the gut microbiome of a mammal, comprising administering to the mammal effective amounts of one or more strains of Lactobacillus mucosae and one or more soluble dietary fibers.

Another aspect of the disclosure is a method of treating or preventing metabolic syndrome in a mammal, comprising administering to the mammal effective amounts of one or more strains of Lactobacillus mucosae and one or more soluble dietary fibers.

The references to methods of treatment by therapy in this description are to be interpreted as references to compounds, pharmaceutical compositions and medicaments of the present invention for use in those methods.

The invention is restricted to the subject-matter of the claims.

Notably, in each of the aspects above pertaining to methods, the *L. mucosae* can be one or more specific strains, including *L. mucosae* DPC6426, can express certain exopolysaccharides, be present in certain concentrations, amounts and/or dosages, be in certain physical forms, possess certain associated physical characteristics, and the like. Likewise, the one or more soluble dietary fibers can possess certain physical characteristics and properties, be present in certain concentrations, amounts and/or dosages, and the like. The method-related aspects can also include administering the effective amount of one or more strains of *Lactobacillus mucosae* and one or more soluble dietary fibers as a composition, e.g., as of the first aspect, above. The compositions can be administered via certain methods, e.g., orally, and administered at certain frequencies over certain lengths of time to, humans and non-human animals. The compositions can also be administered as part of a functional food or beverage (e.g., a medical food or beverage).

Another aspect of the disclosure is a functional food or beverage product (e.g., a medical food or beverage) that includes an effective amount of a composition, e.g., as of the first aspect, above.

Another aspect of the disclosure is an effective amount of one or more strains of *Lactobacillus mucosae* and one or more soluble dietary fibers for use in one or more methods of the disclosure as described herein as aspects of the disclosure. The effective amount of one or more strains of *Lactobacillus mucosae* and one or more soluble dietary fibers may be formulated as one or more compositions. The one or more strains of *Lactobacillus mucosae* and the one or more soluble dietary fibers are those as described herein. It will be appreciated that embodiments or features disclosed in relation to said methods of the current disclosure also apply to said use. Additional aspects of the disclosure will be evident from the disclosure herein.

### BRIEF DESCRIPTION OF THE DRAWINGS

Figures 1A and 1B present graphs that demonstrate body weight reduction utilizing diet supplementation with compositions and methods of the disclosure.
Figures 2A, 2B, 2C, 2D, 2E, 2F and 2G present graphs showing the effects of diet supplementation with compositions of the disclosure on cardiac structural and hemodynamic parameters.
Figures 3A and 3B present graphs showing the effects of diet supplementation with compositions of the disclosure on markers of non-alcoholic fatty liver disease (NAFLD) or nonalcoholic steatohepatitis (NASH).
Figures 4A and 4B present graphs showing the effects of diet supplementation with compositions of the disclosure on the expression of inflammation markers in the terminal ileum.
Figures 5 presents a graph showing the effects of diet supplementation with compositions of the disclosure on the macrophage marker CD163 in the terminal ileum crypts and glands assessed by immunostaining and compared between groups.
Figure 6 presents graphs showing the effects of diet supplementation with compositions of the disclosure on the concentrations of systemic inflammation markers.
Figures 7A and 7B is a series of graphs illustrating the expression of inflammation markers in the left atrium in response to diet.
Figure 8 is a series of graphs illustrating lipids profile of metabolic syndrome pigs in response to different diet supplementation.
Figures 9A, 9B, 9C, 10 and 11 are a series of graphs illustrating the concentration of fasting glucose and insulin level and after a glucose meal in animals at baseline and after diet supplementation over 10-12 weeks.
Figures 12A, 12B, 12C and 12D are graphs illustrating terminal ileum membrane integrity of metabolic syndrome pigs in response to different diet supplementation.
Figures 13-15 are a series of graphs illustrating postprandial plasma concentration of cholecystokinin (CCK), peptide YY (PYY) and glucagon-like peptide-1 (GLP-1) after a glucose meal in animals in response to different diet supplementation over 12 weeks.
Figure 16 presents a series of graphs showing the concentration of short-chain fatty acids (SCFAs) in serum of metabolic syndrome pigs in response to different diet supplementation.
FIGS. 17A, 17B, 17C, 17D, 17E, 17F and 17G are a series of graphs illustrating the alpha diversity levels and kinetics in response to different diet supplementation in a pig model for metabolic syndrome.
FIGS. 18A, 1B, 18C, 18D, 18E, 18F, 18G are a series of diagrams showing the temporal shift of Metabolic Syndrome pig fecal microbiome beta diversity in response to different diet supplementation.
FIGS. 19-26 are a series of diagrams that illustrate the relative abundance of specified bacterial Phyla in pig feces at Baseline, T2, T6 and T12 weeks on specified diets.
FIGS. 27-29 are a series of graphs that quantify the results of FIGS. 19-26 at T2, T6 and T12, respectively.

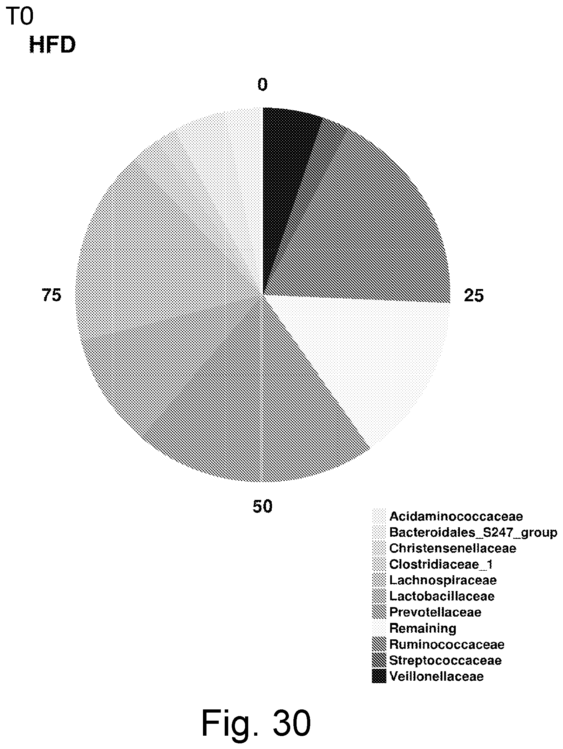

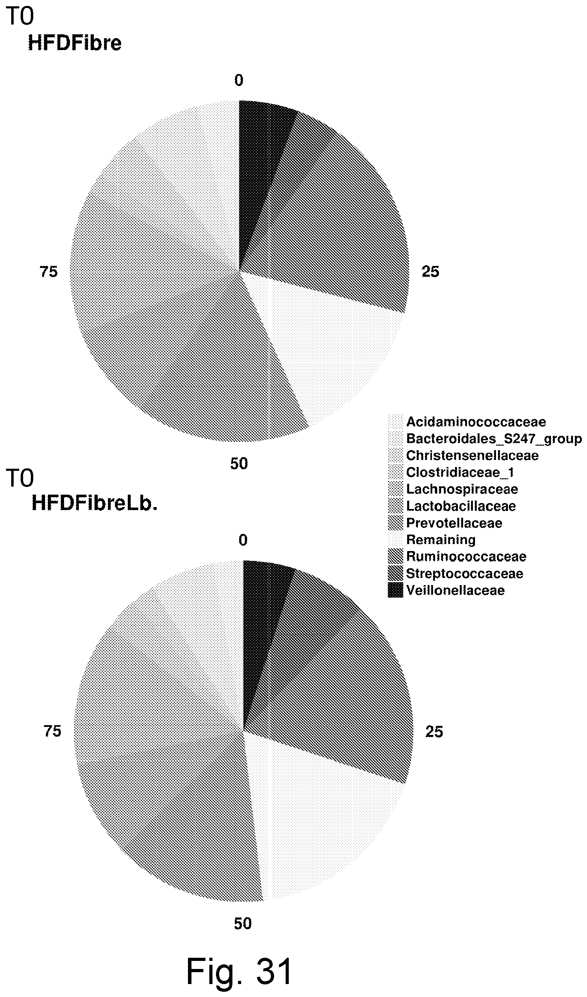

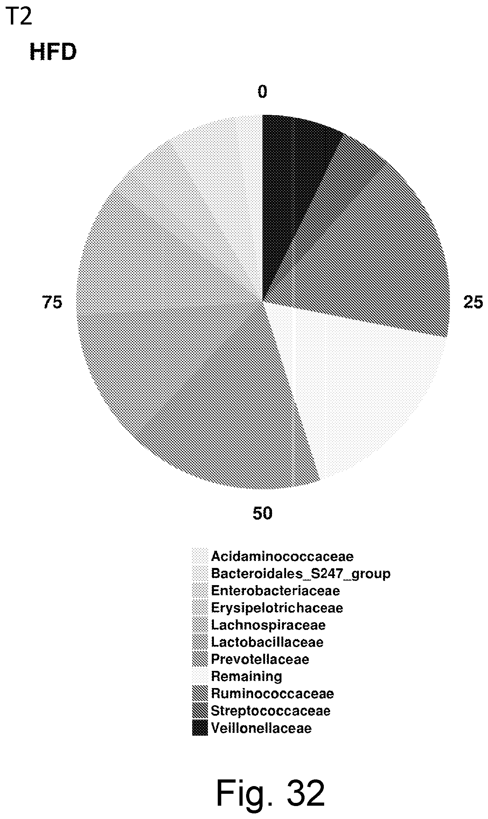

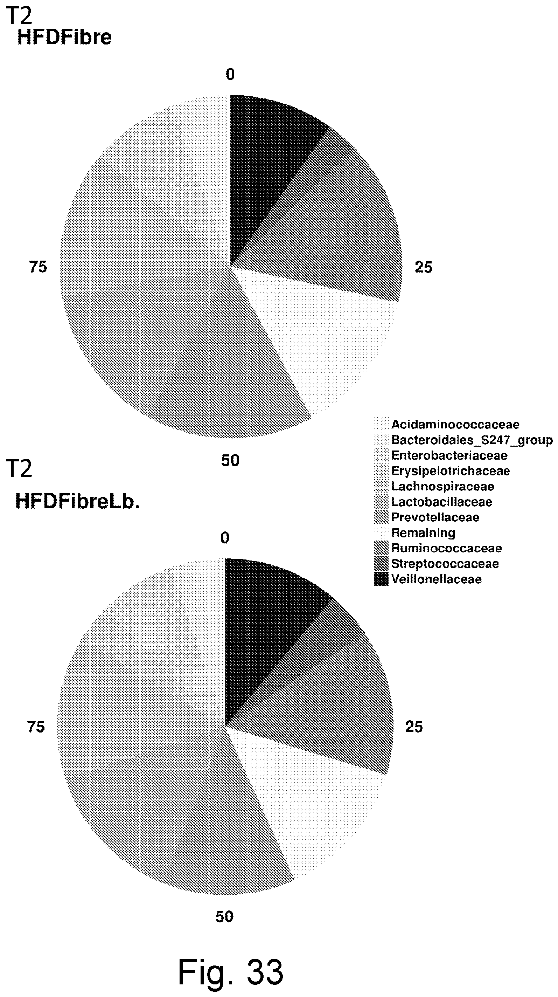

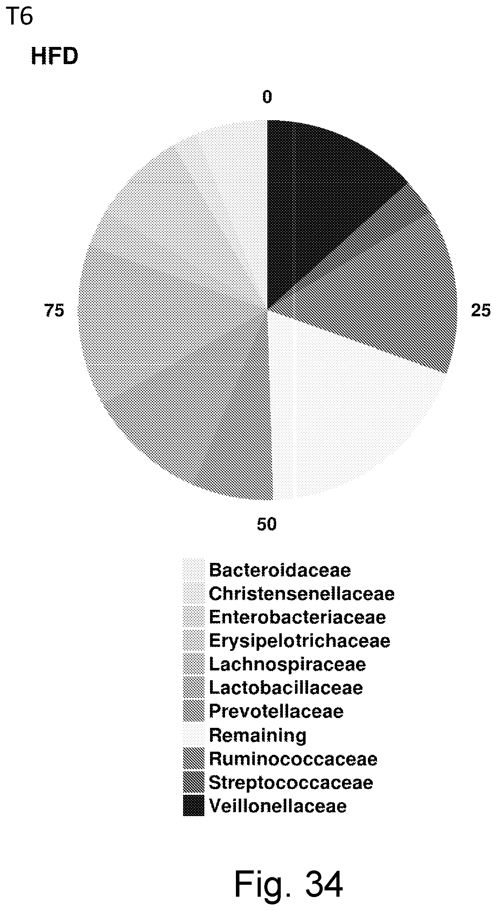

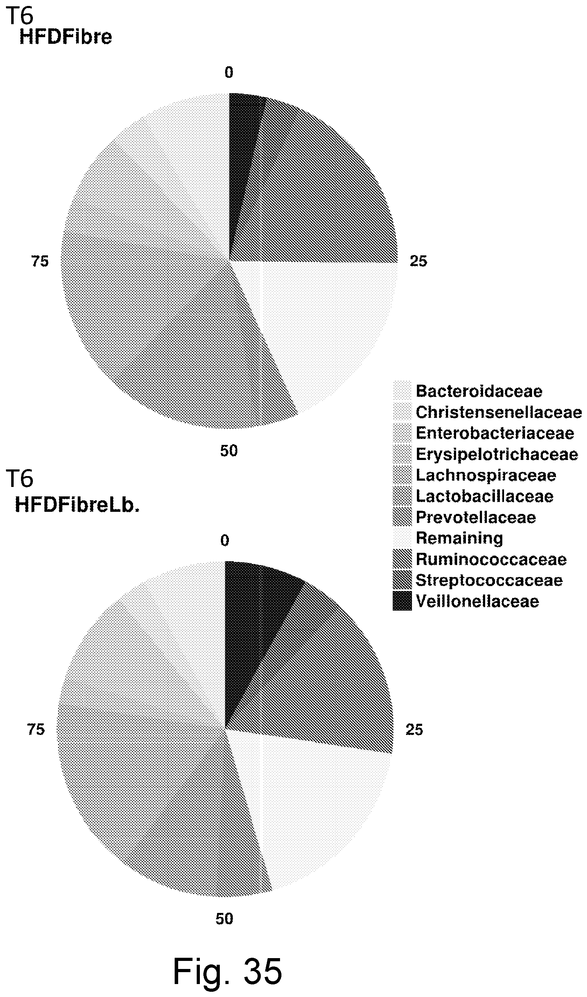

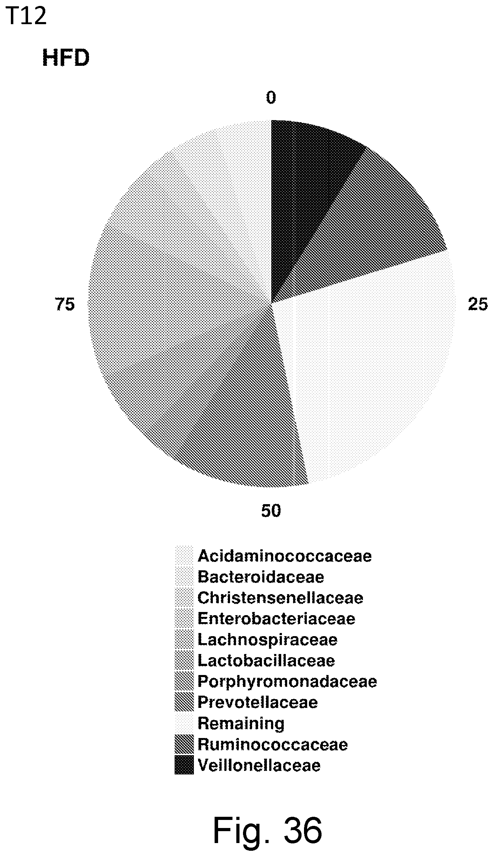

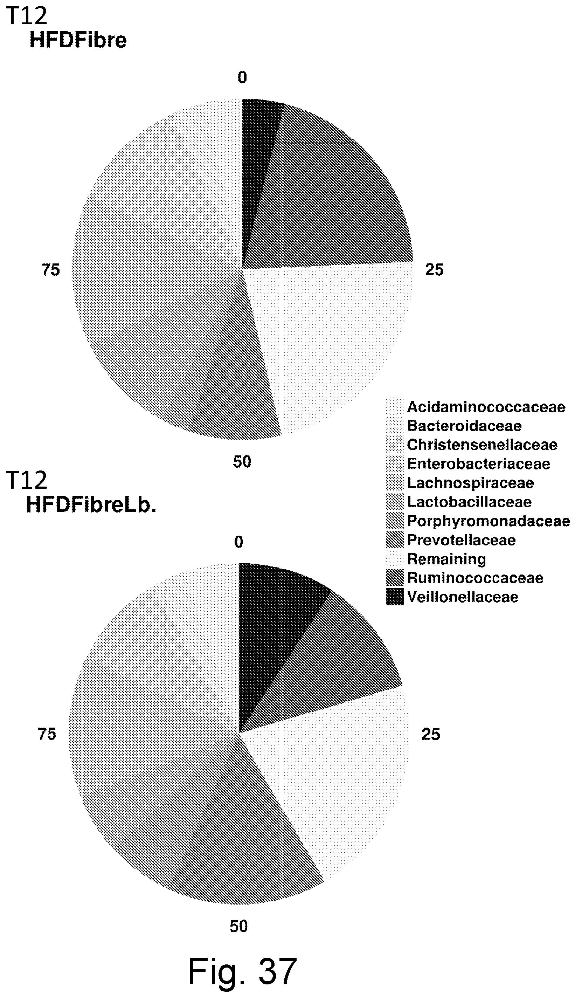

FIGS. 30-37 are a series of diagrams that illustrate the relative abundance of specified bacterial Families in pig feces at Baseline, T2, T6 and T12 weeks on specified diets.
FIGS. 38-40 are a series of graphs that quantify the results of FIGS. 30-37 at T2, T6 and T12, respectively.

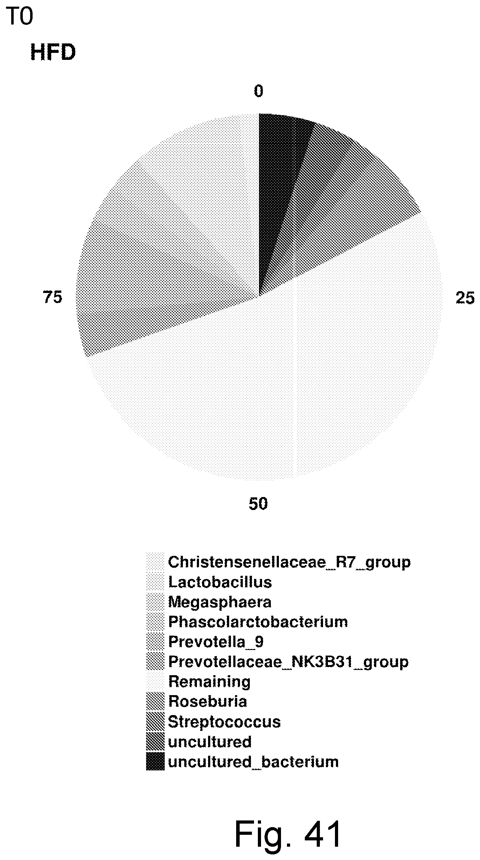

FIGS. 41-48 are a series of diagrams that illustrate the relative abundance of specified bacterial Genera in pig feces at Baseline, T2, T6 and T12 weeks on specified diets.

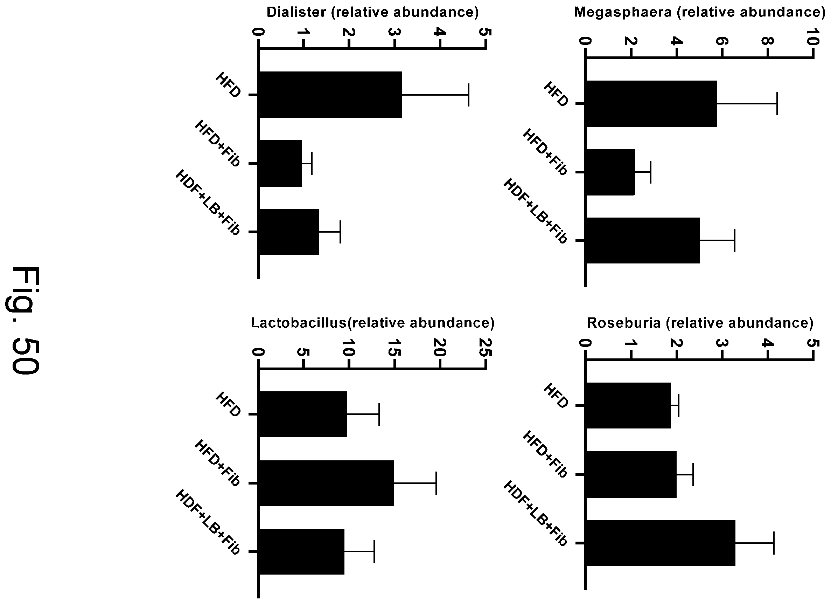

FIGS. 49-51 are a series of graphs that quantify the results of FIGS. 41-48 at T2, T6 and T12, respectively.
FIG. 52 is a chart that illustrates the relative abundance of Phyla, Families and Genera significantly increased or decreased (vertical arrow) or unchanged (horizontal arrow) in response to diet in the development of Metabolic Syndrome.

### DETAILED DESCRIPTION

The invention is restricted to the subject-matter of the claims.

In various studies of combinations of soluble dietary fibers and *Lactobacillus mucosae* bacteria, including *L. mucosae* DPC 6426, the present inventors unexpectedly determined that certain combinations caused unexpected beneficial effects on physiological and anatomical parameters related to metabolic syndrome and associated diseases, disorders and morbidities, including synergistic effects. Accordingly, the present inventors discovered that certain combinations of soluble dietary fibers and *L. mucosae* as described herein can provide a means of treating or preventing one or more physiological and anatomical parameters of metabolic syndrome and associated diseases, disorders and morbidities, and/or positively affecting an abnormal abundance of one or more biomarkers associated with such diseases, disorders and comorbidities.

Accordingly, one aspect of the disclosure is a composition that comprises one or more strains of *Lactobacillus mucosae* and one or more soluble dietary fibers.

In certain embodiments, a composition as otherwise described herein comprises one or more strains of *Lactobacillus* mucosae. *"Lactobacillus mucosae"* (or *L. mucosae*) means any strain of bacterium of the genus *Lactobacillus* and species *mucosae,* including, without limitation, *L. mucosae* DPC6426 (in each case herein also comprising name variants such as "DPC-6426" or "DPC 6426" for this example), *L. mucosae* CNPC006, *L. mucosae* CNPC007, *L. mucosae* CNPC009, *L. mucosae* A13, *L. mucosae* LM1, *L. mucosae* S5, *L*. *mucosae* S14, *L. mucosae* S15, *L. mucosae* S17, *L. mucosae* S32, *L. mucosae* 1028, *L*. *mucosae* 1031, *L. mucosae* 1987, *L. mucosae* ME-340, *L. mucosae* Strain D, *L. mucosae* CNCM 1-4429, *L. mucosae* DSM 13345, *L. mucosae* AGR63, *L. mucosae* DPC 6420, *L*. *mucosae* KHPC15, *L. mucosae* CCF3706, *L. mucosae* CCF3464, and *L. mucosae* MGYG-HGUT-02319. Particular embodiments comprise at least *L. mucosae* DPC6426, and in certain embodiments *L. mucosae* DPC6246 is substantially the only *L. mucosae* present. For example, in certain embodiments, at least 90% (e.g., at least 95%) of the *L. mucosae* present is *L. mucosae* DPC6426.

Some microorganisms, including certain *Lactobacillus mucosae* strains are known to produce exopolysaccharides (EPS; high molecular weight polymers that are composed of sugar residues and expressed by bacteria), the biological functions of which are diverse, including contributing to cell protection and survival, cell adherence, protecting against environmental stresses, and playing a role in pathogenesis and symbiosis. Health benefits have also been identified, including anti-tumoral, antiulcer, and immunostimulatory properties.

In certain embodiments as otherwise described herein, at least one of the one or more strains of *L. mucosae* in the compositions of such embodiments expresses an exopolysaccharide comprising monosaccharide residues xylose, fucose, mannose, glucose, galactose, N-acetylglucosamine, and N-acetylmannosamine.

In certain embodiments as otherwise described herein, the monosaccharide residues that comprise the exopolysaccharide, i.e., xylose, fucose, mannose, glucose, galactose, N-acetylglucosamine, and N-acetylmannosamine comprise a ratio between the number of xylose, fucose, mannose, glucose, galactose, N-acetylglucosamine, and N-acetylmannosamine monosaccharide residues of about 1.3:0.5:10.0:4.8:3.3:1.5:0.2.

A *Lactobacillus mucosae* DPC 6426 strain has been deposited under the terms of the Budapest Treaty on the International Recognition of the Deposit of Microorganisms for the Purpose of Patent Procedure with the National Collection of Industrial and Marine Bacteria Ltd. (NCIMB; Ferguson Building, Craibstone Estate, Blackburn, Aberdeen AB21 9YA UK) under the Accession No. NCIMB 42015 on 23 Jul. 2012 (deposited in the name of Teagasc, Moorepark Food Research Centre, Fermoy, Co. Cork, Ireland). Also suitable for use are variants of *L. mucosae* DPC 6426, wherein the variants are characterized in that they belong to the species *Lactobacillus mucosae,* and they express an EPS, typically the same EPS as expressed by *Lactobacillus mucosae* DPC 6426 or an EPS characterised in that it is comprised of monosaccharide residues xylose, fucose, mannose, glucose, galactose, N-acetylglucosamine, and N-acetylmannosamine in the following ratios: 1.1-1.4:0.4-0.6:9.0-10.0:4.7-4.9:3.2.3.4:1.4-1.6:0.1-0.3, for example approximately 1.3:0.5:10.0:4.8:3.3:1.5:0.2. Preferably, the variant is further characterised in that it has cardioprotective properties on a subject when administered to the subject, typically by dietary administration.

Certain *L. mucosae* strains suitable for use in the combinations, compositions and methods described herein are described in U.S. Patent Application Publication no. 2015/0329923.

The one or more strains of *L. mucosae,* whether included in a composition together with a soluble dietary fiber according to certain embodiments as otherwise described herein; or used as a separate dosage from soluble dietary fiber in a method as described herein (i.e., concurrently or sequentially but separately from the soluble dietary fiber), can be provided in a wide variety of concentrations. For example, in certain embodiments as otherwise described herein, a composition comprising *L. mucosae* and soluble dietary fiber or a dosage of *L. mucosae* as otherwise described herein include *L. mucosae* microorganisms in an amount in the range of 10⁵ to 10¹⁵ colony forming units (cfu)/g, e.g., in the range of 10⁷ to 10¹⁵ cfu/g, or 10⁹ to 10¹⁵ cfu/g, or 10⁵ to 10¹³ cfu/g, or 10⁷ to 10¹³ cfu/g, or 10⁹ to 10¹³ cfu/g, or 10⁵ to 10¹¹ cfu/g, or 10⁷ to 10¹¹ cfu/g, or 10⁹ to 10¹¹ cfu/g. In certain embodiments as otherwise described herein, a composition comprising *L. mucosae* and soluble dietary fiber or a dosage of *L. mucosae* as otherwise described herein include *L*. *mucosae* microorganisms in an amount in the range of 10⁶ to 10¹⁵ colony forming units (cfu)/g, e.g., in the range of 10⁹ to 10¹⁵ cfu/g, or 10¹² to 10¹⁵ cfu/g. In certain embodiments as otherwise described herein, a composition comprising *L. mucosae* and soluble dietary fiber or a dosage of *L. mucosae* as otherwise described herein include *L. mucosae* microorganisms in an amount in the range of 10⁹ to 10¹⁵ colony forming units (cfu)/g, e.g., in the range of 10¹² to 10¹⁵ cfu/g.

*Lactobacillus mucosae* variants are also contemplated for embodiments as otherwise described herein, the term "variant" should be understood to mean a strain of bacteria that can be distinguished from a canonical *L. mucosae* strain, for example, in terms of a genetic or phenotypic trait, belonging to the species *Lactobacillus mucosae,* and preferably expressing an exopolysaccharide that is substantially the same as the EPS as otherwise described herein. The term "variant" is intended to encompass strains that have been genetically modified, for example to strains that are engineered to express an exogenous gene, or strains that are engineered to silence an endogenous gene. The term is also intended to encompass variant strains obtained by serial passage of the strain(s) as otherwise described herein.

Certain embodiments, as otherwise described herein, comprise one of more soluble dietary fibers. As used herein, "soluble dietary fiber" or "soluble dietary fibers" are a composition that is formed chiefly of dextrose oligomers (i.e., oligosaccharide content having at least 98% dextrose monomeric residues, and degree of polymerization in the range of 2-30) optionally together with dextrose (i.e., monosaccharide). As used herein, the total amount of dextrose oligomers and dextrose in a soluble dietary fiber is at least 95%, desirably at least 98%. The total amount of oligomeric sugar alcohol residues in the soluble dietary fiber is no more than 2%, e.g., no more than 1% or even no more than 0.5%. Thus, the soluble dietary fiber is not a "polydextrose" as the term is commonly understood.

Soluble dietary fibers can have a variety of molecular weights (consistent with remaining substantially water-soluble). As used herein, in many end uses, a relative viscosity is desired, and so it can be desirable for a soluble dietary fiber to have a relatively low molecular weight. In certain embodiments as otherwise described herein, the fiber has a weight average molecular weight in the range of 1000 g/mol to 2500 g/mol. For example, in certain such embodiments, the soluble dietary fiber has a weight average molecular weight in the range of 1000 g/mol to 2000 g/mol. In various additional embodiments as otherwise described herein, the soluble dietary fiber has a weight average molecular weight in the range of 1000 to 2250 g/mol, or 1000 g/mol to 1800 g/mol, or 1000 g/mol to 1600 g/mol, or 1200 to 2500 g/mol, or 1200 to 2250 g/mol, or 1200 g/mol to 2000 g/mol, or 1200 g/mol to 1800 g/mol, or 1200 g/mol to 1600 g/mol, or 1400 to 2500 g/mol, or 1400 to 2250 g/mol, or 1400 g/mol to 2000 g/mol, or 1400 g/mol to 1800 g/mol, or 1600 to 2500 g/mol, or 1600 to 2250 g/mol, or 1600 g/mol to 2000 g/mol, or 1800 g/mol to 2500 g/mol, or 1800 to 2250 g/mol, or 2000 g/mol to 2500 g/mol. As used herein, molecular weight of soluble dietary fibers are determined by gel permeation chromatography, using narrow standard pullulans as standards.

In certain embodiments as otherwise described herein, the soluble dietary fiber comprises certain amounts of mono- and/or disaccharides. This will typically be chiefly dextrose and dextrose disaccharides such as maltose and isomaltose, but the person of ordinary skill in the art will appreciate that minor amounts of other mono- and/or disaccharides may be present. In certain embodiments as otherwise described herein, the total amount of mono- and disaccharides is up to 25 wt% on a dry solids basis, e.g., up to 20 wt%, or up to 15 wt%. In certain embodiments, a soluble dietary fiber as otherwise described herein can have a relatively lower amount of mono- and disaccharides, e.g., no more than 15 wt%, no more than 10 wt%. In some embodiments, the soluble dietary fiber has no more than 5 wt% total mono- and disaccharides.

In certain embodiments as otherwise described herein, there is a relatively significant amount of mono- and/or disaccharides in the soluble dietary fiber. For example, in certain embodiments as otherwise described herein, the total amount of mono- and disaccharides is in the range of 3 wt% to 25 wt%. For example, in certain such embodiments, the total amount of mono- and disaccharides is in the range of 3 wt% to 20 wt%, or 5 wt% to 20 wt%, or 10 wt% to 20 wt%, or 15 wt% to 25 wt% on a dry solids basis.

In certain embodiments as otherwise described herein, one of the one or more soluble dietary fibers has a linkage pattern comprising:
25-45% terminally-linked glucopyranosyl residues;
10-22% 6-linked glucopyranosyl residues;
13-32% 4-linked glucopyranosyl residues;
2-11% 3-linked glucopyranosyl residues;
3-13% 4,6-linked glucopyranosyl residues;
1-5% 3,6-linked glucopyranosyl residues;
0.5-4% 2,4-linked glucopyranosyl residues.

The soluble dietary fiber has a linkage pattern comprising:
29-45% terminally-linked glucopyranosyl residues;
10-22% 6-linked glucopyranosyl residues;
13-27% 4-linked glucopyranosyl residues;
2-11% 3-linked glucopyranosyl residues;
3-13% 4,6-linked glucopyranosyl residues;
1-5% 3,6-linked glucopyranosyl residues;
0.5-4% 2,4-linked glucopyranosyl residues.

Linkage patterns are determined using the method of York et al., Methods Enzymol. 116, 3-40 (1985). The method proceeds by permethylating the oligosaccharide, followed by quantitative hydrolysis and acetylation. This results in monomeric species that are acetylated where they were bound to other residues in the oligosaccharide, and methylated everywhere else. The mixture of the monomeric species can be analyzed by gas chromatography to determine relative amounts of different types of linked monomers. All linkages quantified in this disclosure can be determined using this method.

As used herein, a terminal residue is a residue that has only a single linkage to the rest of the oligosaccharide of which it is a part. A 1,X-linked residue is one that is linked to the rest of the oligosaccharide of which it is a part through its 1-position and its X-position (i.e., to two other residues). A 1,X,Y-linked residue is one that is linked to the rest of the oligosaccharide of which it is a part through its 1-position, its X-position, and its Y-position (i.e., to three other residues). As used herein, the term "oligosaccharide" includes disaccharides, trisaccharides, and oligomers of higher degrees of polymerization up to 30. Linkage percentages are provided as the fraction of the total number of terminally-linked residues, di-linked residues and tri-linked residues.

The soluble dietary fiber materials of the disclosure can have a variety of fiber contents. "Fiber content" as the term is used herein is the amount of fiber by weight on a dry solids basis as measured by AOAC 2001.03. As used herein, a soluble dietary fiber has a fiber content of at least 60%. In certain embodiments as otherwise described herein, the soluble dietary fiber has a fiber content of at least 65%. For example, in certain embodiments, a soluble dietary fiber as otherwise described has a fiber content of at least 70%, e.g., at least 75%, at least 80%, or even at least 85%. For example, in various embodiments as otherwise described herein, a soluble dietary fiber has a fiber content in the range of 70% to 95% fiber, e.g., in the range of 70% to 90%, or 70% to 85%, or 70% to 80%. In other embodiments as otherwise described herein, a soluble dietary fiber has a fiber content in the range of 65 to 85% fiber, e.g., in the range of 65% to 80%, or in the range of 65% to 75%.

The soluble dietary fibers of the disclosure can be made in a variety of ways. For example, in certain embodiments, the soluble dietary fiber can be made by a process that includes providing a saccharide feed comprising at least 95% (e.g., at least 97%, at least 98%, or at least 99%) of dextrose and/or dextrose oligomers on a dry solids basis; reacting the saccharide feed in the presence of water and in the substantial absence of sugar alcohols at a total solids concentration of at least 80 wt% and a temperature of at least 120 °C with at least one acid catalyst that accelerates the rate of cleavage and formation of glucosyl bonds for a time sufficient to produce a product composition having a fiber content of at least 60%.

The saccharide feed can be provided by a variety of materials. In certain embodiments, it has a significant content of linear dextrose oligomers, i.e., oligomers in which dextrose residues are bonded only by 1,4-alpha linkages. In certain embodiments as otherwise described herein, the saccharide feed comprising at least 95% (e.g., at least 97%, at least 98%, or at least 99%) of dextrose and/or linear dextrose oligomers on a dry solids basis. Starch hydrolysate can be suitably used as feed compositions, e.g., having dextrose equivalence values in the range of 26-95, e.g., 26-50, 40-70, or 60-95. These can have varying amounts of dextrose, maltose and higher dextrose oligomers. A variety of starch sources are suitable, e.g., corn, rice, wheat, tapioca and potato. Higher purity dextrose (e.g., at least 97%, at least 98% or at least 99%) is also suitable as a feed composition.

The saccharide feed is reacted in the presence of water at a total solids concentration of at least 80%. The use of high solids concentration will drive the reaction towards condensation to build to a desired molecular weight (e.g., as described above) and to provide condensation of dextrose residues with one another. Notably, this condensation can provide a variety of different types of bonds, including non-1,4-alpha glucosyl bonds that are not so easily digested by the human digestive system. However, it is desirable to have some water present to hydrolyze a proportion of existing 1,4-alpha bonds in the feed (e.g., in linear dextrose oligomers). The person of ordinary skill in the art will select a solids content in conjunction with other process conditions to provide a desired soluble dietary fiber. For example, in certain embodiments as otherwise described herein, the reaction is performed at a total solids concentration of at least 85%, or even at least 90%. In various embodiments as otherwise described herein, the reaction is performed at a total solids concentration the range of 80 wt% to 99 wt%, e.g., 85-99 wt%, or 90-99 wt%, or 93-99 wt%, or 80-98 wt%, or 85-98 wt%, or 90-98 wt%, or 93-98 wt%, or 80-96 wt%, or 85-96 wt%, or 90-96 wt%, or 93-96 wt%.

Of course, a saccharide feed can be provided at a relatively lower solids content (e.g., a pumpable syrup at 60-70%), then concentrated under the reaction conditions to the ultimate desired solids content for the reaction. The reaction can be performed while allowing escape of water (e.g., passively by venting or actively under vacuum), to not only concentrate a lower solids feed but also to drive the condensation by removal of water. It can be desirable to add portions of water to maintain the solids content at a desirable level (e.g., 93-98 wt% or any other amount described above) as water is removed from the system.

As condensation will produce water, the reaction can be performed while allowing water to escape the system, e.g., passively through venting the system, or actively using vacuum pumping.

Notably, the reaction is performed in the substantial absence of sugar alcohols, as is consistent with the fact that the soluble dietary fibers of the disclosure are not "polydextrose." As used herein, a "substantial absence of sugar alcohols" means no more than 0.5 wt% of the feed. Desirably, the reaction is performed with no more than trace amounts of sugar alcohol present.

The reaction is performed at a temperature of at least 120 °C. The person of ordinary skill in the art will select a solids content in conjunction with other process conditions to provide a desired soluble dietary fiber. For example, in certain embodiments as otherwise described herein, the reaction is performed at a temperature of at least 130 °C, at least 140 °C, or even at least 149 °C. In various embodiments as otherwise described herein, the reaction is performed at a temperature in the range of 120 °C to 190 °C, e.g., 120-180 °C, or 120-170 °C, or 130-190 °C, or 130-180 °C, or 130-170 °C, or 140-190 °C, or 140-180 °C, or 140-170 °C, or 140-190 °C, or 140-180 °C, or 140-170 °C.

A variety of acid catalysts are known to catalyze the formation and hydrolysis of glucosyl bonds. For example, in certain embodiments, at least one acid catalyst is selected from hydrochloric acid, phosphoric acid and sulfuric acid. In one embodiment, a combination of hydrochloric acid and phosphoric acid is used. Of course, other acid catalysts may also be suitable, e.g., citric acid, acetic acid, and malic acid. However, in certain embodiments, no carboxylic acid catalyst is used. In certain embodiments, at least part of the acid catalyst is present from earlier processing (e.g., from the formation of a starch hydrolysate used as feed). The person of ordinary skill in the art will select an amount of acid suitable to provide a desired reaction rate in view of other reaction conditions. For example, in certain embodiments, sufficient acid is present to provide a reaction mixture pH of no more than 4, e.g., no more than 3 or no more than 2.5, such as in the range of 1.0-2.5.

The reaction time will vary depending on reaction conditions, as the person of ordinary skill in the art will appreciate. A wide variety of times can be used. However, in certain embodiments, the reaction time (i.e., time under the recited temperature, acid and solids content conditions) is in the range of 0.1-60 minutes, e.g., 0.1-30 minutes, or 0.1-15 minutes, or 0.1-10 minutes, or 0.5-60 minutes, or 0.5-30 minutes, or 0.5-15 minutes, or 0.5-10 minutes, or 1-60 minutes, or 1-30 minutes, or 1-15 minutes, or 1-10 minutes.

The reaction can be performed in any convenient system, e.g., in a batch reactor, or in a continuous reactor (e.g., a pipe) with continuous flow.

In certain embodiments, the soluble dietary fiber can be made by a process that includes providing a saccharide feed comprising at least 98% (or at least 99%) of dextrose and/or dextrose oligomers on a dry solids basis; reacting the saccharide feed in the presence of water and in the substantial absence of sugar alcohols at a total solids concentration of at least 90 wt% and a temperature of at least 149 °C with at least one acid catalyst that accelerates the rate of cleavage and formation of glucosyl bonds (e.g., at a pH no more than 4, or 1.0-2.5) for a time sufficient to produce a product composition having a fiber content of at least 60% (e.g., 0.1-15 minutes).

As described above, the reaction is performed such that the product composition (i.e., the reaction product) has a fiber content of at least 60%. In certain embodiments, the reaction is performed to provide the product composition with a fiber content that is about the same as that of the soluble dietary fiber (e.g., any value described above with respect to the soluble dietary fiber). However, in other embodiments, the product composition has an intermediate fiber content and a fractionation is performed to improve the fiber content to that of the soluble dietary fiber. Fractionation can be performed, for example, to selectively remove lower molecular weight components (e.g., DP1, or DP1+DP2, or DP1-DP3) as compared to higher molecular weight components. Membrane filtration or sequential simulated moving bed chromatography, for example, can be used in the fractionation.

Further processing by enzymes can also be used, e.g., before or after any fractionation steps. However, in certain embodiments, no processing by enzyme is performed at any point during the reaction or purification sequence.

The person of ordinary skill in the art will appreciate that conventional methodologies can be used for further purifying the product, e.g., decolorization and ion exchange.

Suitable soluble dietary fibers and methods for making them are further described in U.S. Patents nos. 9868969, 9957537, 9963726 and 10344308, and in U.S. Patent Application Publication no. 2012/0034366. The person of ordinary skill in the art can further adapt the methods and materials as described herein based on these references.

Suitable soluble dietary fibers include those sold by Tate & Lyle Ingredients Americas LLC under the tradename PROMITOR^{®}, including but not limited to, PROMITOR^{®} 70, PROMITOR^{®} 85, and PROMITOR^{®} 90.

A major therapeutic target in cardiometabolic syndrome is the cardiac structural change that occurs in the form of left ventricle hypertrophy (LVH) and left atrial enlargement (LAE) both of which are associated with significant morbidity (heart failure, reduced exercise tolerance, angina, atrial fibrillation) and mortality in human subjects with metabolic syndrome. The porcine model described has proven predictive of the biochemical, metabolic and organ specific changes that occur in human MetS. The examples described herein demonstrate a novel, potent cardiac remodeling effect with the use of *L. mucosae* (and *L. mucosae* DPC 6426 in particular) plus soluble dietary fiber supplementation in a clinical scale model of evolving MetS. Moreover, reduction in LVH and LAE was achieved without any effect on hypertension, a traditional therapeutic target in this condition.

Surprisingly, *L. mucosae* DPC6426 + soluble dietary fiber was also shown to reduce both organ and systemic inflammation, both of which contribute to the cardiovascular changes in MetS. Additionally, *L. mucosae +* soluble dietary fiber supplementation corrected key abnormalities of bile acid signaling present in metabolic syndrome in the form of FGF19 regulation. *L. mucosae +* soluble dietary fiber supplementation also augmented gut microbes associated mitigation of metabolic syndrome and obesity markers. In support of a gut microbiome effect, live culture of feces in preliminary group of animals showed significant preservation of therapeutic *L. mucosae* in the mucosal layer of the terminal ileum in *L. mucosae +* soluble dietary fiber supplemented animals compared to those animals supplemented with *L. mucosae* alone.

Pigs fed with only the soluble dietary fiber as described herein over twelve weeks also showed a significant decrease in lipid accumulation in the liver, and the fiber supplementation tended to reduce glucose tolerance indices, total cholesterol, inflammatory and bile acids dysregulation markers.

Across all inflammatory parameters the combination of *L. mucosae* and fiber was far more effective in reducing multiple inflammatory parameters. For instance, *L. mucosae* and fiber supplementation of a HFD significantly decreased bodyweight, as well as all parameters measured associated with cardiac risk in MetS, including inflammation in heart, liver and circulation, fibrosis and lipid accumulation in the liver, and bile acid dysregulatory indices such as FGF19 suppression. Additionally, strong trends were observed for alteration of specific anti-inflammatory microbes in the gut and improvement in gut permeability.

These data suggest that soluble dietary fiber and *L. mucosae* can be used positively to affect numerous physiological parameters (e.g., levels of relevant biomarkers) and anatomical parameters (e.g., heart muscle) related to metabolic syndrome and related constellation of diseases, disorders and morbidities.

Not to be bound by theory, but it appears that fiber acts synergistically with *L*. *mucosae* to augment existing anti-inflammatory bacteria within the gut and to retain more live *L. mucosae* in the mucosal layer where established beneficial effects of these bacteria such as exopolysaccharide production and bile salt hydrolase synthesis have specific anti-inflammatory effects in the intestine, but also systemically and within organs affected by MetS.

Accordingly, the disclosure provides a number of therapeutic methods using combinations of *L. mucosae* and soluble dietary fiber, wherein the *L. mucosae* and soluble dietary fiber are as defined in the claims.

For example, another aspect of the disclosure is a method of reducing weight gain in a mammal, comprising administering to the mammal effective amounts of one or more strains of *Lactobacillus mucosae* and one or more soluble dietary fibers, wherein the *L*. *mucosae* and soluble dietary fiber are as defined in the claims.

Another aspect of the disclosure is a method of treating or preventing cardiovascular disease in a mammal, comprising administering to the mammal effective amounts of one or more strains of *Lactobacillus mucosae* and one or more soluble dietary fibers, wherein the *L. mucosae* and soluble dietary fiber are as defined in the claims.

Another aspect of the disclosure is a method of treating or preventing heart failure in a mammal, comprising administering to the mammal effective amounts of one or more strains of *Lactobacillus mucosae* and one or more soluble dietary fibers, wherein the *L*. *mucosae* and soluble dietary fiber are as defined in the claims.

Another aspect of the disclosure is a method of reducing left ventricle wall thickness in a mammal, comprising administering to the mammal effective amounts of one or more strains of *Lactobacillus mucosae* and one or more soluble dietary fibers, wherein the *L. mucosae* and soluble dietary fiber are as defined in the claims.

Another aspect of the disclosure is a method of reducing left atrial area in a mammal, comprising administering to the mammal effective amounts of one or more strains of *Lactobacillus mucosae* and one or more soluble dietary fibers, wherein the *L. mucosae* and soluble dietary fiber are as defined in the claims.

Another aspect of the disclosure is a method of reducing systemic inflammation in a mammal, comprising administering to the mammal effective amounts of one or more strains of *Lactobacillus mucosae* and one or more soluble dietary fibers, wherein the *L*. *mucosae* and soluble dietary fiber are as defined in the claims.

Another aspect of the disclosure is a method of reducing TNF-alpha in a mammal, comprising administering to the mammal effective amounts of one or more strains of *Lactobacillus mucosae* and one or more soluble dietary fibers, wherein the *L. mucosae* and soluble dietary fiber are as defined in the claims.

Another aspect of the disclosure is a method of increasing FGF19 in a mammal, comprising administering to the mammal effective amounts of one or more strains of *Lactobacillus mucosae* and one or more soluble dietary fibers, wherein the *L. mucosae* and soluble dietary fiber are as defined in the claims.

Another aspect of the disclosure is a method of reducing hsCRP in a mammal, comprising administering to the mammal effective amounts of one or more strains of *Lactobacillus mucosae* and one or more soluble dietary fibers, wherein the *L. mucosae* and soluble dietary fiber are as defined in the claims.

Another aspect of the disclosure is a method of reducing IL1beta in a mammal, comprising administering to the mammal effective amounts of one or more strains of *Lactobacillus mucosae* and one or more soluble dietary fibers, wherein the *L. mucosae* and soluble dietary fiber are as defined in the claims.

Another aspect of the disclosure is a method of reducing PAI1 in a mammal, comprising administering to the mammal effective amounts of one or more strains of *Lactobacillus mucosae* and one or more soluble dietary fibers, wherein the *L. mucosae* and soluble dietary fiber are as defined in the claims.

Another aspect of the disclosure is a method of reducing IFN-gamma in a mammal, comprising administering to the mammal effective amounts of one or more strains of *Lactobacillus mucosae* and one or more soluble dietary fibers, wherein the *L. mucosae* and soluble dietary fiber are as defined in the claims.

Another aspect of the disclosure is a method of reducing CD163 in a mammal, comprising administering to the mammal effective amounts of one or more strains of *Lactobacillus mucosae* and one or more soluble dietary fibers, wherein the *L. mucosae* and soluble dietary fiber are as defined in the claims.

Another aspect of the disclosure is a method of reducing macrophage infiltration in a tissue of mammal, comprising administering to the mammal effective amounts of one or more strains of *Lactobacillus mucosae* and one or more soluble dietary fibers, wherein the *L*. *mucosae* and soluble dietary fiber are as defined in the claims.

Another aspect of the disclosure is a method of increasing IL17A in a mammal, comprising administering to the mammal effective amounts of one or more strains of *Lactobacillus mucosae* and one or more soluble dietary fibers, wherein the *L. mucosae* and soluble dietary fiber are as defined in the claims.

Another aspect of the disclosure is a method of reducing NF-Kb in a mammal, comprising administering to the mammal effective amounts of one or more strains of *Lactobacillus mucosae* and one or more soluble dietary fibers, wherein the *L. mucosae* and soluble dietary fiber are as defined in the claims.

Another aspect of the disclosure is a method of reducing TLR4 in a mammal, comprising administering to the mammal effective amounts of one or more strains of *Lactobacillus mucosae* and one or more soluble dietary fibers, wherein the *L. mucosae* and soluble dietary fiber are as defined in the claims.

Another aspect of the disclosure is a method of treating or preventing non-alcoholic fatty liver disease (NAFLD/NASH) in a mammal, comprising administering to the mammal effective amounts of one or more strains of *Lactobacillus mucosae* and one or more soluble dietary fibers, wherein the *L. mucosae* and soluble dietary fiber are as defined in the claims.

Another aspect of the disclosure is a method of reducing liver fibrosis in a mammal, comprising administering to the mammal effective amounts of one or more strains of *Lactobacillus mucosae* and one or more soluble dietary fibers, wherein the *L. mucosae* and soluble dietary fiber are as defined in the claims.

Another aspect of the disclosure is a method of reducing aSMA in a mammal, comprising administering to the mammal effective amounts of one or more strains of *Lactobacillus mucosae* and one or more soluble dietary fibers, wherein the *L. mucosae* and soluble dietary fiber are as defined in the claims.

Another aspect of the disclosure is a method of reducing fat deposition in the liver in a mammal, comprising administering to the mammal effective amounts of one or more strains of *Lactobacillus mucosae* and one or more soluble dietary fibers, wherein the *L*. *mucosae* and soluble dietary fiber are as defined in the claims.

Another aspect of the disclosure is a method of increasing the abundance of Porphyromonadaceae in the gut microbiome of a mammal, comprising administering to the mammal effective amounts of one or more strains of *Lactobacillus mucosae* and one or more soluble dietary fibers, wherein the *L. mucosae* and soluble dietary fiber are as defined in the claims. In some embodiments, the Porphyromonadaceae is a Parabacteroides.

Another aspect of the disclosure is a method of treating or preventing metabolic syndrome in a mammal, comprising administering to the mammal effective amounts of one or more strains of *Lactobacillus mucosae* and one or more soluble dietary fibers, wherein the *L. mucosae* and soluble dietary fiber are as defined in the claims.

Another aspect of the disclosure is an effective amount of one or more strains of *Lactobacillus mucosae* and one or more soluble dietary fibers for use in reducing weight gain in the mammal, treating or preventing cardiovascular disease in the mammal, treating or preventing heart failure in the mammal, reducing left ventricle wall thickness in the mammal, reducing left atrial area in the mammal, reducing systemic inflammation in the mammal, treating or preventing non-alcoholic fatty liver disease (NAFLD/NASH) in the mammal, reducing liver fibrosis in the mammal, reducing fat deposition in the of the mammal, or treating or preventing metabolic syndrome in the mammal, wherein the *L*. *mucosae* and soluble dietary fiber are as defined in the claims.

Another aspect of the disclosure is an effective amount of one or more strains of *Lactobacillus mucosae* and one or more soluble dietary fibers for use in a method of reducing TNF-alpha, increasing FGF19, reducing hsCRP, reducing IL1beta, reducing PAI1, reducing IFN-gamma, reducing CD163, increasing IL17A, reducing NF-Kb, reducing TLR4, or reducing aSMA in a mammal, or of reducing macrophage infiltration in a tissue of mammal, wherein the *L. mucosae* and soluble dietary fiber are as defined in the claims.

Another aspect of the disclosure is an effective amount of one or more strains of *Lactobacillus mucosae* and one or more soluble dietary fibers for use in a method of increasing the abundance of Parabacteroides in the gut microbiome of a mammal, wherein the *L. mucosae* and soluble dietary fiber are as defined in the claims.

Notably, the one or more strains of *Lactobacillus mucosae* may be administered in an amount of 10⁶ to 10¹⁶ cfu/day.

Typically, the one or more soluble dietary fibers may be administered in an amount of 5 g/day to 50 g/day.

Notably, the one or more soluble dietary fibers and the one or more strains of *L*. *mucosae* may be administered at least once every seven days.

Another aspect of the disclosure, provides a non-therapeutic method of reducing weight gain in a mammal, comprising administering to the mammal effective amounts of one or more strains of *Lactobacillus mucosae* and one or more soluble dietary fibers, wherein the *L. mucosae* and soluble dietary fiber are as defined in the claims.

In certain methods, the mammal is administered effective amounts of one or more strains of *Lactobacillus mucosae* and one or more soluble dietary fibers, included in a composition according to certain embodiments as otherwise described herein or utilized apart from such compositions (i.e., concurrently or sequentially but *L. mucosae* administered separately from the soluble dietary fiber(s)). The terms "effective amount", "effective dosage", "therapeutically effective amount" and "therapeutically effective dosage" can be used interchangeably and are an amount of one or more strains of *L. mucosae* and one or more soluble dietary fibers sufficient to accomplish a stated purpose (e.g. reducing left ventricle wall thickness, reducing left atrial area, reducing TNF-alpha, and the like, as described herein) relative to an untreated mammal. Effective amounts and schedules for administering the agent may be determined empirically by one skilled in the art. The dosage ranges for administration are those large enough to produce the desired effect in which one or more symptoms of the disease or disorder, or presence or abnormal abundance of one or more biomarkers associated with a disease or disorder, e.g., elevated TNF-alpha and chronic inflammation, are affected (e.g., reduced, increased or delayed). The dosage should not be so large as to cause substantial adverse side effects, such as unwanted cross-reactions, anaphylactic reactions, and the like. Generally, the dosage will vary with the species, size, weight, surface area, age, sex, condition, type of disease, the extent of the disease or disorder, route of administration, or whether other drugs are included in the regimen, and can be determined by one of skill in the art. Suitable dosages of the one or more *L. mucosae* and the one or more soluble dietary fibers are provided elsewhere herein. The dosage can be adjusted by the individual physician in the event of any contraindications. Guidance can be found, e.g., in the literature or from a physician, for appropriate, acceptable and/or desirable physiological responses. For example, for a given parameter (e.g., biomarker, physiologic response, and the like), an effective amount may show an increase (e.g., IL-17A) or decrease (NF-Kb, TNF-alpha, others) of at least 5%, 10%, 15%, 20%, 25%, 40%, 50%, 60%, 75%, 80%, 90%, or at least 100%. Efficacy can also be expressed as "-fold" increase or decrease. For example, a therapeutically effective amount can have at least a 1.2-fold, 1.5-fold, 2-fold, 5-fold, or more effect over a control.

In certain embodiments, compositions as otherwise described herein can exhibit "synergy" or "synergistic" behavior, which refers to an effect produced by a combination (e.g., of soluble dietary fiber(s) and one or more *L. mucosae*) that is greater than the sum of their separate effects of the separate components.

The exact dose and formulation for a given human or non-human mammal will depend on the purpose of the treatment, and will be ascertainable by one skilled in the art using known techniques (see, e.g., Lieberman, Pharmaceutical Dosage Forms (vols. 1-3, 1992); Lloyd, The Art, Science and Technology of Pharmaceutical Compounding (1999); Remington: The Science and Practice of Pharmacy, 20th Edition, Gennaro, Editor (2003), and Pickar, Dosage Calculations (1999)).

For prophylactic use, a therapeutically effective amount of the one or more strains of *Lactobacillus mucosae* and one or more soluble dietary fibers, included in a composition according to certain embodiments as otherwise described herein or utilized apart from such compositions (i.e., concurrently or sequentially but *L. mucosae* administered separately from the soluble dietary fiber(s)), are administered to a subject prior to or during early onset (e.g., upon initial signs and symptoms of a disease or disorder, or presence or abnormal abundance of one or more biomarkers associated with a disease or disorder, e.g., elevated TNF-alpha and chronic inflammation). Therapeutic treatment involves administering to a subject a therapeutically effective amount of the one or more strains of *Lactobacillus mucosae* and one or more soluble dietary fibers, again included in a composition or apart from such compositions, after diagnosis or development of a disease or disease. Thus, in another aspect, a method of treating or preventing a disease (e.g., metabolic syndrome, heart failure, non-alcoholic fatty liver disease (NALD/NASH)) in a human or non-human mammal in need thereof is provided.

The terms "mammal" and "non-human mammal" are synonymous with "subject," "patient," "individual," etc., and are not intended to be limiting and can be generally interchanged. That is, a mammal or non-human mammal described as a "patient" does not necessarily have a given disease, but may be merely seeking medical advice.

As used herein, "treating" or "treatment of" a condition, disease or disorder or symptoms associated with a condition, disease or disorder refers to an approach for obtaining beneficial or desired results, including clinical results. Beneficial or desired clinical results can include, but are not limited to, alleviation or amelioration of one or more symptoms or conditions, diminishment of extent of condition, disorder or disease, stabilization of the state of condition, disorder or disease, prevention of development of condition, disorder or disease, prevention of spread of condition, disorder or disease, delay or slowing of condition, disorder or disease progression, delay or slowing of condition, disorder or disease onset, amelioration or palliation of the condition, disorder or disease state, and remission, whether partial or total. "Treating" can also mean prolonging survival of a subject beyond that expected in the absence of treatment. "Treating" can also mean inhibiting the progression of the condition, disorder or disease, slowing the progression of the condition, disorder or disease temporarily, although in some instances, it involves halting the progression of the condition, disorder or disease permanently. As used herein, the terms treatment, treat, or treating refers to a method of reducing the effects of, or biomarkers related to, one or more symptoms of a disease or condition. Thus, in the disclosed methods, treatment can refer to a 5%, 10%, 20%, 30%, 40%, 50%, 60%, 70%, 80%, 90%, or 100% reduction in the severity of an established disease, condition, or symptom of the disease or condition (e.g., cardiovascular disease, heart failure, non-alcoholic fatty liver disease, metabolic syndrome) or biomarker related thereto (e.g., reducing left ventricle wall thickness, reducing left atrial area, reducing TNF-alpha, increasing FGF19, and others as described herein). It is understood that treatment does not necessarily refer to a cure or complete ablation of the disease, condition, or symptoms of the disease or condition, or permanent or non-permanent restoration of normal, healthy levels of biomarkers related thereto. Further, as used herein, references to decreasing, reducing, or inhibiting include a change of 10%, 20%, 30%, 40%, 50%, 60%, 70%, 80%, 90% or greater as compared to a control level and such terms can include but do not necessarily include complete elimination.

Compositions comprising one or more strains of *Lactobacillus mucosae* and one or more soluble dietary fibers, included in a composition according to certain embodiments as otherwise described herein or utilized apart from such compositions (i.e., concurrently or sequentially but *L. mucosae* administered separately from the soluble dietary fiber(s)), can be administered to the gastrointestinal tract of a subject by nasoduodenal catheter, orally in a consumable capsule (including softgel and the like), pill, or ingestion of functional food or beverage product, which should be understood to include comestible products, such as foods and drinks, including medical foods and beverages.

Regardless of how the compositions are formulated, the dosages of the soluble dietary fiber and the *L. mucosae* required will depend on the route of administration, the nature of the formulation, the nature of the human or non-human animal, e.g., their species, size, weight, surface area, age, and sex, other drugs being administered, and the judgment of the attending clinicians. In certain embodiments, suitable dosages of the one or more *L*. *mucosae* are as described elsewhere herein, whether included in a composition or utilized apart from such compositions (i.e., concurrently or sequentially but separately from the soluble dietary fiber).

The person of ordinary skill in the art will, based on the disclosure herein, determine an appropriate dosage of the one or more strains of *Lactobacillus mucosae.* For example, in certain embodiments as otherwise described herein, the one or more strains of *Lactobacillus mucosae* are administered in an amount of 10⁶ to 10¹⁶ cfu/day, e.g., in the range of 10⁸ to 10¹⁶ cfu/day, or 10¹⁰ to 10¹⁶ cfu/day. In certain embodiments as otherwise described herein, the one or more strains of *Lactobacillus mucosae* are administered in an amount of 10⁶ to 10¹⁴ cfu/day, e.g., in the range of 10⁸ to 10¹⁴ cfu/day, or 10¹⁰ to 10¹⁴ cfu/day. In certain embodiments as otherwise described herein, the one or more strains of *Lactobacillus mucosae* are administered in an amount of 10⁶ to 10¹² cfu/day, e.g., in the range of 10⁸ to 10¹² cfu/day, or 10¹⁰ to 10¹² cfu/day.

The person of ordinary skill in the art will, based on the disclosure herein, determine an appropriate dosage of the one or more soluble dietary fibers. For example, in certain embodiments as otherwise described herein, the one or more soluble dietary fibers are administered in an amount of 0.1 g/day to 50 g/day, e.g., 0.1 g/day to 40 g/day, or 0.1 g/day to 35 g/day, or 0.1 g/day to 25 g/day, or 0.1 g/day to 15 g/day, or 0.1 g/day to 10 g/day. In certain embodiments as otherwise described herein, the one or more soluble dietary fibers are administered in an amount of 0.5 g/day to 50 g/day, e.g., 0.5 g/day to 40 g/day, or 0.5 g/day to 35 g/day, or 0.5 g/day to 25 g/day, or 0.5 g/day to 15 g/day, or 0.5 g/day to 10 g/day. In certain embodiments as otherwise described herein, the one or more soluble dietary fibers are administered in an amount of 2 g/day to 50 g/day, e.g., 2 g/day to 40 g/day, or 2 g/day to 35 g/day, or 2 g/day to 25 g/day, or 2 g/day to 15 g/day, or 2 g/day to 10 g/day. In certain embodiments as otherwise described herein, the one or more soluble dietary fibers are administered in an amount of 5 g/day to 50 g/day, e.g., 5 g/day to 40 g/day, or 5 g/day to 35 g/day, or 5 g/day to 25 g/day, or 5 g/day to 15 g/day. In certain embodiments as otherwise described herein, the one or more soluble dietary fibers are administered in an amount of 10 g/day to 50 g/day, e.g., 10 g/day to 40 g/day, or 10 g/day to 35 g/day, or 10 g/day to 25 g/day, or 10 g/day to 15 g/day. In certain embodiments as otherwise described herein, the one or more soluble dietary fibers are administered in an amount of 20 g/day to 50 g/day, e.g., 20 g/day to 40 g/day, or 20 g/day to 35 g/day. Fiber dosages as described herein are measured on a dry weight basis, i.e., excluding the mass of any water present and excluding the mass of any food or beverage carrier in which the fiber is disposed. The soluble dietary fiber can be provided in any suitable form, e.g., in the form of a food or beverage.

Administrations can be single or multiple (e.g., 2- or 3-, 4-, 6-, 8-, 10-, 20-, 50-, 100-, 150-, or more fold). The duration of treatment with any composition provided herein or treatment with one or more strains of *Lactobacillus mucosae* and one or more soluble dietary fibers utilized apart from such compositions (i.e., concurrently or sequentially but *L*. mucosae administered separately from the soluble dietary fiber(s)), can be any length of time from as short as one day to as long as the life span of the host (e.g., many years). For example, a composition (or *L. mucosae* and soluble dietary fiber(s) apart from a composition described herein) can be administered 1 or more times per day, 1, 2, 3, 4, 5, 6, or 7 times a week (for, for example, 4 weeks to many months or years); once a month (for example, three to twelve months or for many years); or once a year for a period of 5 years, ten years, or longer. It is also noted that the frequency of treatment can be variable. For example, the present compositions can be administered once (or twice, three times, etc.) daily, weekly, monthly, or yearly.

The compositions (and *L. mucosae* and soluble dietary fiber(s) apart from a composition described herein) may also be administered in conjunction with other therapeutic agents, including procedures (e.g., surgery) and behavior modification (e.g., dietary changes). Concurrent administration of two or more therapeutic agents does not require that the agents be administered at the same time or by the same route, as long as there is an overlap in the time period during which the agents are exerting their therapeutic effect. Simultaneous or sequential administration is contemplated, as is administration on different days or weeks.

In certain embodiments as otherwise described herein, the compositions can further include a pharmaceutically acceptable excipient, carrier, diluent or vehicle. "Pharmaceutically acceptable excipient" refers to substances that while having no significant medicinal properties, they can, e.g., facilitate manufacture and/or physiological absorption of an active substance such as the one or more strains of *Lactobacillus mucosae* and one or more soluble dietary fibers. Excipients might aid in lubricity, flowability, disintegration, taste and may confer some form of antimicrobial or other preservative function. A "pharmaceutically acceptable carrier" refers to substances that are designed to interact with and enhance the properties of active substances, often pertaining to formulation of such substances. Carriers can, e.g., facilitate powder flowability or enhance non-stick properties. "Pharmaceutically acceptable diluent" refers to substances that can act as fillers in tablets to increase weight and improve content uniformity. Natural diluents include starches, hydrolyzed starches, and partially pregelatinized starches. Common diluents include anhydrous lactose, lactose monohydrate, and sugar alcohols such as sorbitol, xylitol and mannitol. Diluents provide better tablet properties such as improved cohesion or to promote flow. "Pharmaceutically acceptable" refers to substances approved by a regulatory agency of the Federal or a state government or listed in the U.S. Pharmacopeia or other generally recognized pharmacopeia for use in human and non-human animals that aid the administration of an active agent to and absorption by a subject and can be included in the compositions of the present disclosure without causing a significant adverse toxicological effect on the human or non-human animal subject. Non-limiting examples of pharmaceutically acceptable excipients include water, NaCl, normal saline solutions, lactated Ringer's, normal sucrose, normal glucose, binders, fillers, disintegrants, lubricants, coatings, sweeteners, flavors, salt solutions (such as Ringer's solution), alcohols, oils, gelatins, carbohydrates such as lactose, amylose or starch, fatty acid esters, hydroxymethycellulose, polyvinyl pyrrolidine, and colors, and the like. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances and the like that do not deleteriously react with the compounds of the invention. One of skill in the art will recognize that other pharmaceutical excipients are useful in the present invention.

Non-limiting examples of pharmaceutically-acceptable excipients can be found, for example, in Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pa. 1975; Liberman, H. A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins1999).

In certain embodiments, the compositions provided herein may be administered orally, or gastrointestinally. Administration can be in the form of a single bolus dose, regular dosing over a set period of time or open-ended period of time (as described elsewhere herein), or may be, for example, by a continuous perfusion pump over a set period of time or open-ended period of time.

The dosage can be in the form of a solid, semi-solid, or liquid composition. Non-limiting examples of dosage forms suitable for use include feed, functional food product, pellet, lozenge, liquid, elixir, powder, tablet, pill, capsule, gel, geltab, nanosuspension, nanoparticle, microgel, troches, aqueous or oily suspensions, emulsion, creams, dispersible powders or granules, emulsion in hard or soft gel capsules, syrups, nutraceuticals, dietary supplement, and any combination thereof.

In certain embodiments, the compositions provided herein can be administered orally. Such compositions can be administered in one or more forms noted above, for example, through a capsule, pill, powder, tablet, gel, or liquid, designed to release the composition in the gastrointestinal tract.

In certain embodiments, the one or more strains of *Lactobacillus mucosae* and one or more soluble dietary fibers provided herein is combined with one or more excipients, for example, a disintegrant, a filler, a glidant, or a preservative. In embodiments, the one or more strains of *Lactobacillus mucosae* and one or more soluble dietary fibers provided herein forms part of a capsule. The compositions can be administered orally, for example, through a capsule, pill, powder, tablet, gel, or liquid, designed to release the composition in the gastrointestinal tract. Suitable capsules include both hard-shell capsules or soft-shelled capsules. Any lipid-based or polymer-based colloid may be used to form the capsule. Exemplary polymers useful for colloid preparations include gelatin, plant polysaccharides or their derivatives such as carrageenans and modified forms of starch and cellulose, e.g., hypromellose. Optionally, other ingredients may be added to the gelling agent solution, for example plasticizers such as glycerin and/or sorbitol to decrease the capsule's hardness, coloring agents, preservatives, disintegrants, lubricants and surface treatment.

In certain embodiments, the compositions as otherwise described herein can further include one or more agents that can enhance stability and/or survival of one or more strains of *L. mucosae.* Non-limiting example of stabilizing agents include glycerin, ascorbic acid, skim milk, lactose, tween, alginate, xanthan gum, carrageenan gum, mannitol, palm oil, and poly-L-lysine (POPL). In certain embodiments, the compositions as otherwise described herein can further include a saline solution, glycerin, and combinations thereof.

In some embodiments, the compositions as otherwise described herein can be formulated such that the one or more strains of *L. mucosae* can replicate once they are delivered to the target habitat (e.g. gut). In some embodiments, the composition is formulated such that the one or more strains of *L. mucosae* are viable in the target habitat (e.g., gut). In one non-limiting example, the composition is formulated in a pill or capsule, such that the pill or capsule has a shelf life of at least about: 1, 2, 3, 4, 5, 6, 7, 8, 9, 10, 11, or 12 months. In another non-limiting example, the storage of the composition is formulated so that one or more strains of *L. mucosae* can reproduce in the target habitat (e.g., gut). In some embodiments, other components may be added to aid in the shelf life of the microbial composition. In some embodiments, the composition comprising one or more strains of *L*. *mucosae* may be formulated in a manner that it is able to survive in a non-natural environment. For example, a microbe that is native to the gut may not survive in an oxygen-rich environment. To overcome this limitation, the composition comprising one or more strains of *L. mucosae* can be formulated in a pill, capsule or other oral, gastrointestinal or rectal delivery vehicle that can reduce or eliminate the exposure to oxygen.

In some embodiments, the composition as otherwise described herein is formulated as an enteric-coated pill or capsule for oral administration. In some embodiments, the composition is formulated for delivery of one or more strains of *L*. *mucosae* and one or more soluble dietary fibers to the ileum region of a subject. In some embodiments, the composition is formulated for delivery of the *L. mucosae* and fiber to the colon region (e.g. upper colon) of a subject. In some embodiments, the composition is formulated for delivery of the *L. mucosae* and fiber to the ileum and colon (e.g., upper colon) regions of a subject.

An enteric-coating can protect the contents of a formulation, for example, oral formulation such as pill or capsule, from the acidity of the stomach. An enteric-coating can provide delivery to the ileum and/or upper colon regions. A composition as otherwise described herein can be formulated such that the contents of the composition may not be released in a body part other than the gut region, for example, ileum and/or colon region of the subject. Non-limiting examples of enteric coatings include pH sensitive polymers (e.g., eudragit FS30D), methyl acrylate-methacrylic acid copolymers, cellulose acetate succinate, hydroxy propyl methyl cellulose phthalate, hydroxy propyl methyl cellulose acetate succinate (e.g., hypromellose acetate succinate), polyvinyl acetate phthalate (PVAP), methyl methacrylate-methacrylic acid copolymers, shellac, cellulose acetate trimellitate, sodium alginate, zein, other polymers, fatty acids, waxes, shellac, plastics, and plant fibers.

The enteric coating can be designed to dissolve at any suitable pH. In some embodiments, the enteric coating is designed to dissolve at a pH greater than from about pH 6.5 to about pH 7.0. In some embodiments, the enteric coating is designed to dissolve at a pH greater than about pH 6.5. In some embodiments, the enteric coating is designed to dissolve at a pH greater than about pH 7.0. The enteric coating can be designed to dissolve at a pH greater than about: 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, or 7.5 pH units. The enteric coating can be designed to dissolve in the gut, for example, ileum and/or colon region. The enteric coating can be designed to not dissolve in the stomach.

In embodiments, compositions can be formulated in a unit dosage form, each dosage containing, for example, from 10³-10¹⁵ cfu (or any other dosage described herein with respect to a daily dosage) of the one or more strains of *L. mucosae* as otherwise described herein and from 0.1-50 g (or any other dosage described herein with respect to a daily dosage) of the one or more soluble dietary fibers according to certain embodiment as otherwise described herein. The term "unit dosage forms" refers to physically discrete units suitable as unitary dosages for human and non-human mammals, each unit containing a predetermined quantity of "active materials" (i.e., *L. mucosae* and soluble dietary fiber(s)) calculated, known, observed or otherwise expected to produce the desired therapeutic effect, in association with a suitable pharmaceutical excipient.

For preparing solid compositions (e.g., capsules, tablets and functional foods such as medical foods), the "active materials" are mixed with a pharmaceutical excipient to form a solid preformulation composition containing a homogeneous mixture of *L. mucosae* and soluble dietary fiber as otherwise described herein. When referring to these preformulation compositions as homogeneous, the active materials are typically dispersed evenly throughout the composition so that the composition can be readily subdivided into equally effective unit dosage forms such as tablets, pills and capsules. For functional foods (e.g., medical foods), the active materials can be mixed with other edible ingredients necessary to make the functional food (e.g., to make a healthy bar functional food product, fruits, seeds (e.g., chia), nuts (including nut flours), grains (including flours), sweeteners, and flavorants may be used to make a solid preformulation as well, though attaining homogeneity is not necessary for such solid compositions. Solid preformulations are then subdivided into unit dosage forms of the type described above containing a desired amount of at least one of the one or more strains of *L. mucosae* as otherwise described herein and at least one of the one or more soluble dietary fibers as otherwise described herein.

*Lactobacillus mucosae DPC 6426* has been demonstrated to have technological and biological robustness, and thus would be understood to be suitable for use in a variety of food applications. Indeed, *L. mucosae* DPC 6426 exhibits increased survivability under a variety of food processing conditions (e.g., 120-min exposure to 5 mol NaCl, 90-min exposure to 0.7% (w/v) bile, exposure to simulated gastric juice for 10 min, 60-min exposure to HCI, 30-min heat exposure to 55°C) compared to a non-EPS-producing Lactobacilli of the same species, and in different food applications where it remains viable throughout storage (e.g., yoghurt, cheese). *See, e.g.*, P.M. Ryan et al., "Reduced-fat Cheddar and Swiss-type cheeses harboring exopolysaccharide-producing probiotic Lactobacillus mucosae DPC 6426," J Dairy Sci., 98(12), 8531-44 (2015); L. London et al., "Use of Lactobacillus mucosae DPC 6426, an exopolysaccharide-producing strain, positively influences the techno-functional properties of yoghurt," International Dairy Journal. 40, 10 (2015); L.E. London, "Characterization of a bovine isolate Lactobacillus mucosae DPC 6426 which produces an exopolysaccharide composed predominantly of mannose residues," J. Appl. Microbiol. 117(2), 509-17 (2014).

Compositions as otherwise described herein can be packaged as a kit. In some embodiments, a kit includes written instructions on the administration/use of the composition. The written material can be, for example, a label. The written material can suggest conditions and methods of administration. The instructions provide the subject and the supervising physician with the best guidance for achieving the optimal clinical outcome from the administration of the therapy. The written material can be a label. In some embodiments, the label can be approved by a regulatory agency, for example the U.S. Food and Drug Administration (FDA), the European Medicines Agency (EMA), or other regulatory agencies.

While various embodiments and aspects of the present invention are shown and described herein, it will be obvious to those skilled in the art that such embodiments and aspects are provided by way of example only. Numerous variations, changes, and substitutions will now occur to those skilled in the art without departing from the invention. It should be understood that various alternatives to the embodiments of the invention described herein may be employed in practicing the invention.

The section headings used herein are for organizational purposes only and are not to be construed as limiting the subject matter described.

In this disclosure, "comprises," "comprising," "containing," and "having" and the like can have the meaning ascribed to them in U.S. Patent law and can mean "includes," "including," and the like. "Consisting essentially of" or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments. By contrast, the transitional phrase "consisting of" excludes any element, step, or ingredient not specified.

As used herein, the term "about" in the context of a numerical value or range means ±10% of the numerical value or range recited or claimed, unless the context requires a more limited range.

In the descriptions herein and in the claims, phrases such as "at least one of" or "one or more of" may occur followed by a conjunctive list of elements or features. The term "and/or" may also occur in a list of two or more elements or features. Unless otherwise implicitly or explicitly contradicted by the context in which it is used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together." In addition, use of the term "based on," above and in the claims is intended to mean, "based at least in part on," such that an unrecited feature or element is also permissible.

As used in the description herein and throughout the claims that follow, the meaning of "a," "an," and "the" includes plural reference unless the context clearly dictates otherwise.

### Examples

The examples herein were carried out utilizing a pig model of metabolic syndrome and various methods as described in the Methods section below.

### Example 1 - Body weight

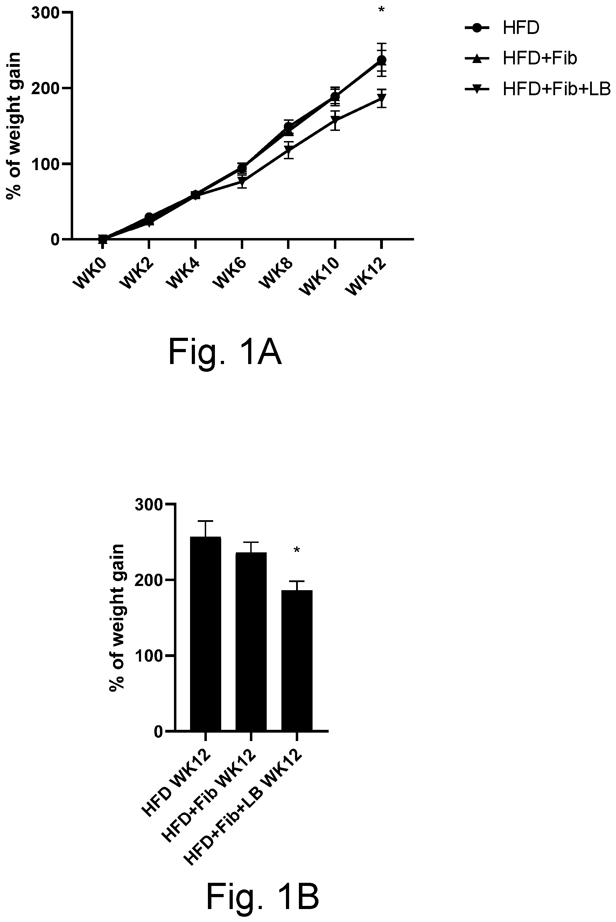

The percentage of body mass gain progression was measured over twelve weeks, where the percentage of body mass gain was lower from T6 (weeks) and was significantly decreased at T12 (weeks) in pigs supplemented with *Lactobacillus mucosae* DPC6426 (also referred to as "LB" and "Lb" herein; in certain embodiments strain DPC 6426) and soluble dietary fiber (generically "Fiber") compared to HFD (high fat diet) alone (p=0.03, Figures 1A and 1B). However, supplementation with Fiber alone had no significant effect on weight loss. Thus, Figures 1A and 1B demonstrate that body weight was reduced by diet supplementation. Percentage of body weight gain compared between diets over time from baseline to T12 (A) and compared among test groups at T12 (B). Diet: HFD (circles), HFD+Fiber (up-point triangles), HFD+LB+Fiber (down-point triangles). Significance was calculated using ANOVA followed by Tukey's multiple comparisons test using PRISM. P=0.03 (*).

### Example 2 - The Heart -Cardiac structure, hemodynamics, inflammation and fibrosis

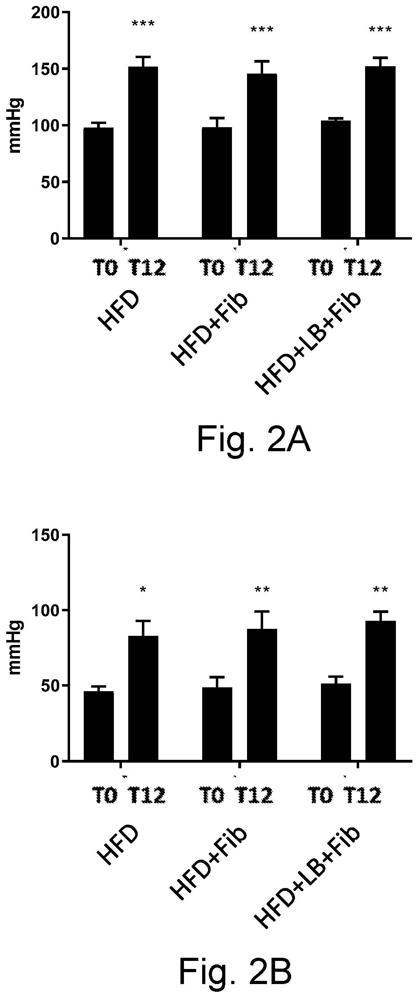

Cardiac structural and hemodynamic parameters were also assessed. All pigs exhibited significantly elevated values for systolic blood pressure (HFD:1.54-fold, HFD+Fib: 1.48-fold, HFD+LB+Fib:1.46-fold, p<0.001, Figure 2A) and diastolic blood pressure (HFD:1.79-fold p=0.019, HFD+Fib:1.79-fold p=0.0065 HFD+LB+Fib: 1.81-fold p=0.013, Figure 2B) at T12 compared to T0. At T12, no significant difference was observed between pigs fed with HFD and pigs receiving different supplementation (Figures 2A and B).

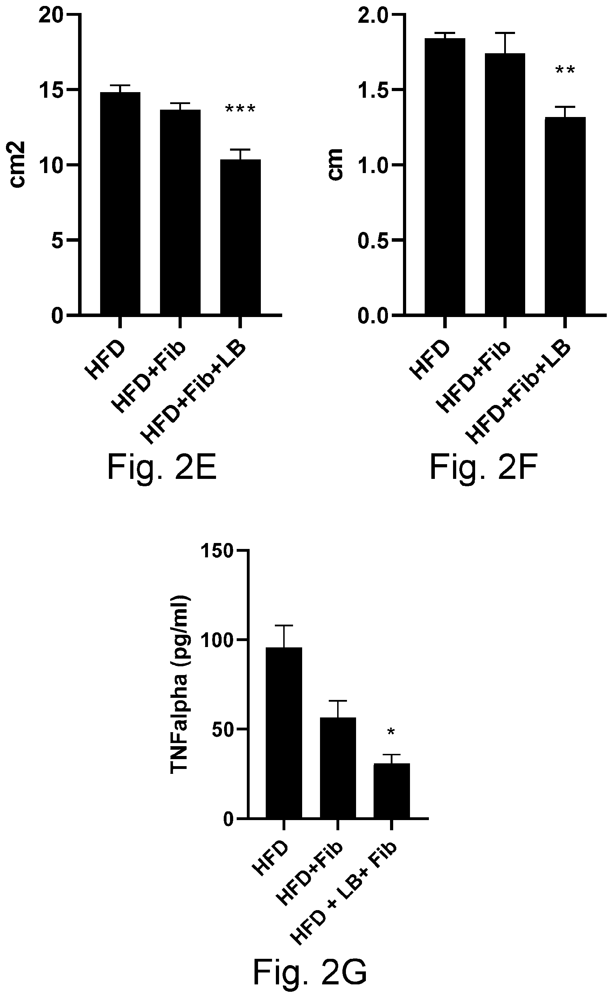

Left ventricle (LV) wall thickness (1.39-fold, p=0.0044) and left atrium (LA) area (1.42-fold, p<0.0001), two markers of cardiovascular risk and shown to be increased in metabolic syndrome (also "MetS"), were significantly decreased by supplementation of LB and fiber (Figure 2F and 2E, respectively). Moreover, calculated LV mass, assuming a cylindrical bullet LV shape, was >20% reduced using LB+ fiber supplementation in MetS animals compared to no supplementation (p<0.001). Fiber alone was comparable to MetS pigs receiving HFD.

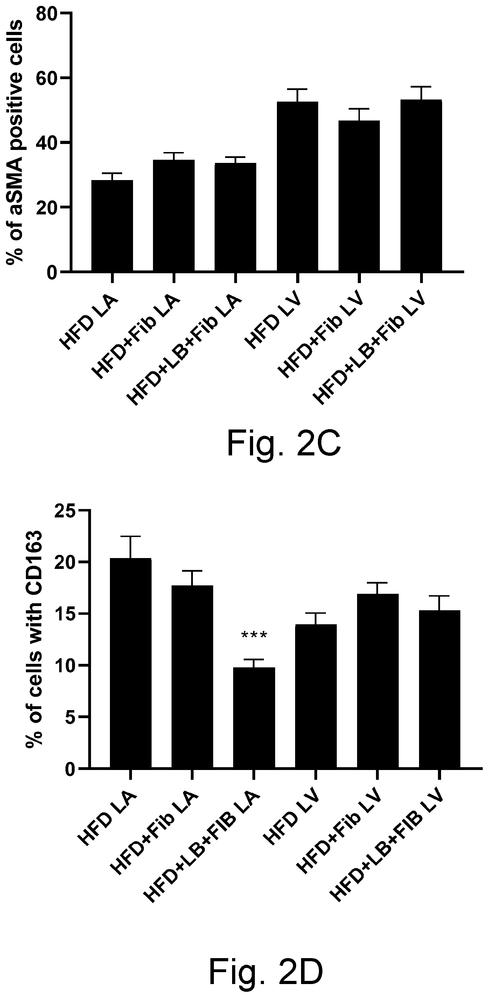

Cardiac fibrosis in the form of alpha smooth muscle actin (aSMA) immunoreactivity was not significantly reduced by any supplementation in LA and LV (Figure 2C). Inflammation in the form of CD163 immunoreactivity was significantly decreased in LA by LB+Fiber supplementation compared to MetS animals (HFD vs HFD+LB+Fib p<0.0001, Figure 2D). None of the supplementations demonstrated a significant effect on inflammation in LV tissue (Figure 2D). In line with these results, the levels of TNF-alpha, one of the major pro inflammatory cytokines in heart failure and shown to be increased in the serum of MetS pigs (PMID: 29218105, PMID: 22699305), was significantly decreased by LB+Fiber supplementation (p=0.01, Figure 2G). These findings alone are novel, and that LB+Fiber supplementation decreased two markers of cardiovascular risk independently of affecting blood pressure is particularly surprising as mechanisms that affect the structure and function of the heart independently from blood pressure are unknown in the art.

Significant difference was calculated using ANOVA followed by Tukey's multiple comparisons test using PRISM. P<0.05 (*) P<0.01 (**), P< 0.001 (***).

### Example 3 - The Liver - Fat infiltration, inflammation and fibrosis

Hepatic inflammation in the form of CD163 immunoreactivity and liver fibrosis assessed by aSMA marker were also determined. In the liver, accumulation of hepatic lipid activates Kupffer cells, which are macrophages that express CD163. CD163 is detectible marker that correlates with the degree of Kupffer cell activation and is also a marker in NAFLD and other disorders (PMID: 2587793, PMID: 20206177).

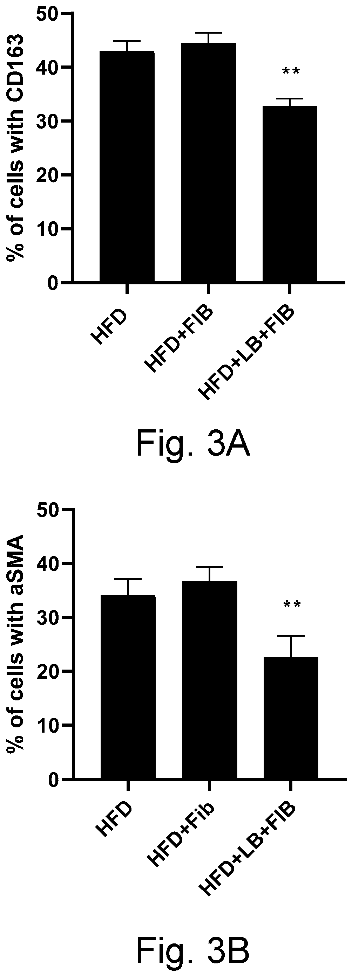

Once activated, macrophages produce pro-inflammatory markers, such as TNF-alpha, increasing the differentiation of hepatic stellate cells into myofibroblasts, leading, e.g., to the progression of non-alcoholic steatohepatitis (NASH) (PMID: 12161342, PMID: 25561778, PMID: 28267621, PMID: 28082803). Hepatic inflammation in the form of CD163 immunoreactivity was significantly decreased by LB+fiber supplemented pigs compared to HFD pigs (1.31-fold, p=0.0019, Figure 3A).

Fibrosis in the liver was assessed by the presence of alpha smooth muscle actin (aSMA) marker, which was significantly decreased by supplementation of LB+fiber compared to pigs fed with HFD alone (HFD vs HFD+LB+Fib:1.51-fold, p=0.03, Figure 3B). The Fiber only supplementation group was not significantly different to the HFD group.

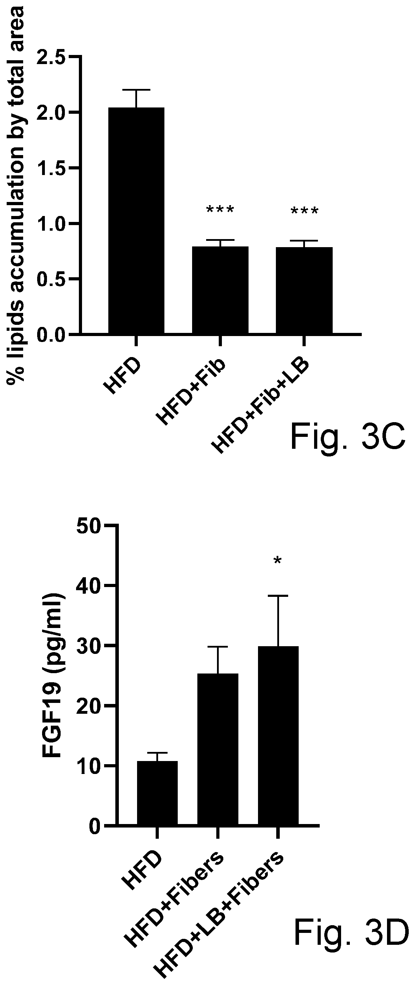

Fat deposition in the liver was significantly decreased by the different supplementation groups compared to MetS pigs on HFD (HFD vs HFD+Fib: 2.58-fold, p<0.0001, HFD vs HFD+LB+Fib: 2.6-fold, p<0.0001, Figure 3C). Consequently. Fiber+LB is expected to attenuate the development of NAFLD/NASH in metabolic syndrome.

Fibroblast growth factor 9 (FGF19) is a protein that regulates bile acid synthesis. When secreted into portal blood, FGF19 inhibits the production of bile acids in the liver, and can regulate glucose and lipids metabolism by reducing the transcription of CYP7A1 (PMID: 12815072, PMID: 16213224, PMID: 22302876, PMID: 19233843). In the MetS animals described herein, a decrease of FGF19 in the circulation was detected. However, FGF19 serum level was significantly increased by the LB+Fiber supplementation (returning to near normal levels as seen in control non-MetS pigs) and appeared non-significantly elevated by Fiber only supplementation compared to MetS pigs (HFD vs HFD+LB+Fib: 2.77-fold, p=0.04, Figure 3D).

Significant difference was calculated using ANOVA followed by Tukey's multiple comparisons test using PRISM. P<0.05 (*) P<0.01 (**), P< 0.001 (***)

### Example 4 - Systemic and cellular inflammation

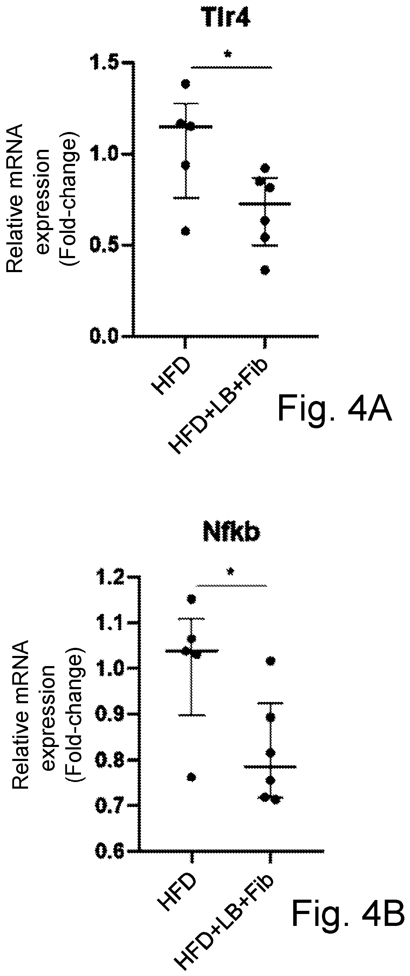

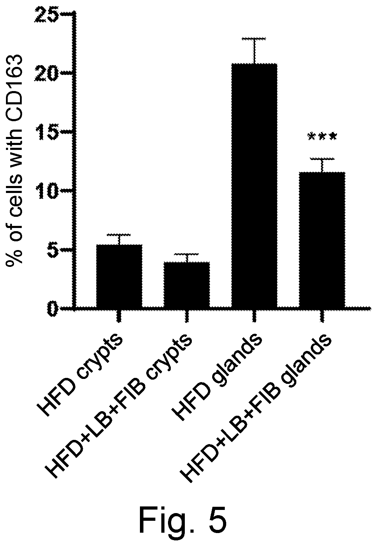

We assessed cellular and multiple cytokine markers of inflammation in the gut (terminal ileum), the circulation, the heart. LB+Fiber significantly suppressed key inflammatory signals associated with macrophage activation in the terminal ileum (TI), including relative mRNA expression of TLR4 (Figure 4A) and NFkB assessed with real-time PCR (Figure 4B), and reduction of CD163 staining in glands of the terminal ileum (Figure 5), and compared among diets. A non-significant reduction of CD163 in TI crypts was noted. Significant difference was calculated using T- test using PRISM. P<0.05 (*).

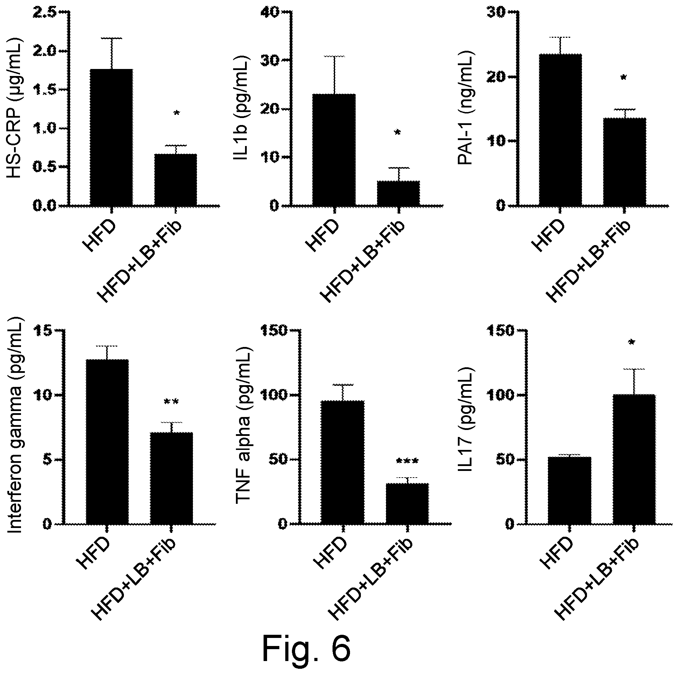

The concentration of inflammation markers in the systemic circulation in response to HFD and HFD+LB+Fiber diet was assessed. Serum concentrations of hsCRP (Figure 6, top left, IL1b (Figure 6, top center), PAI1 (Figure 6, top right), IFN-gamma (Figure 6, bottom left), and TNFα (Figure 6, bottom center) were all significantly reduced, and IL17A, a counter-regulatory T cell associated cytokine known to be reduced in MetS in humans, was significantly increased (Figure 6, bottom right).

Significant difference was calculated using T-test using PRISM. P<0.05 (*) P<0.01 (**), P< 0.001 (***).

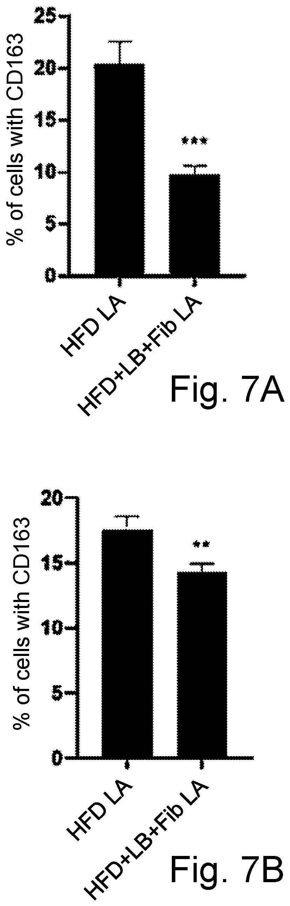

In the heart, expression of inflammation markers in the LA and RA, HFD+LB+Fiber reduced macrophage infiltration in the left atrium as evidenced by significantly reduced CD163 and CD16 staining (Figures 7A and 7B, respectively). Significant difference was calculated using T-test using PRISM.P<0.01 (**), P< 0.001 (***).

Together these data indicate LB + Fiber reduce gut inflammation with concomitant reduction in systemic and end organ inflammation.

### Example 5 - Lipid and glucose metabolism - Lipid Glucose insulin profiles

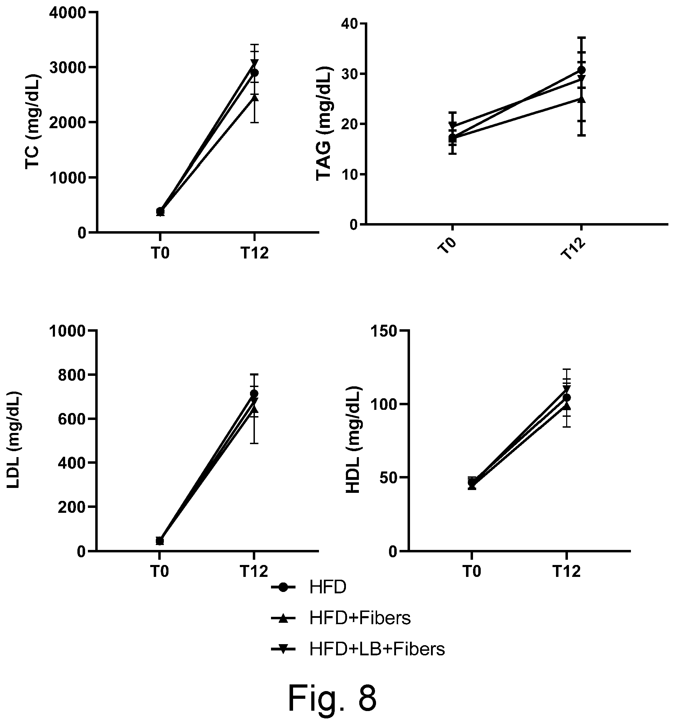

MetS pigs exhibit a mixed hyperlipidaemia, so the effects of *L. mucosae* and Fiber diet supplementation on the lipid profile of MetS pigs was assessed. Serum concentration of total cholesterol (TC; Figure 8, top left), triglyceride (TAG; Figure 8, top right), low-density lipoprotein (LDL; Figure 8, bottom left) and high density lipoproteins (HDL; Figure 8, bottom right) at baseline (T0) and 12 weeks (T12) were measured and compared between diets (HFD (circles), HFD+Fiber (up-point triangle), HFD+LB+Fiber (down-point triangle)). Significant difference was calculated using ANOVA followed by Tukey's multiple comparisons test using PRISM. In the current study fasting serum lipids including triglyceride, total cholesterol, LDL, were not were not significantly reduced by LB and Fiber supplementation in pigs fed HFD at T12, although supplementation with fiber demonstrated some reduction of total cholesterol.

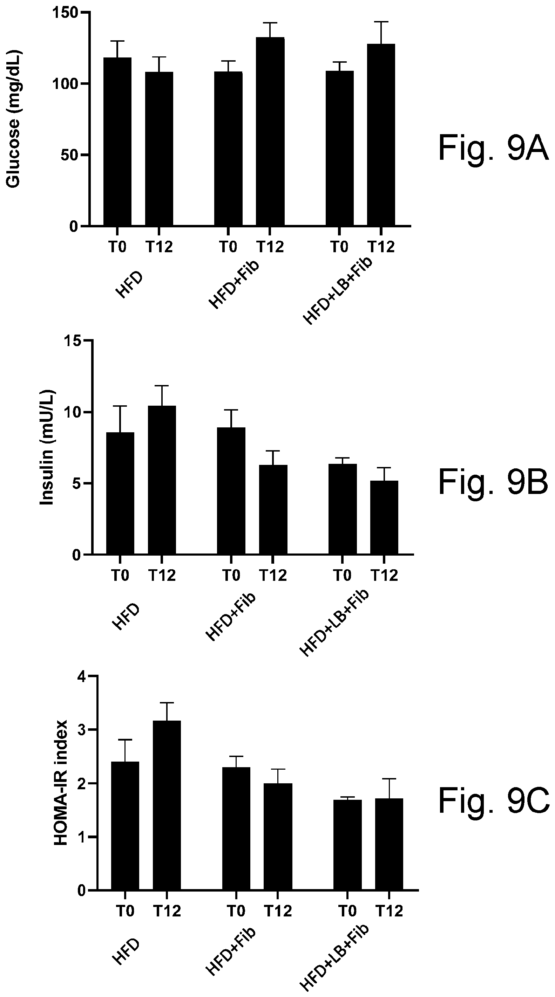

The MetS pig model utilized in these examples shows an early form of insulin resistance. For example, at T12 fasting insulin levels and HOMA insulin resistance index was significantly increased in MetS compared to control animals. MetS pigs fed with LB and Fiber supplementation did not exhibit significantly reversed early insulin resistance. No significant difference was observed in fasting glucose (Figure 9A), insulin levels (Figure 9B), or HOMA calculation (Figure 9C) in pigs supplemented with LB and Fiber.

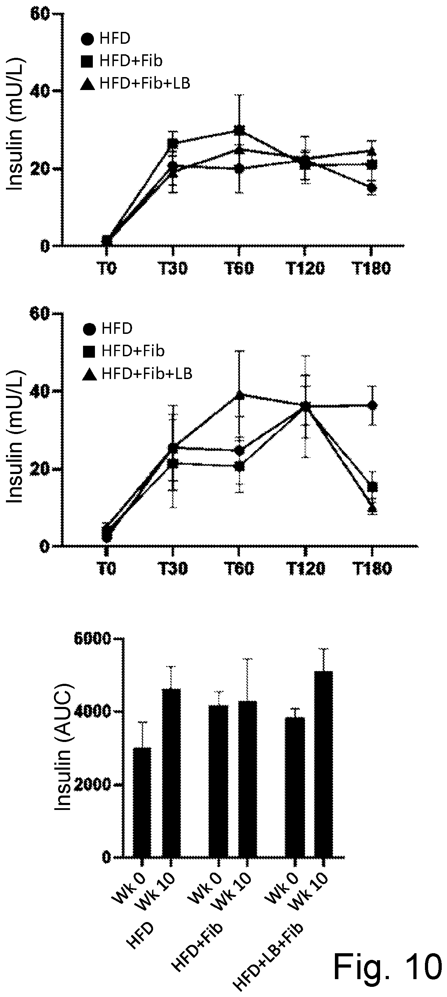

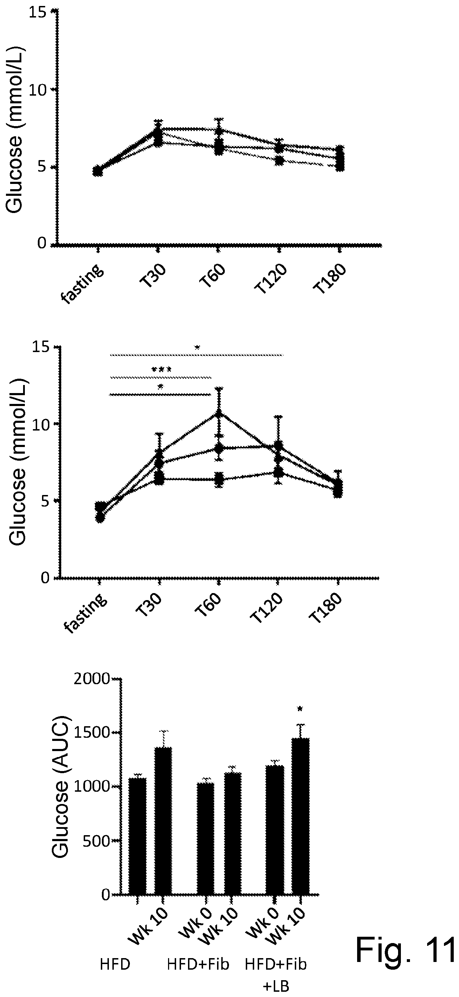

After a glucose meal, the level of insulin and glucose were measured over 3h and no significant difference in the level of insulin between the groups was observed (Figure 10). At T0, no significant difference was seen in glucose level overtime between the 3 groups (Figure 11, top). However, at T10 (weeks), the level of glucose was significantly increased 60 and 120 minutes after a glucose meal compared to fasting level in HFD (p=0.018, p=0.013) and 60 minutes in HFD+LB+Fiber (p<0.001) (Figure 11, center). Fiber supplementation alone appears to prevent the increase in glucose level observed in the two other groups of animals. Measuring the area under the curve at T0 and T12 and comparing among groups show that only HFD+LB+Fiber AUC was increased significantly (p=0.0159, Figure 11, bottom).

Of note is that the Landrace porcine MetS model is primarily designed to mimic the lipid, hepatic, gut microbiome and cardiovascular aspects of MetS and associated diseases, disorders and morbidities rather than pre-diabetes or diabetes. Further, the pig is generally resistant to diabetes due to a larger beta cell capacity in the pancreas compared to humans.

### Example 6 - Gut health - Intestinal membrane integrity

Terminal ileum membrane integrity of MetS pigs in response to different diet supplementation was assessed. Zonulin is a biomarker of intestinal permeability. Elevated serum zonulin is considered to be a marker of impaired intestinal barrier in patients with inflammatory bowel disease (PMID: 22902773, PMID: 16644703, PMID: 24868498). Zonulin binds epidermal growth factor receptor (EGFR) and protease-activated receptor 2 (PAR2) in the intestinal epithelium, leading to the tight junction disassembling in the small intestine (PMID: 19805376).

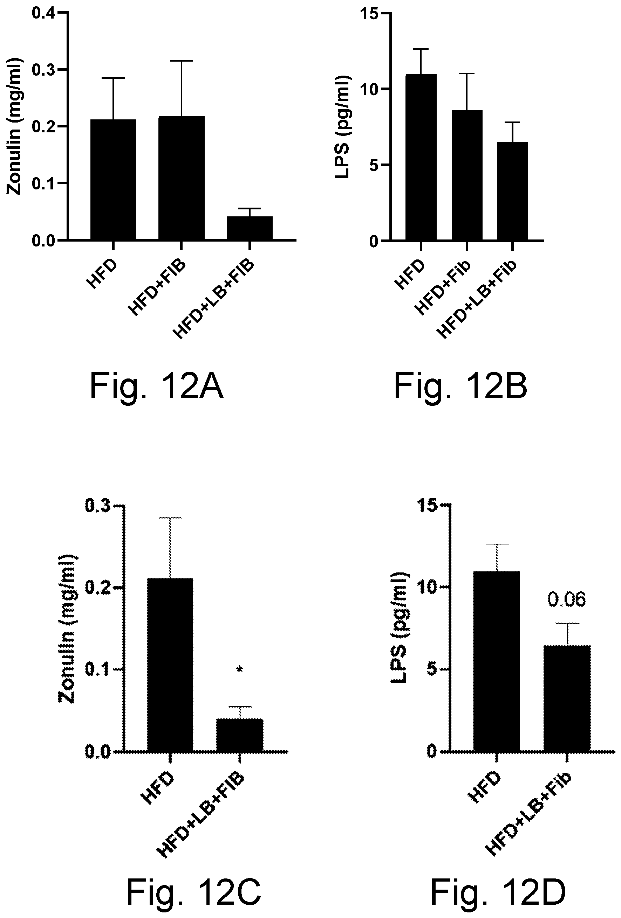

Bacteria and gluten have been identified as powerful triggers to release zonulin in the small intestine (PMID: 21248165). Zonulin is secreted by enterocytes in the small intestine, but also by adipose tissue, heart tissue, immune cells and liver cells in metabolic syndrome patients (PMID: 11082037, PMID: 24868498). An elevated level of zonulin in the serum of obese patients with non-alcoholic fatty liver disease has recently been demonstrated. Liver and adipose tissue in overweight subjects with or without liver steatosis may also release zonulin (PMID: 25493023, PMID: 11082037, PMID: 24868498). Fasting serum zonulin was not significantly different between the animal groups, however, LB+Fiber supplementation tended to reduce this marker compared to HFD fed MetS pigs (ANOVA p=0.3 Figure 12A, T-test p= 0.04 Figure 12C). A decrease in intestinal permeability associated with HFD has been correlated with an increase in the levels of circulating LPS in obese and metabolic disease patients (PMID: 27098727, PMID: 18815435, PMID: 26151336). The level of LPS in serum was reduced but not significantly so for the groups of HFD+Fiber and HFD+LB+Fiber diets (ANOVA p=0.4, Figure 12B). HFD+LB+Fiber supplementation reduced LPS the most compared to HFD-only MetS pigs (T-test p=0.06, Figure 12D).

Significant difference was calculated using T-test and/or ANOVA followed by Tukey's multiple comparisons test using PRISM

### Example 7 - Gut Satiety hormones - release profiles and SCFA

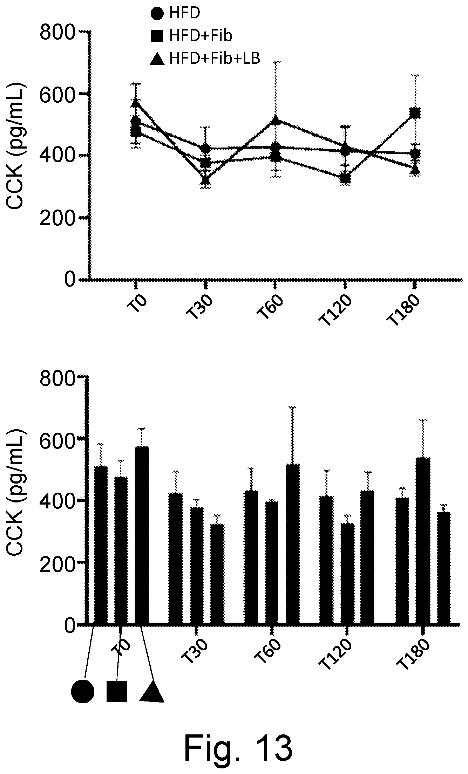

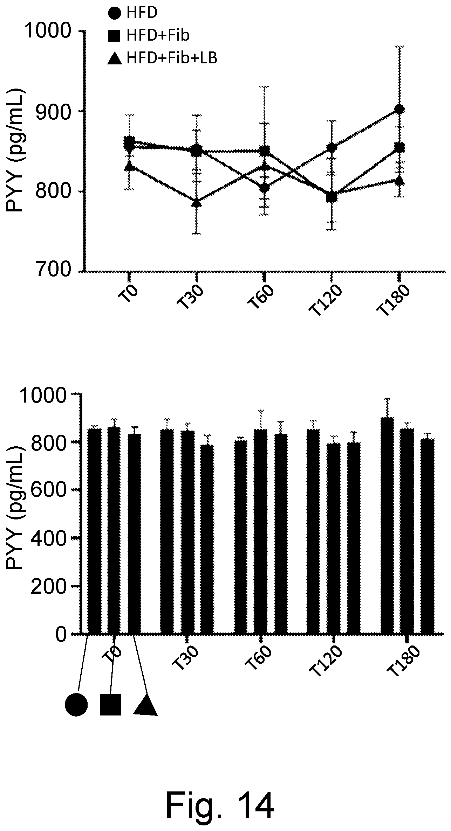

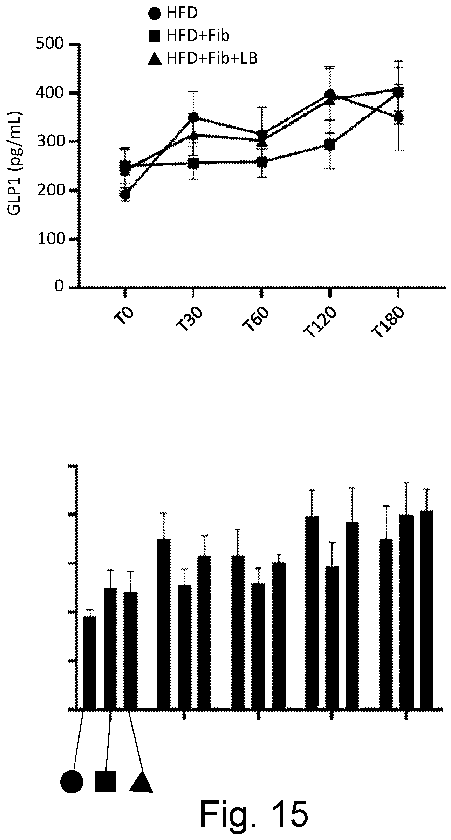

Fiber fermentation by gut microbiota produces short-chain fatty acids (SCFAs) in the terminal ileum and large intestine, and high fecal SCFA concentrations are associated with gut dysbiosis, permeability, and cardiometabolic risk factors (PMID: 30591685). SCFAs have also been shown to regulate satiety gut hormones in human studies, including increasing peptide YY (PYY) and glucagon-like peptide-1 (GLP-1) by binding L-cell receptors in the intestine (PMID: 12496283, PMID: 17899402, PMID: 16828127, PMID: 12954742, PMID: 24811133). Fiber enrichment of a meal induces a high level of postprandial cholecystokinin (CCK), decreases PYY responses and, depending on the type and amount of fiber, can increase, decrease or have no effect on GLP-1 responses. So, postprandial plasma concentration of CCK, PYY and GLP-1 after a glucose meal in animals in response to different diet supplementation over 12 weeks were assessed. Postprandial PYY (Figure 14), CCK (Figure 13) and GLP1 (Figure 15) responses was blunted in the groups of animals analyzed. In line with these results, human studies have suggested that obesity is associated with a blunted postprandial response of satiety factors such as GLP-1 and PYY, CCK (PMID: 17443025, PMID: 16022753, PMID: 26499438). Thus, LB and Fiber supplementation had a significant effect on postprandial gut hormone regulation in our pig MetS model. Significant difference was calculated using ANOVA followed by Tukey's multiple comparisons test using PRISM.

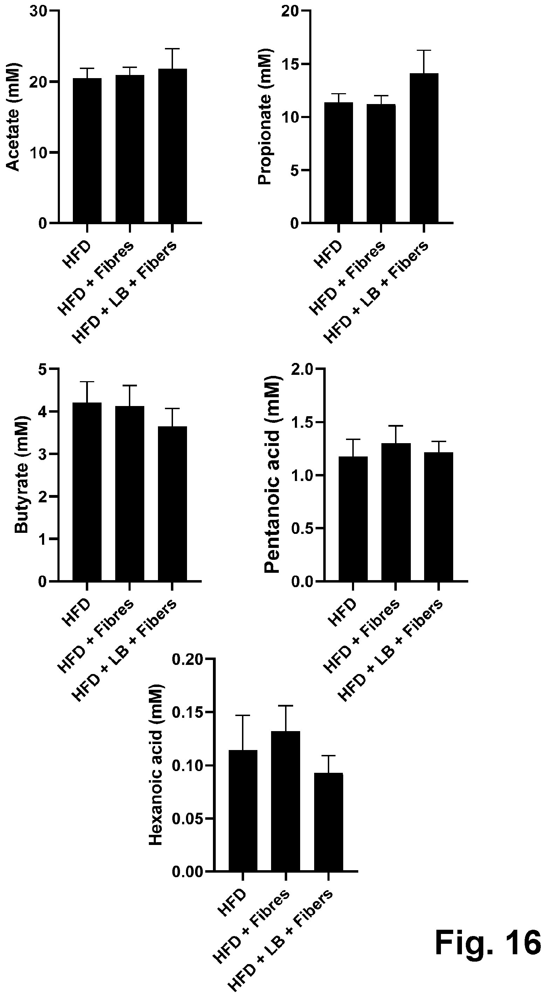

The concentration of SCFAs in serum of MetS pigs in response to different diet supplementation was also assessed. No significant difference in the SCFA profile between all treatment groups was detected for serum concentrations of acetate (Figure 16, top left), propionate (Figure 16, top right), butyrate (Figure 16, center left), pentanoic acid (Figure 16, center right) and hexanoic acid (Figure 16, bottom). Significant difference was calculated using ANOVA followed by Tukey's multiple comparisons test using PRISM.

These results indicate that administration of compositions of *L. mucosae +* soluble dietary fiber significantly decreases cardiovascular risk and liver damage in a manner that is independent of SCFAs.

### Example 8 - The gut microbiome - diversity and specific taxa

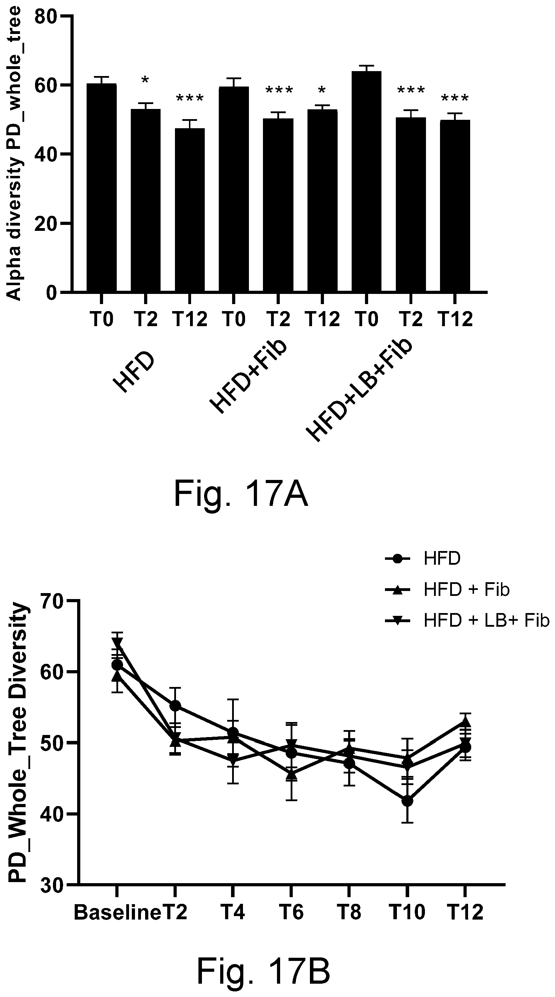

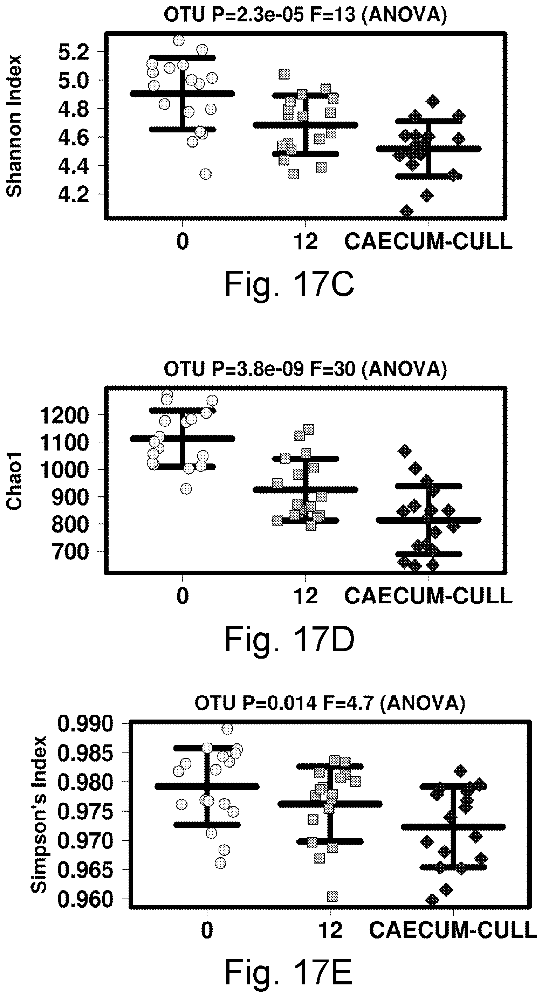

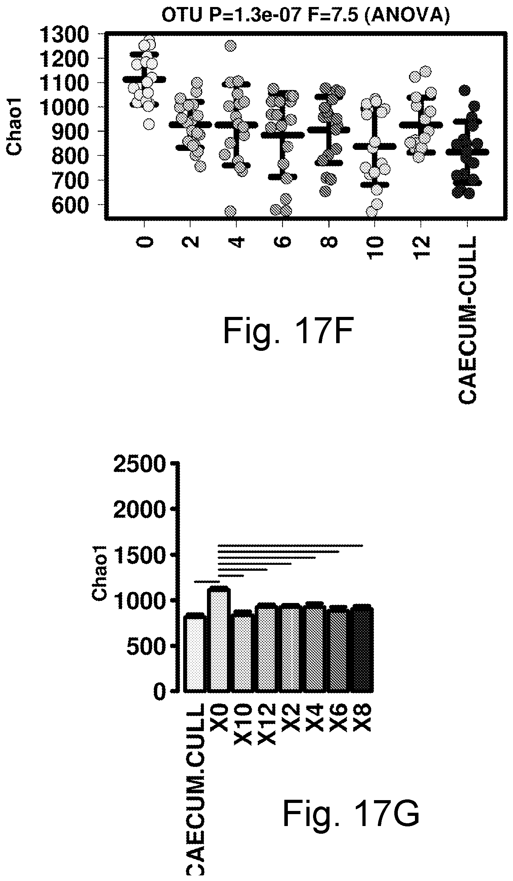

Alpha diversity is a measure of the diversity in a single ecosystem or sample. The simplest measure is richness, the number of species (or OTUs) observed in the sample. Alpha diversity was measured using Simpson, Chao1, Shannon, Phylogenetic Diversity (PD Whole tree) indices and observed species. Alpha diversity was significantly reduced in each of the groups at T12 compared to baseline T0 (Figure 17A: T0 vs T12 HFD p<0.0001, HFD+Fib p=0.02, HFD+LB+Fib p<0.0001. Figure 17C: Shannon p<0.001. Figure 17D: Chao1 p<0.001. Figure 17E: Simpson p=0.017). This effect was observed as early as two weeks in all 4 groups and was maintained throughout the subsequent 12-week study period (Figure 17A: T0 vs T2 HFD p=0.01, HFD+Fib p=0.0043, HFD+LB+Fib p<0.0001. Figure 17F and 17G: Chao1 p<0.0001). However alpha diversity was not significantly different between groups at each time point (Figure 17B). Community diversity represented by Shannon, Chao1 and Simpson's were at an OTU level compared between baseline (0) and 12 weeks (12) in fecal samples and 12 weeks in cecum samples ("Caecum-cull"), all diet confounded. Community diversity represented by Chao1 index and significant representation (Figure 17G) at an OTU level compared between time points all diets diet confounded. Significant difference was calculated using ANOVA followed by Original FDR method of Benjamini and Hochberg multiple comparisons test using PRISM or calypso software, P<0.05 (*) P<0.01 (**), P< 0.001 (***).

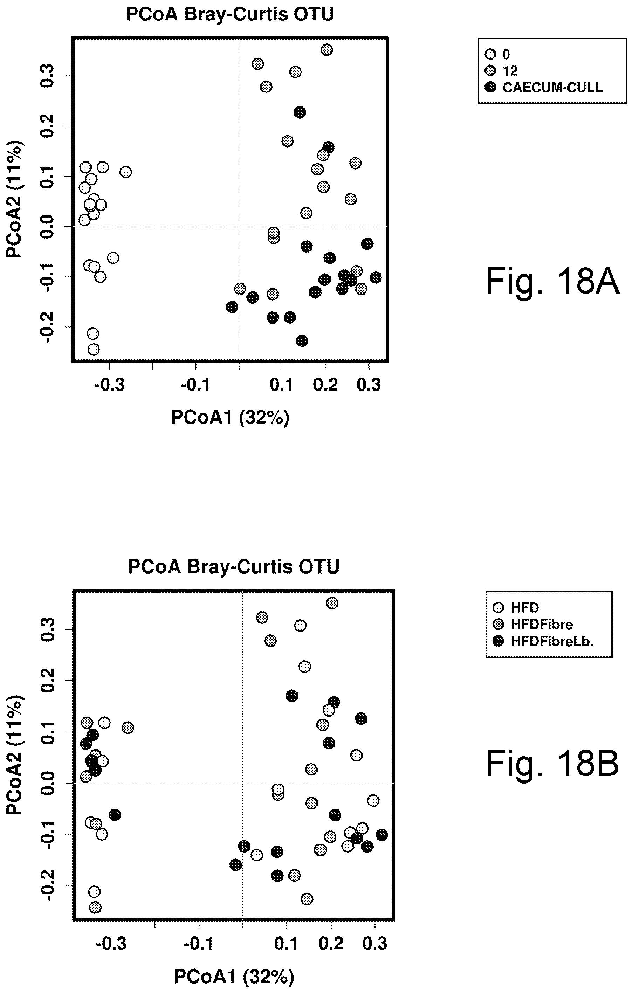

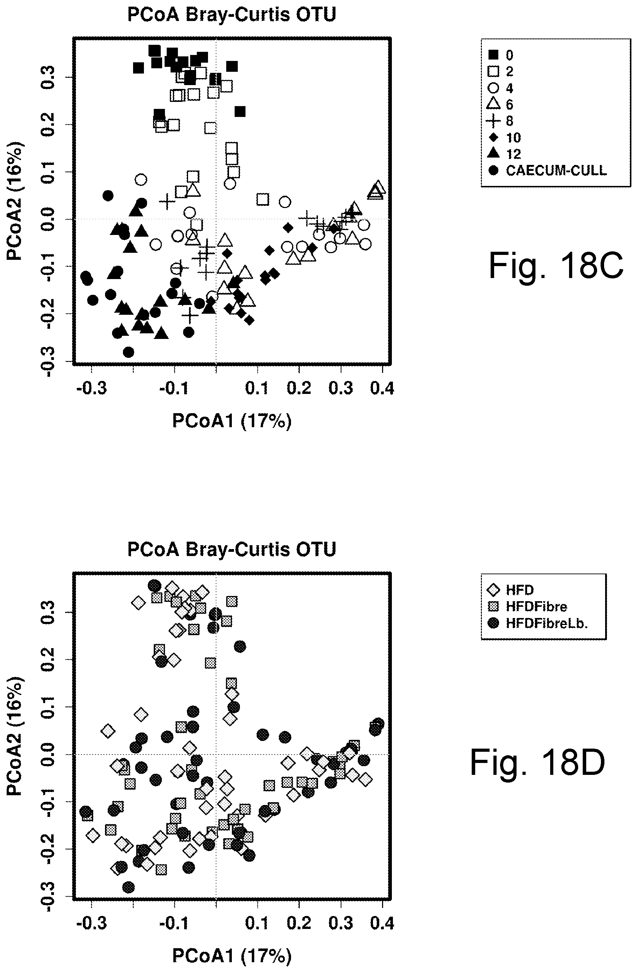

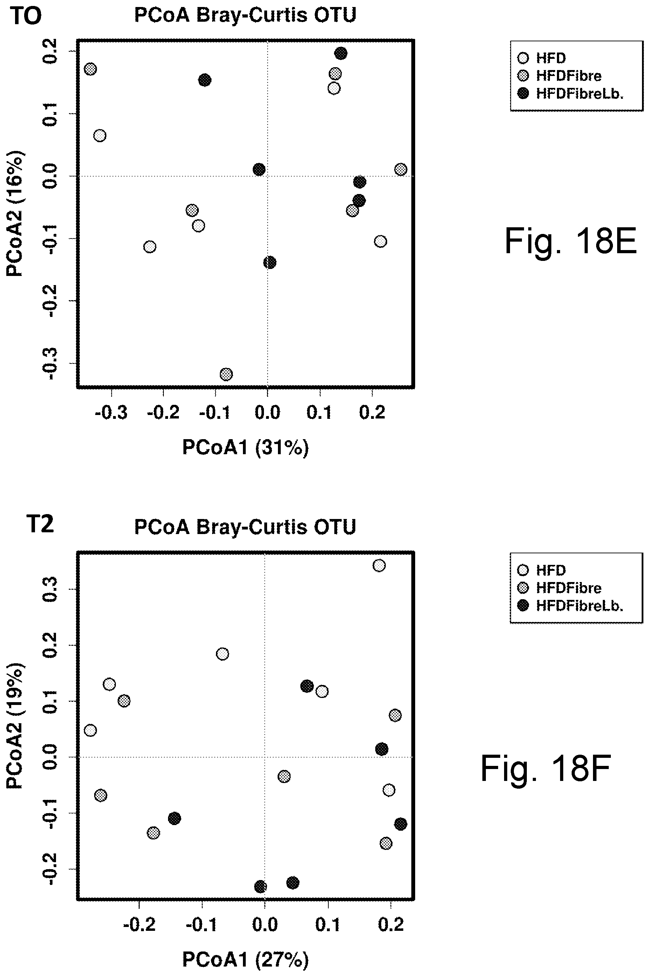

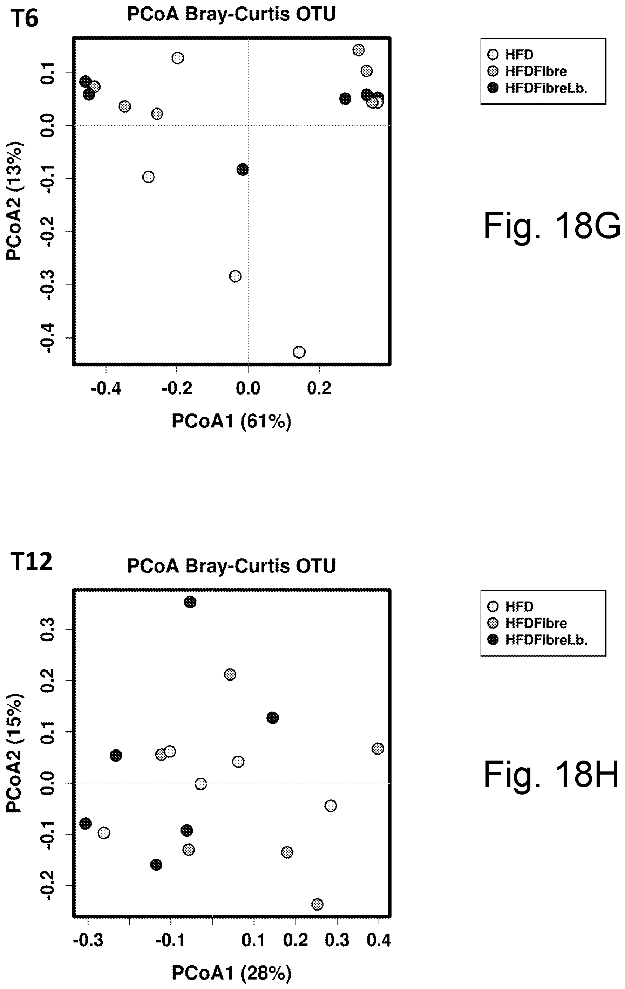

A principal coordinate analysis (PCoA) plot based on Bray-Curtis (beta diversity) distance matrices found distinct separation in microbiota between all animals at baseline compared to T12 (Figure 18A). This microbiota diversity separation was seen from T2 of feeding HFD and was maintained out to week 12 (Figure 18C). However, no difference in microbial diversity was observed at T2, T6 and T12 between the groups receiving different supplementation (Figure 18B, 18D, and 18E). We have shown in our previous experiments that this porcine model demonstrates a reduction in alpha diversity in MetS compared to the control pigs and a shift in beta diversity was observed at T2. The different supplementations of HFD did not demonstrate a significant effect on either alpha or beta diversity in our current study.

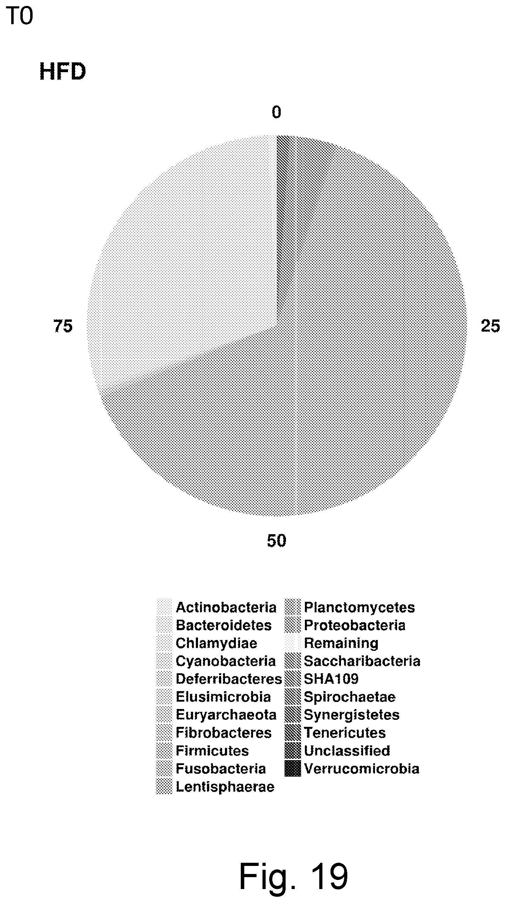

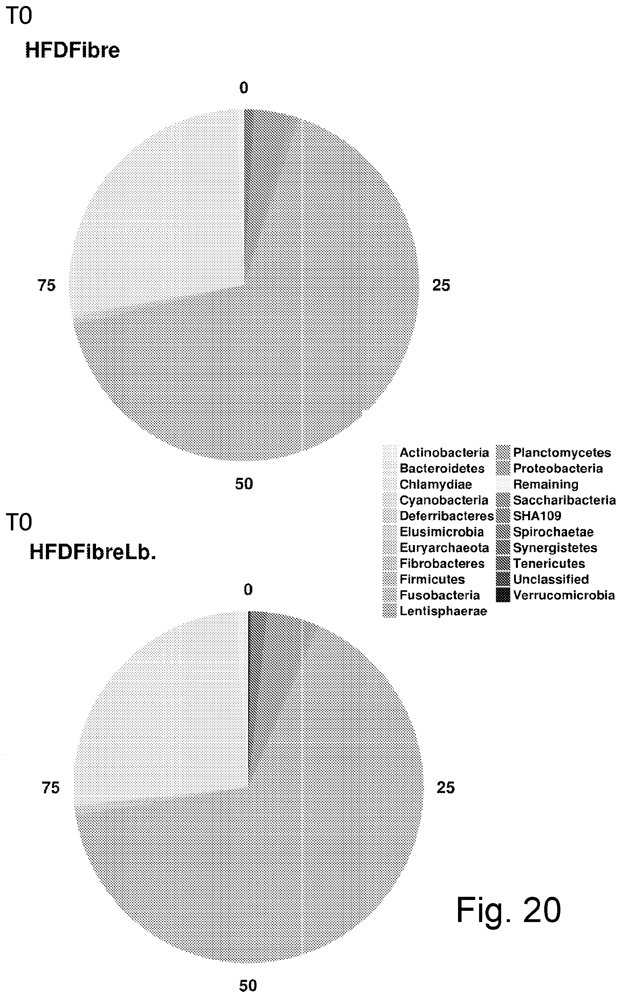

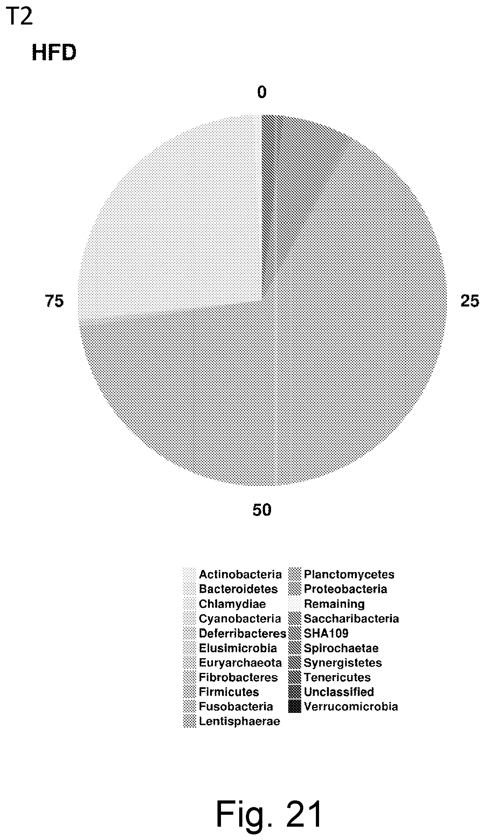

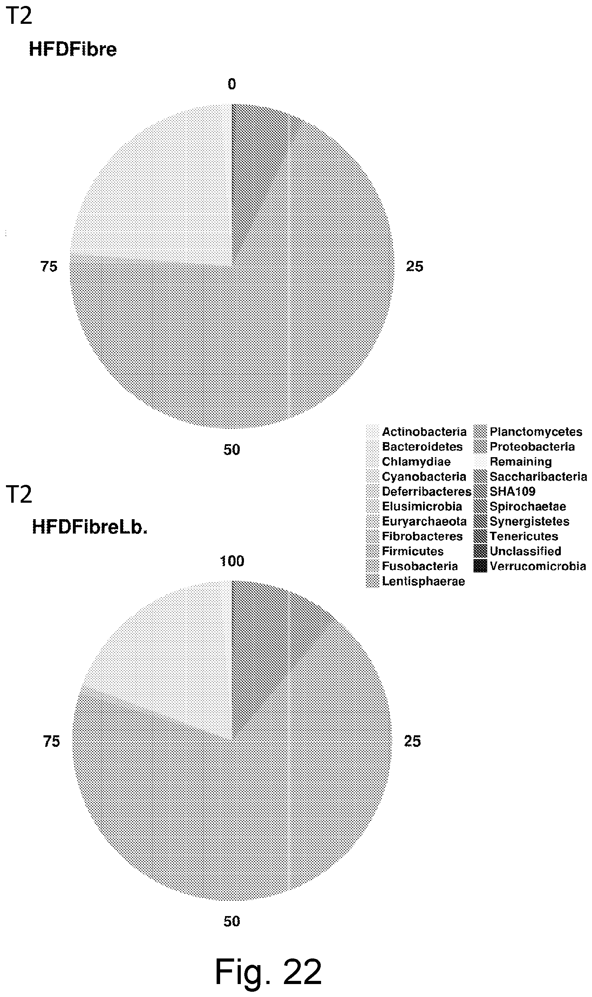

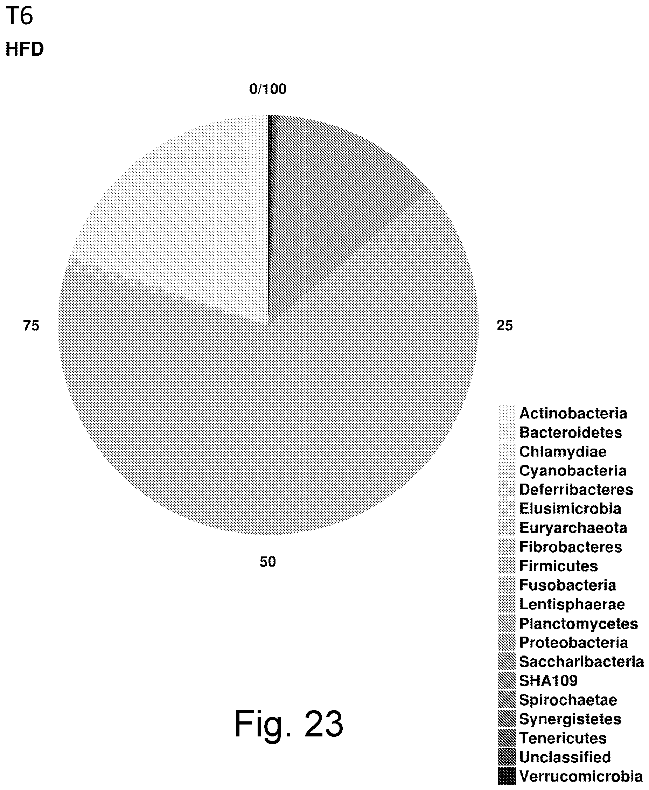

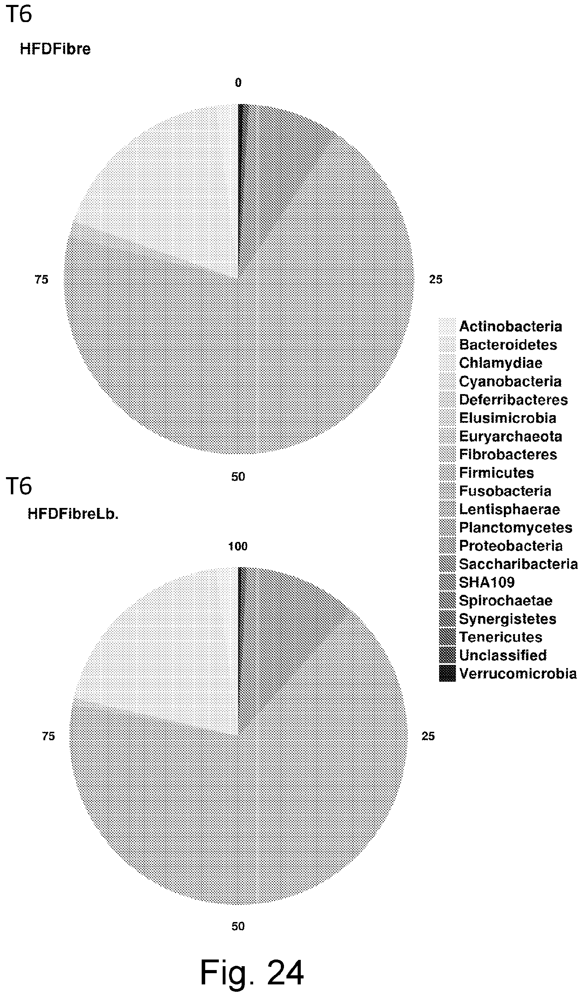

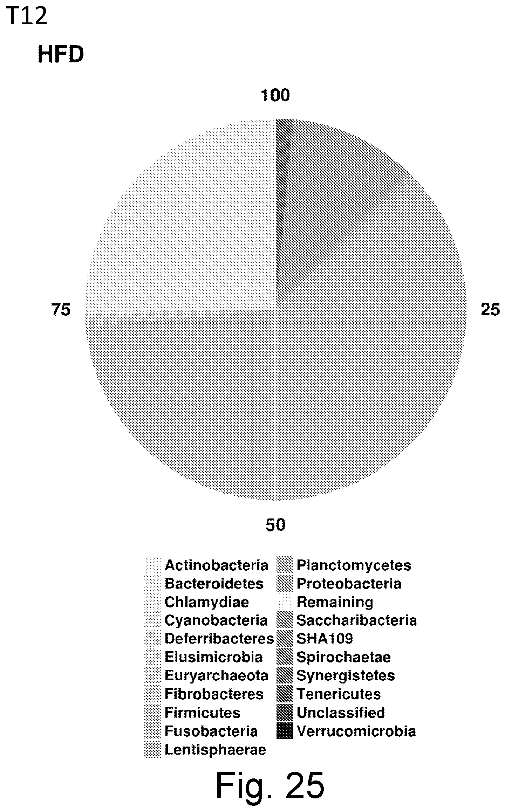

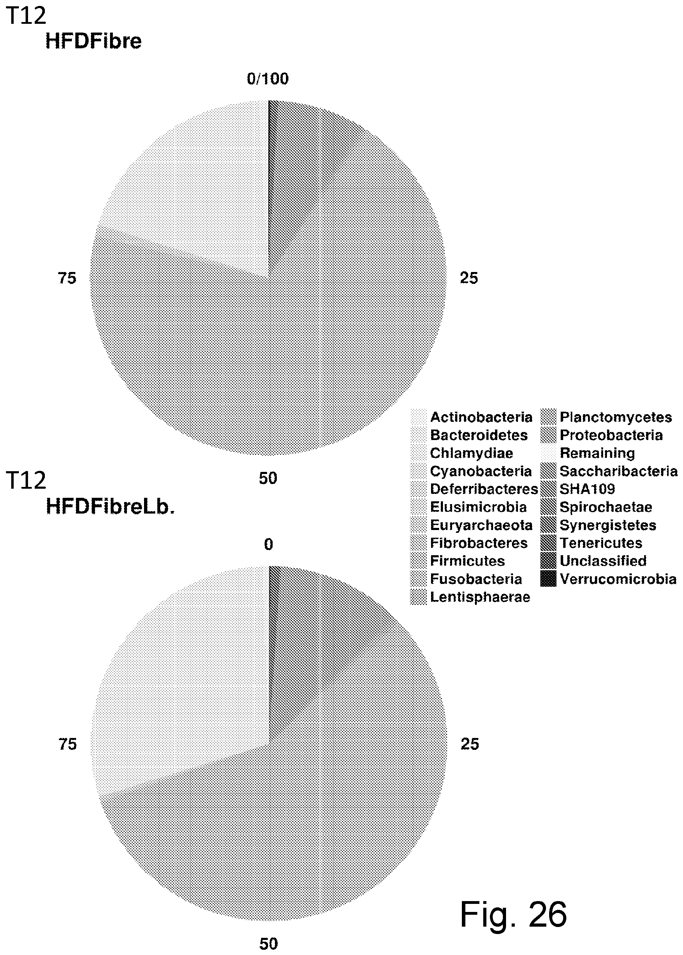

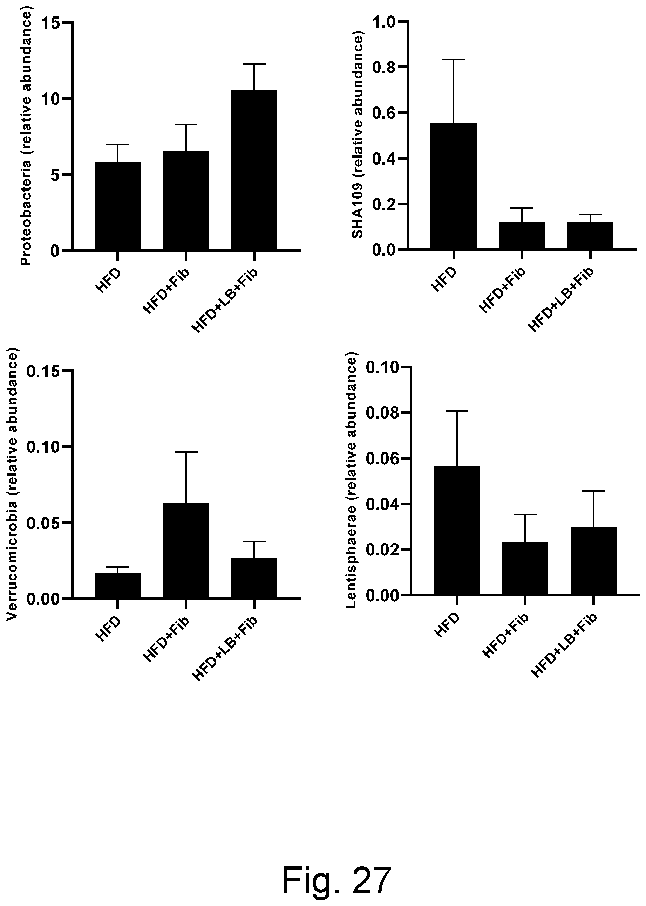

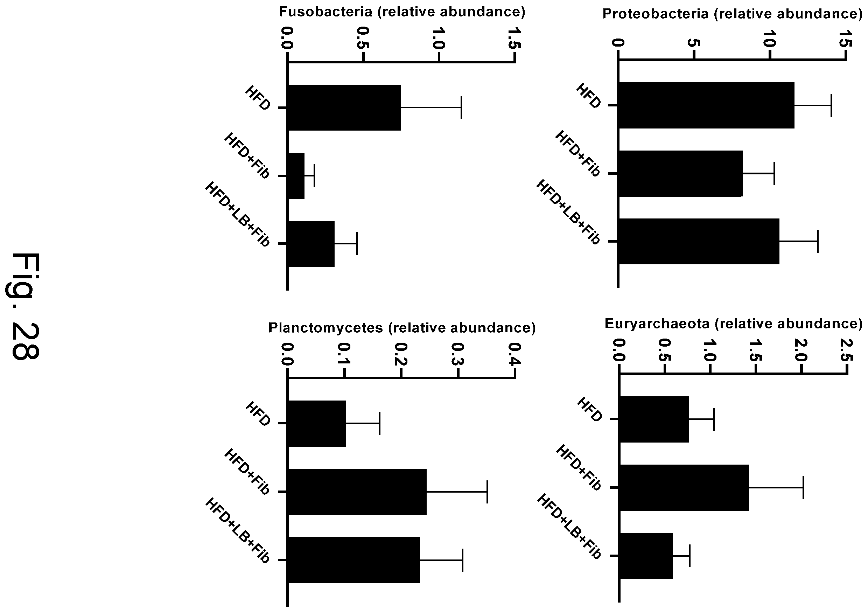

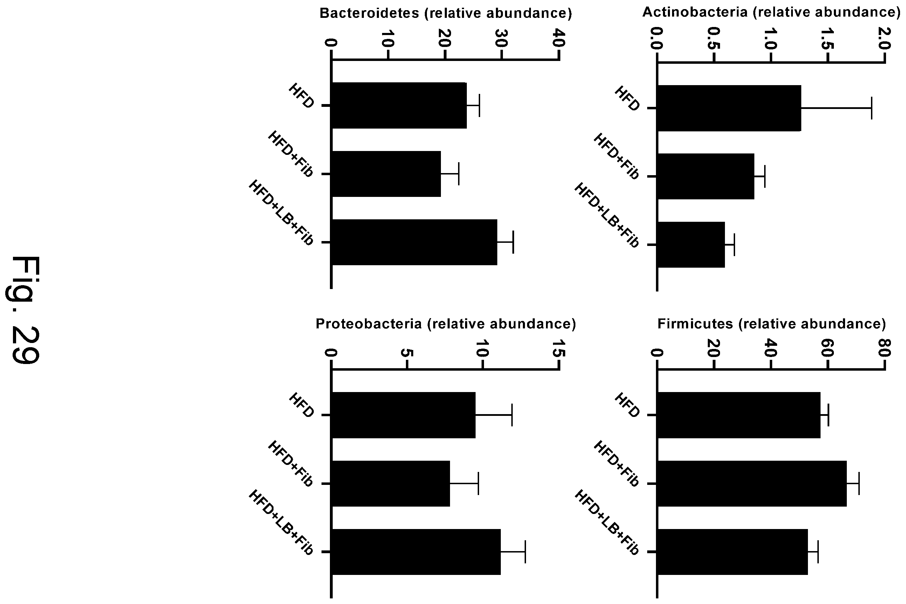

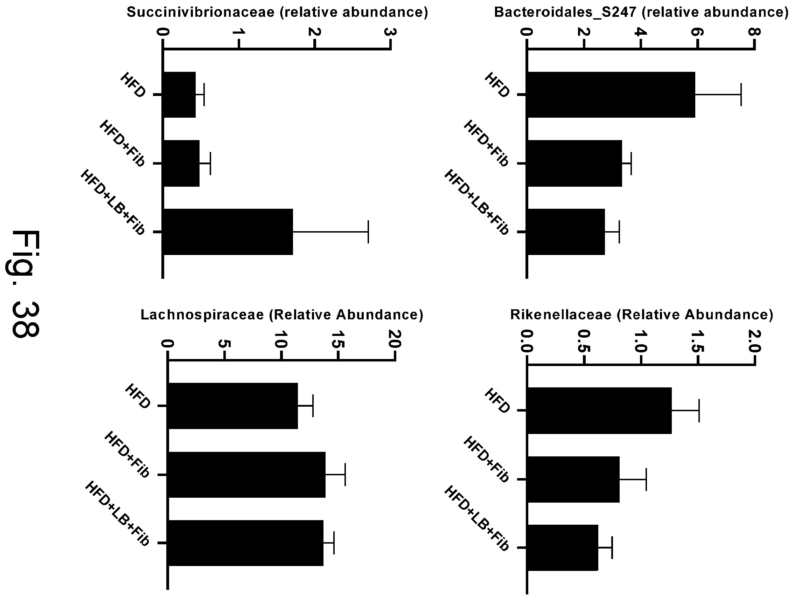

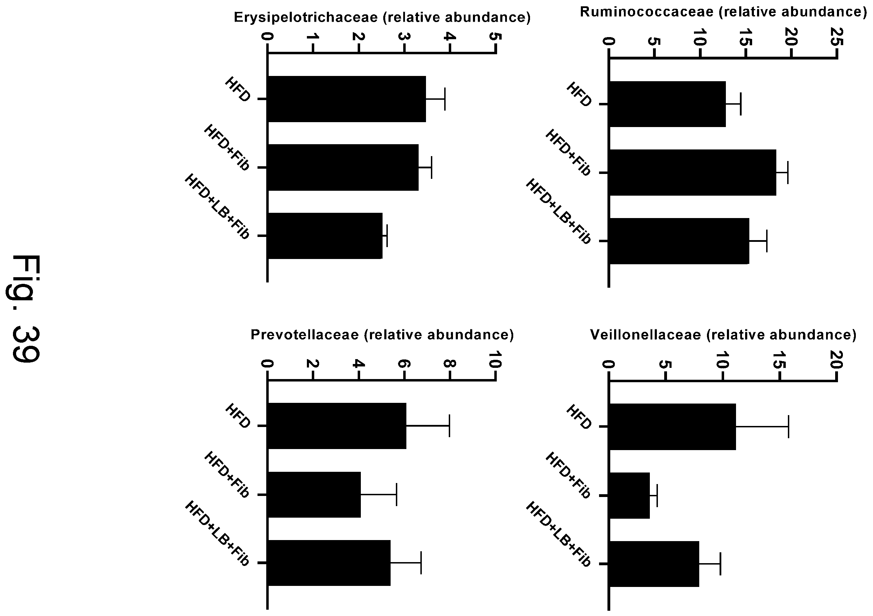

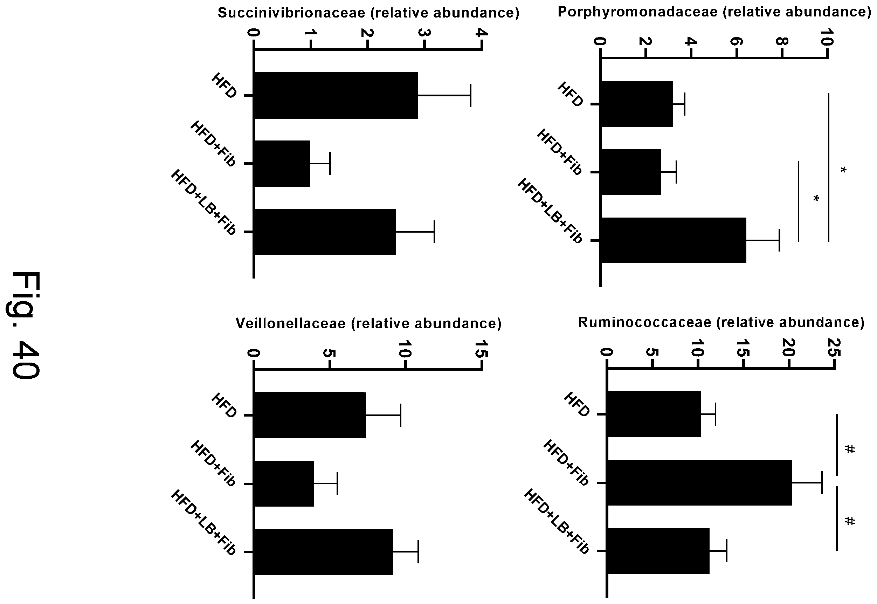

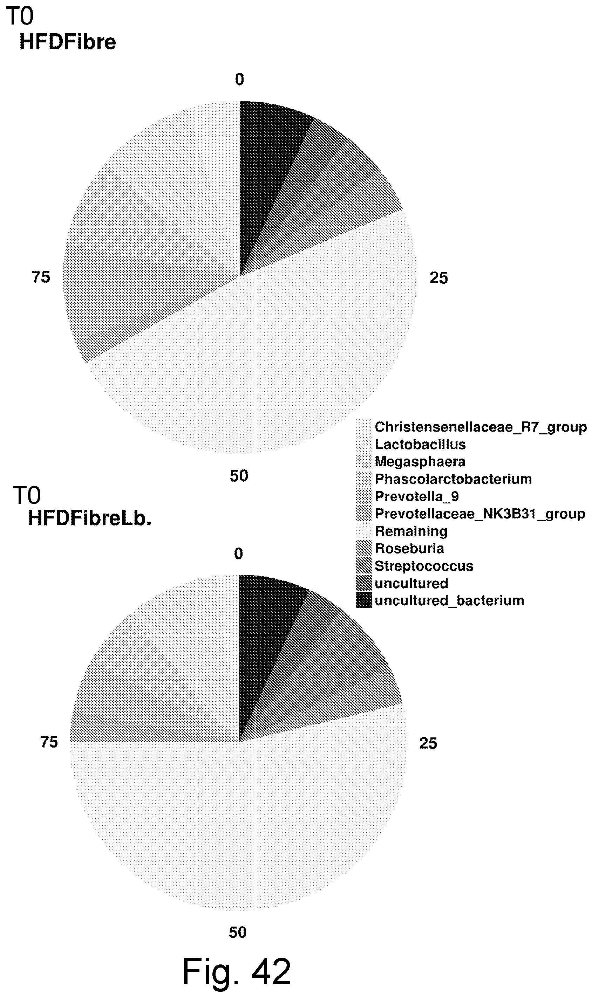

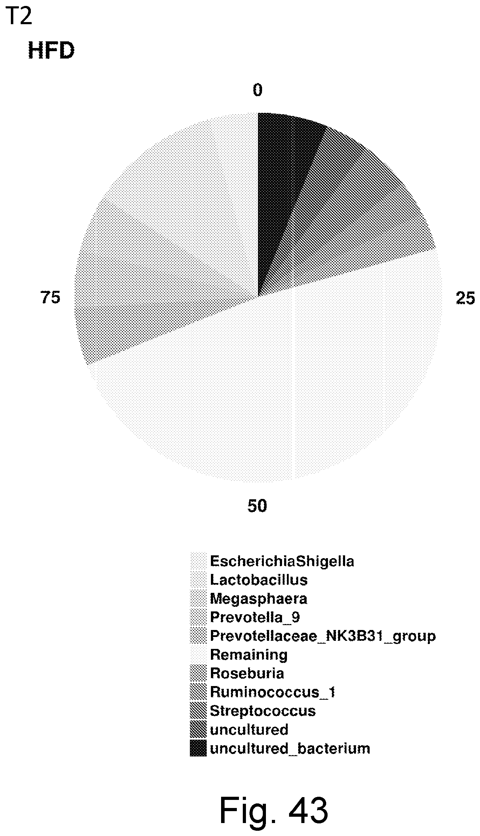

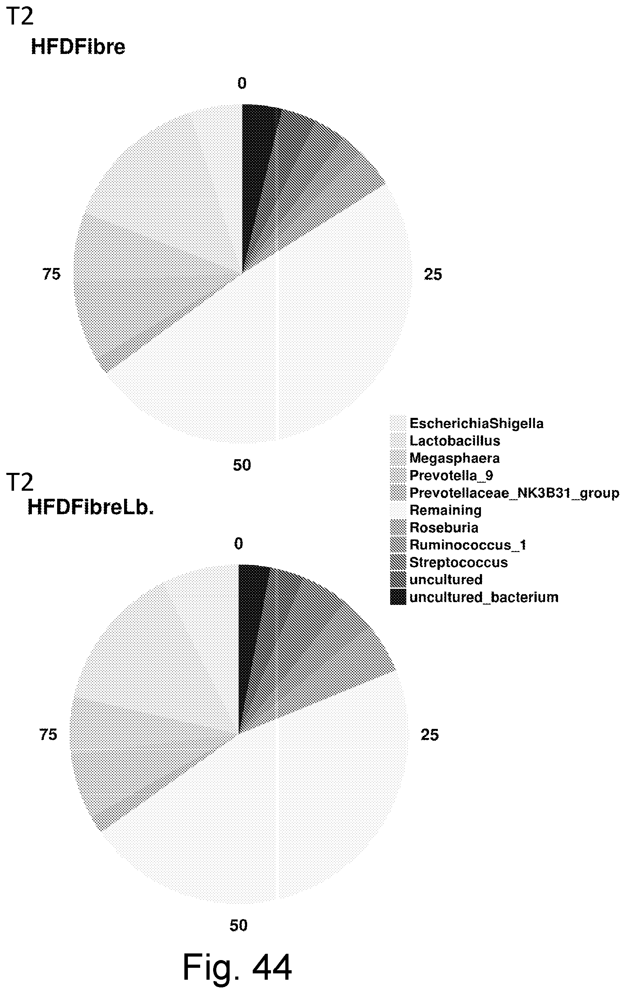

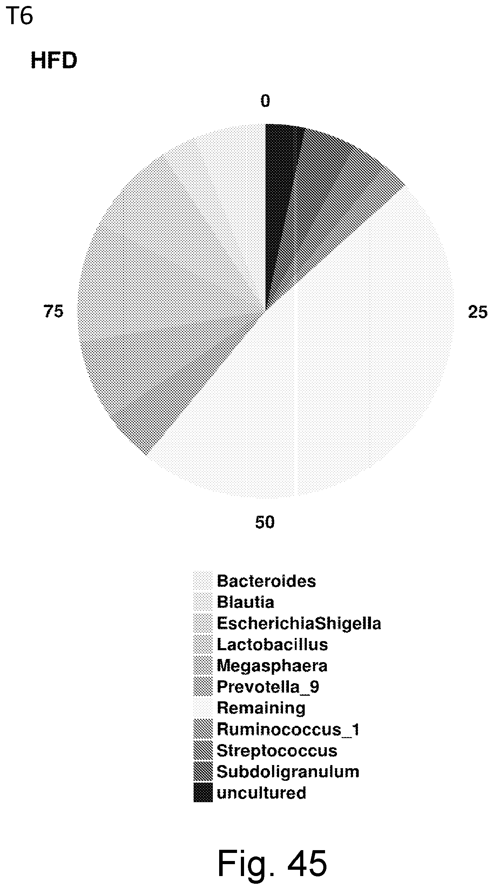

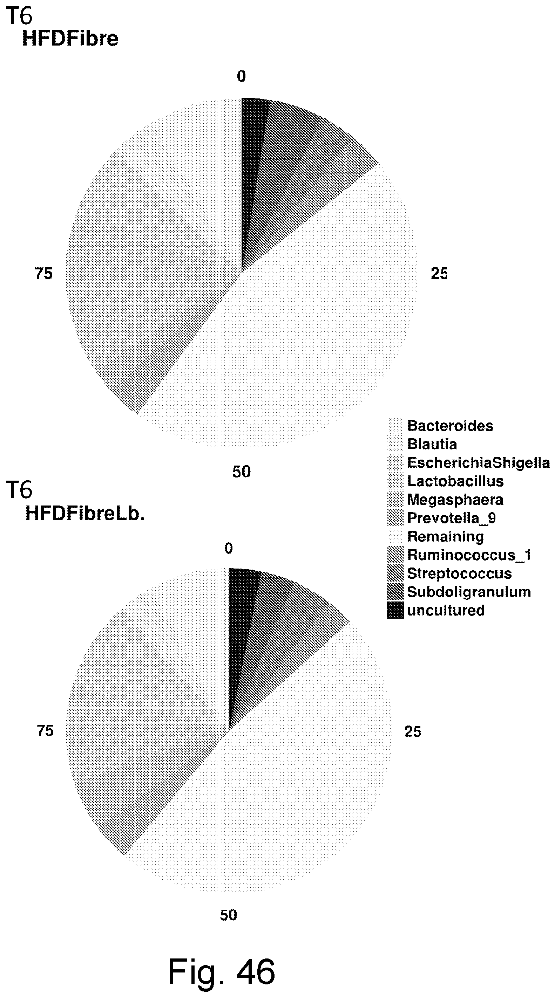

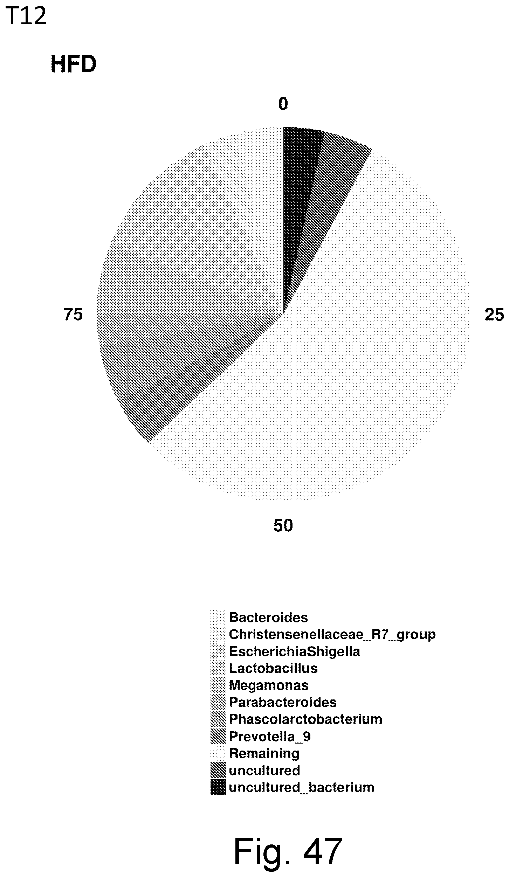

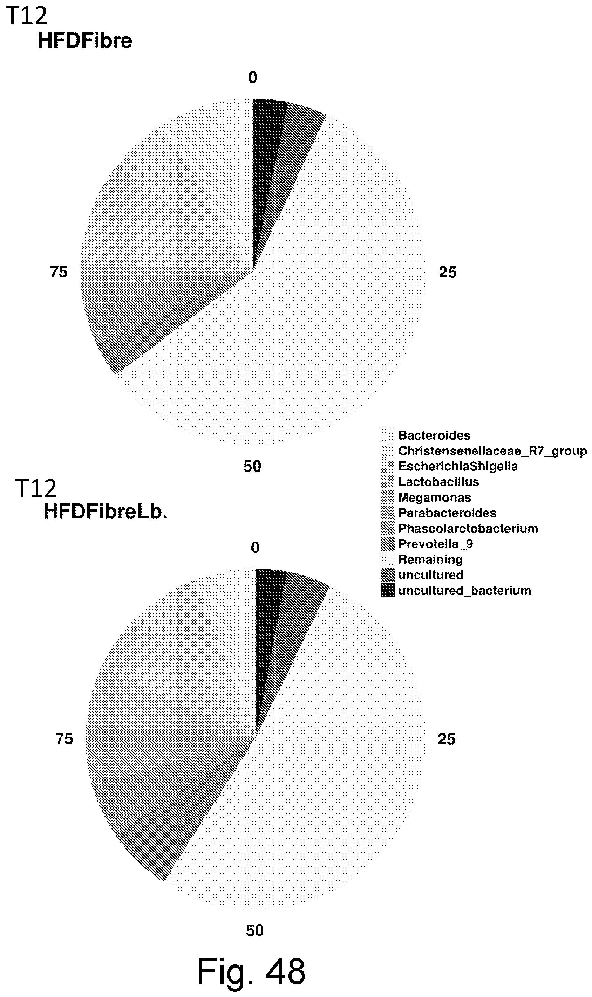

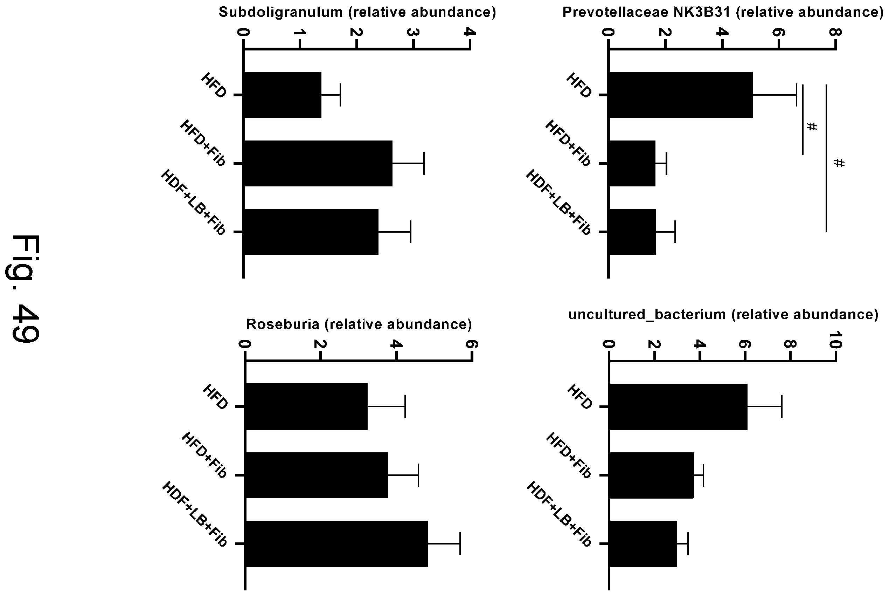

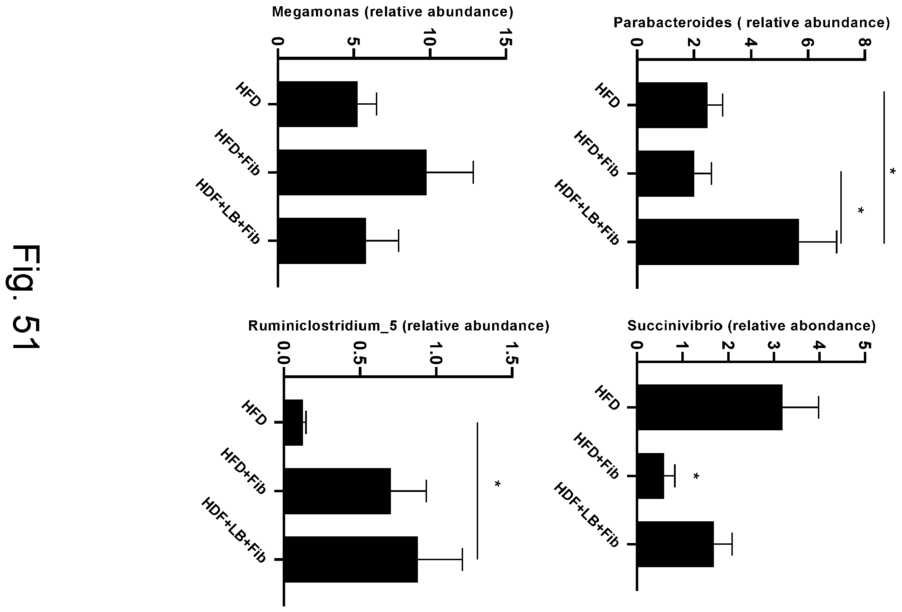

Microbiome analyses of the response to diet in the development of metabolic syndrome at phylum, family and genus levels showed no differences between groups at T0 (Figures 19-26 for phylum, Figures 42-49 for family and Figures 53-60 for genus; pie charts indicate the relative abundance of specified phyla, families, and genera in pig feces at Baseline, T2, T6 and T12 between diets). No predominant phyla at T2, T6 and T12 were significantly affected by the different diets (Figures 27-29). At the family level, there were no significant difference at T2 and T6 between the groups (Figures 38-40). However, at T12, Porphyromonadaceae was significantly increased in HFD+LB+Fiber compared to the 3 other groups (HFD+LB+Fib vs HFD p=0.04, HFD+LB+Fib vs HFD+LB p=0.04, HFD+LB+Fib vs HFD+Fib p=0.04, Figure 40, top left). Moreover, at T12, Ruminococaceae tended to be increased in HFD+Fib compared to HFD (p=0.06) and HFD+LB+Fib (p=0.06) (Figure 40, top right). At the genus level, there were three significant changes in gut microbiota at T12 (Figure 51). Parabacteroides was increased in HFD+LB+Fib compared to the other groups (HFD+LB+Fib vs HFD p=0.03, HFD+LB+Fib vs HFD+Fib p=0.03, Figure 51, top left). Succinivibrio was significantly decreased in HFD+Fib (p=0.037, Figure 51, top right). Ruminiclostridium 5 was significantly increased in HFD+LB+Fib compared to HFD (p=0.036) (Figure 51, bottom right). At two weeks post feeding, Prevotellaceae NK3B31 decreased in HFD+Fib and HFD+LB+Fib compared to HFD (p=0.02). Extended error bar plots identify significantly different bacterial families or genera between mean relative abundance in response to diet at T2, T6 and T12. Significant difference was calculated using ANOVA followed by Original FDR method of Benjamini and Hochberg multiple comparisons test using PRISM. P=0.06 (#) P<0.05 (*).

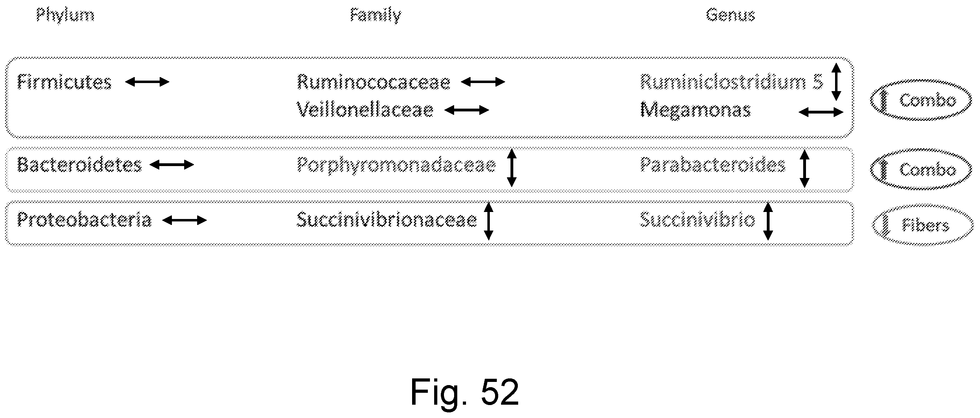

Certain of these data are summarized by the chart of Figure 52, which illustrates the relative abundance of certain Phyla, Families and Genera significantly increased or decreased (vertical arrow) or unchanged (horizontal arrow) in response to diet in the development of Metabolic Syndrome.

Relative abundance of Ruminiclostridium 5 have been shown to be positively correlated to the concentration of SCFA production (PMID: 29267960). Significant enrichment was observed herein in animals receiving LB + Fiber supplementation, though no significant difference was been observed in the concentration of fecal SCFA.

Porphyromonadaceae is a family belonging to the phylum of Bacteroidetes. Among others, Parabacteroides genera is under Porphyromonadaceae. Porphyromonadaceae relative abundance has been shown to be decreased after 10 days in a HFD mouse model (PMID: 26154056) and in intestinal inflammation conditions (PMID: 18005726). Parabacteroides supplementation has been shown to improve metabolic dysfunction in mouse models, and Parabacteroides distason significantly decreased weight gain, hyperglycemia, and hepatic steatosis in ob/ob and HFD-fed mice (PMID: 30605678). This bacteria via the production of succinate activates intestinal gluconeogenesis and via the production of bile acids the FXR pathway in the gut, resulting in the alleviation of obesity and NAFLD (PMID: 30605678). Parabacteroides has been negatively correlated with inflammatory bowel disease (IBD), and an increase of succinate in feces is associated with the regulation of intestinal inflammation and fibrosis via a succinate receptor 1 (SUCNR1) (PMID: 30583500, PMID: 30279517). Interestingly, animals receiving LB+Fiber supplementation had an increase in relative abundance of Porphyromonadaceae /Parabacteroides.

These microbiome findings positively reinforce the reduction in systemic and organ inflammation, lipid accumulation in the liver and correction of dysregulated FGF19 release described herein and indicate that supplementation with LB+Fiber may additionally modify succinate and bile acid production through increased abundance of Parabacteroides.

Fiber supplementation decreased the relative abundance of Succinivibrio, a succinate producing bacterium. It has previously been shown that a reduction of 4% protein level in diet increases the abundances of Succinivibrio, an essential bacterium involved in the degradation of starch and hemicelluloses (PMID: 32542194). Moreover, a significant enrichment of the genus Succinivibrio has been observed in Brazilian subjects with early obesity, hyperinsulinemia, and hyperlipidemia (PMID: 28814977). The abundance of Prevotellaceae NK3B31 group belonging to Bacteroidetes has been shown to be increased in response to a 2% reduction in the dietary protein in mice, which may in part explain the significant increase in pigs receiving HFD compared to the group of animals supplemented with fiber (PMID: 32542194). Prevotellaceae NK3B31 has been positively associated with type 2 diabetes mellitus in rats (PMID: 29487347). Prevotellaceae family has been associated with chronic inflammatory and mucin degradation diseases (PMID: 28542929 PMID: 22012254), and members of the Prevotella genus were previously described as succinate producers and associated with carbohydrate metabolism and energy production (PMID: 26224452, PMID: 27411015, PMID: 29434314). In summary, the porcine study described herein suggests soluble dietary fibers may decrease the abundance of Succinibrio, a bacteria significantly associated with obesity parameters.

The work described herein demonstrates that supplementation with soluble dietary fiber and *L. mucosae* DPC6426 has significant effects on gut microbes producing succinate. Supplementation increased bacteria associated with protective metabolic effects and decreased bacteria associated with obesity.

### Methods

***Study design and sampling:*** Three-month old Landrace female piglets, weighing 20-25kg were acquired and housed in individual stalls within the Biological Service Unit, University College Cork. Animals were separated into four groups: MetS, the control group (*n* = 6) was fed with high fat diet (HFD): 0.5kg/day Connoly's Redmills normal chow, supplemented with 1kg/day 4% salt, 4% cholesterol, 25% crude fat, 0.5% cholate and 26% sugar (high fat, salt and sugar diet-HFD, from SAFE, Augy, R8620 version 0011). The second group was fed HFD supplemented with 10¹⁰ cfu/day *L. mucosae* DPC6426 (n = 6). The third group was fed HFD supplemented with 43g/day Tate & Lyle's PROMITOR^{®} 70 Soluble Fiber (n = 6). The fourth group was fed with HFD supplemented with 10¹¹ cfu/day *L*. *mucosae* DPC6426 and 43g/day PROMITOR^{®} Soluble Fiber (n = 6).

All animals had free access to water at all times. In conjunction with the supplemented diet, the pigs underwent surgical implantation of a subcutaneous mineralocorticoid deoxycorticosterone acetate (DOCA) depot (200mg/kg, 90 days release pellets from Innovative Research of America) in the inguinal region prior to trial initiation to induce sodium and water retention, ultimately resulting in hypertension. Pigs were fed with the aforementioned respective diets over a 12-week period. Fecal samples were collected for a total of three time points: at baseline prior to initiation of study diet (Baseline - n=6 per group), as well as six weeks (T6 - *n*=6 per group) and 12 weeks (T12 - *n*=6 per group) post diet commencement. Following 12 weeks of feeding test diets, pigs were fasted for 24 hours, at which point body mass, blood pressure and hemodynamic parameters were measured. Pigs were administered with propofol (3 mg/kg i.v.), intubated with 1-2% isoflurane (Vetflurane, Virbac) to maintain anesthesia while echocardiographic and hemodynamic examinations were performed. ECG, non-invasive blood pressure and oxygen saturation were continuously monitored throughout hemodynamic study. Once cardiac parameters were completed, animals were sacrificed via bolus IV injection of pentobarbitone (50mg/kg, Dolethal, Vetaquinol). Blood and organs were subsequently collected from the pigs, while fecal samples were collected throughout the study (at T0, T6 and T12). Whole blood was collected into serum clot activator tubes and allowed to clot by leaving specimens undisturbed at room temperature. After 15 minutes, the clot was removed by centrifugation at 2,000 x g for 10 minutes in a refrigerated centrifuge and the resulting supernatant was stored at -80°C until usage. Organs were weighed and statistical comparisons calculated between groups. Fecal samples were stored at 4°C for a maximum of 2 hours prior to bacterial DNA extraction.

***Echocardiographic examination of the heart:*** Echocardiographic measurements were performed according to the recommendations of American Society of Echocardiography guideline using an ultrasound diagnostic system (*Vivid S5*, GE Medical Systems, USA) equipped with a 1.7/3.3 MHz probe. All studies were performed with pigs lying in the left lateral decubitus position while echo variables were measured three times with average values recorded. Left atrial area, left ventricular septum, and posterior wall thickness were measured using 2D image tracings on parasternal long-axis and short-axis views.

***Blood measurements:*** Fasted serum levels of total cholesterol (TC), low density lipoprotein (LDL), triglyceride (TAG), glucose and insulin, FGF19 (Fibroblast growth factor 19), TNF-alpha, Zonulin and LPS were quantified at T12, according to manufacturer's instructions with the following kits: a cholesterol assay kit - TC and LDL/VLDL (ab65390, Abcam, Cambridge, MA); Triglyceride Assay Kit - Quantification (ab65390, Abcam, Cambridge, MA); Maltose and Glucose Assay Kit (ab65335, Abcam, Cambridge, MA); Insulin ELISA assay (Mercodia AB, catalog number 10-1256-01, Sylveniusgatan 8A, SE-754 50, Uppsala, Sweden); pig fibroblast growth factor 19 (FGF19) ELISA Kit (EKC38180, Biomatik, DE, United States); a swine TNF-alpha ELISA Kit (KSC3011, Invitrogen, Invitrogen Corporation, Carlsbad, USA); pig haptoglobin ELISA Kit (ab205091, Abcam, Cambridge, MA); and human lipopolysaccharides, ELISA kit (EKC34448, Biomatik, DE, United States), respectively. Glucose, insulin, PYY, CCK and GLP-1 serum level were quantified at different time point: at fasting 0 (approximatively 24 h) and at 30, 60, 120 and 180 minutes after pigs ingested a meal composed of: 1.5g glucose/kg pigs and 0.5kg regular diet. The quantification was made according to manufacturer's instructions with Maltose and Glucose Assay Kit (ab65335, Abcam, Cambridge, MA), Insulin ELISA assay (Mercodia AB, catalog number 10-1256-01, Sylveniusgatan 8A, SE-754 50, Uppsala, Sweden), Pig Peptide YY (PYY) ELISA Kit (EKC41864, Biomatik, DE, United States), Pig Cholecystokinin (CCK) ELISA Kit (EKU03134, Biomatik, DE, United States), Human GLP1 (7-36) ELISA Kit (ab184857, Abcam, Cambridge, MA), respectively.

***Immunofluorescence** -* ***Inflammation and fibrosis in heart and liver*:** A small portion of LA, LV and liver tissue was collected and embedded with optimal cutting temperature compound (OCT) (ThermoFisher scientific, Ireland) for cryostat sectioning at 5-µm thickness. The sections were mounted on microscope slides and frozen at -80°C. Slides were fixed in methanol for 10 minutes at 4°C or 4% paraformaldehyde at RT for 20 minutes, incubated for 1h at RT in blocking buffer (10% serum in PBS-Tween) and incubated with the primary antibody (diluted in 1% Bovine serum albumin and PBS-Tween) overnight at 4°C for CD163 for macrophages (mouse anti pig CD163 IgG1, 0.1mg, BIORAD, 1:1000) or aSMA for myofibroblast (mouse anti-pig aSMA IgG1, 0.1mg, Abcam 1:500). The slides were washed in PBS three times for 5 mins and were then incubated with the secondary antibody (goat anti-mouse IgG Alexa Fluor 488, 2mg/ml, Invitrogen, 1:1000, diluted in 1% Bovine serum albumin and PBS-Tween) for 1h at RT in the dark. After washings, slides were stained with DAPI and coverslipped using mounting media. Liver and heart samples from 6 pigs per group were prepared, stained and 10 fragments from each tissue were further analysed. Sections were observed with a confocal laser microscope (Nikon D-Eclipse) using 60x magnification, and pictures were taken. Pictures were analysed using NIS-Elements BR 3.0 software. The 10 fragments for a given tissue were combined and averaged for one value for the animal.

***Liver histologic and lipid analysis*:** A small portion of liver tissue was collected and embedded with optimal cutting temperature compound (OCT) (ThermoFisher scientific, Ireland) for cryostat sectioning at 5-µm thickness. The sections were mounted on microscope slides and frozen at -80°C. The samples were subsequently thawed, fixed with 4% paraformaldehyde solution (PFA) (Sigma Aldrich, Ireland) for 10 minutes and stained with Oil Red O (ORO, Sigma-Aldrich, St. Luis, MO, United States) to detect hepatic lipid droplets. Liver samples from 6 pigs per group were prepared, stained and 3 fragments from each liver were further analysed. Each section was photographed with bright field microscopy (Olympus BX43 microscope, Tokyo, Japan) using 40X magnification. The amounts of lipid droplets were quantified in at least six different high-power fields for each fragment using Nikon NIS-Elements BR 3.0 software. Results are presented as number of lipid droplets per pixel per µm². Lipids in liver were analysed by ANOVA using PRISM (GraphPad Software, Inc.).

***Alcian blue staining*:** A small portion of terminal ileum tissue was collected and chemically fixed using methacarn solution for 2h, then transfer to 70% ethanol overnight. Samples were dehydrated by increasing concentrations of ethanol (70% -100%, 6h) followed by ethanol: histolene (45 min), histolene (4h), Paraffin (6h) using a Hystokinette. Then samples were infiltrated with solidified paraffin wax. Paraffin blocks were cut in sections at 5-µm thickness using a rotary microtome (Leica RM2135) and mounted on microscope slides. Serial sections were deparaffinized with xylene (10 min) and rehydrated by descending concentrations of ethanol (100% - 70%, 3 min) and rinsed in running tap water. Slides were then dipped in 3% acetic acid (3 min) and stained in Alcian Blue solution (Alcian Blue 8GX in 3% acetic acid (pH 2.5)- 30 min), briefly rinsed in 3% acetic acid and washed in running tap water. Slides were treated with 0.5% periodic acid (5 min) and washed in running tap water before being stained with Schiff's reagent (10 min), washed in running tap water and counterstained in 0.1% nuclear fast red solution (5min). Slides were then placed in 95% and 100% ethanol before being mounted in DPX mounting glue to coverslip the tissues. Terminal ileum samples from 6 pigs per group were prepared, and at least 30 frames per animal were further analysed. Sections were observed with a bright field microscope confocal laser microscope (Olympus BX43) using x20 magnification and pictures were taken. Pictures were analysed using NIS-Elements BR 3.0 software.

### Microbiome analysis:

***DNA Extraction:*** Bacterial genomic DNA was extracted from 0.25g of fecal material using the repeat bead beating plus column (RBB+C) protocol as described by Yu and Morrison, (2004), in combination with the QIAmp Fast DNA Stool Mini Kit (Qiagen, UK) described by Fouhy et al, (2015) with slight modifications: 1.0mm and 2.3mm sterile zirconia beads were used during the homogenizing step instead of 0.5mm sterile zirconia beads, in order to prevent sedimentation of the 0.1mm beads and the fecal material. The DNA was eluted with 200µL of Buffer AE (Qiagen) and stored at -80°C. (PMID: 15152600, PMID: 25748176, PMID: 28095889)

***16S rRNA gene sequencing and analysis*:** Following DNA extraction, the V3-V4 regions of the 16S rRNA gene were amplified through 30 cycles of PCR reactions on the template DNA using the primer pair 5'-TCGT CGGC AGCG TCAG ATGT GTAT AAGA GACA GCCT ACGG GNGG CWGC AG-3' and 5'-GTCT CGTG GGCT CGGA GATG TGTA TAAG AGAC AGGA CTAC HVGG GTAT CTAA TCC-3', according to the 16S Metagenomic Sequencing preparation protocol for Illumina MiSeq and as previously described by Fouhy et al, (2015) ( PMID: 25748176) Indexed products were visualized using gel electrophoresis and cleaned with AMPure XP magnetic based beads prior to DNA quantification using the Qubit (BioSciences, Dublin, Ireland), along with the Qubit High Sensitivity DNA kit (Life Technologies). Samples were pooled at equimolar concentrations and high-throughput sequencing was completed on the Illumina MiSeq platform in the Teagasc sequencing facility using a 2 x 300 cycle kit, following standard Illumina sequencing protocols.

***Bioinformatic analysis*:** Resulting 300-bp paired-end reads were assembled using FLASH (fast length adjustment of short reads to improve genome assemblies). QIIME (QIIME: Quantitative Insights into Microbial Ecology; version 1.8.0) was used for quality filtering of pair-end reads, which is based on a quality score of >25 and the removal of mismatching barcodes (PMID: 20383131). Prior to the analysis as part of the quality control process, sequences which produced less than 40,000 reads were manually removed, which accounted for the difference in the number of animals between groups and between time points. USEARCH (version 7, 64-bit) was employed for deionization, chimera detection and clustering into operational taxonomic units (OTUs; 97% identity). Alignment of OTUs was carried out using PyNAST (python nearest alignment space termination) and assignment of taxonomy of 97% similar identity against the SILVA SSURef database release v123.

***Statistical Analysis*:** Data were presented as mean ± SEM and analysed by an ANOVA test using PRISM (GraphPad Software, Inc.), where p < 0.05 was used to reject the null hypothesis. Estimates of alpha diversities were generated with QIIME and visualized through principal co-ordinate analysis (PCoA) plots generated using the web-based software Calypso (version 8.72). Multiple beta diversity metrics were also calculated, including Bray-Curtis, UniFrac PCoA plots, highlighting the alterations based on diet and time point. In order to assess phylum, family and genus level changes in the pigs, all significant taxa analysed by ANOVA, were graphically represented as relative abundance with the mean ± SEM, p values, confidence intervals. All significant taxa analysed by an ANOVA test using PRISM (GraphPad Software, Inc.) were adjusted by a false discovery rate (FDR) q value less than 0.05 was considered statistically significant.

### Determination of molecular weight of soluble dietary fiber

Samples are dissolved in GPC eluent (aqueous 0.1 N NaNO₃, 1 mM NaN₃, 0.4% methanol flow rate marker) at 50 mg in 10 g total eluent at room temperature with stirring. Solutions are filtered through 0.45 µm nylon syringe filters directly into GPC autosampler vials. All samples are injected in duplicate with 50 µL injections. GPC analysis was performed at 70°C on two Waters Ultrahydrogel 250Å and 150Å, 7.8 x 300 mm columns plus 7.8 x 50 mm guard at 1.3 mL/min with aqueous 1 N NaNO₃, 1 mM NaN₃. Third order regression fit is applied to flow marker corrected retention time versus log M from a series of narrow standard pullulans ranging from 180 to 404,000 Da Mp. Results are reported as pullulan equivalent molecular weight.

## Claims

1. A composition comprising one or more strains of *Lactobacillus mucosae* and one or more soluble dietary fibers, wherein the soluble dietary fiber has a linkage pattern comprising:
29-45% terminally-linked glucopyranosyl residues;
10-22% 6-linked glucopyranosyl residues;
13-27% 4-linked glucopyranosyl residues;
2-11% 3-linked glucopyranosyl residues;
3-13% 4,6-linked glucopyranosyl residues;
1-5% 3,6-linked glucopyranosyl residues;
0.5-4% 2,4-linked glucopyranosyl residues;
wherein the soluble dietary fiber has a fiber content of at least 70%, and wherein the linkage pattern is determined by the method described in the description; and
wherein one of the one or more strains of *Lactobacillus mucosae* is *L. mucosae* DPC6426.

2. The composition of claim 1, wherein at least one of the one or more strains of the *L*. *mucosae* expresses an exopolysaccharide comprising monosaccharide residues xylose, fucose, mannose, glucose, galactose, N-acetylglucosamine, and N-acetylmannosamine, wherein a ratio between the number of xylose, fucose, mannose, glucose, galactose, N-acetylglucosamine, and N-acetylmannosamine monosaccharide residues is about 1.3:0.5:10.0:4.8:3.3:1.5:0.2.

3. The composition of claim 1, wherein the one or more strains of *L. mucosae* are present in an amount of 10⁵ to 10¹⁵ colony forming units (cfu)/g, and/or wherein the soluble dietary fiber has a weight-average molecular weight in the range of 1000 g/mol to 2500 g/mol.

4. The composition of any of claims 1-3, wherein the soluble dietary fiber is made by a process comprising:
providing a saccharide feed comprising at least 95 wt% (e.g., at least 97 wt%, at least 98 wt% or at least 99 wt%) on a dry solids basis of dextrose and/or dextrose oligomers;
reacting the saccharide feed in the presence of water and in the substantial absence of sugar alcohols at a total solids concentration of at least about 80% by weight and a temperature of at least about 120 °C with at least one acid catalyst that accelerates the rate of cleavage and formation of glucosyl bonds for a time sufficient to produce a product composition having a fiber content of at least 60% fiber.

5. The composition of any of claims 1-4, wherein the composition is in the form of a functional food or beverage (e.g., a medical food or beverage).

6. An effective amount of one or more strains of *Lactobacillus mucosae* and one or more soluble dietary fibers for use in reducing weight gain in the mammal, treating or preventing cardiovascular disease in the mammal, treating or preventing heart failure in the mammal, reducing left ventricle wall thickness in the mammal, reducing left atrial area in the mammal, reducing systemic inflammation in the mammal, treating or preventing non-alcoholic fatty liver disease (NAFLD/NASH) in the mammal, reducing liver fibrosis in the mammal, reducing fat deposition in the of the mammal, or treating or preventing metabolic syndrome in the mammal, reducing TNF-alpha, increasing FGF19, reducing hsCRP, reducing IL1beta, reducing PAI1, reducing IFN-gamma, reducing macrophage infiltration marker CD163, increasing IL17A, reducing NF-Kb, reducing TLR4, reducing aSMA in a mammal, reducing macrophage infiltration in a tissue of mammal, or increasing the abundance of Parabacteroides in the gut microbiome of a mammal, wherein the soluble dietary fiber has a linkage pattern comprising:
29-45% terminally-linked glucopyranosyl residues;
10-22% 6-linked glucopyranosyl residues;
13-27% 4-linked glucopyranosyl residues;
2-11% 3-linked glucopyranosyl residues;
3-13% 4,6-linked glucopyranosyl residues;
1-5% 3,6-linked glucopyranosyl residues;
0.5-4% 2,4-linked glucopyranosyl residues;
wherein the soluble dietary fiber has a fiber content of at least 70%, and wherein the linkage pattern is determined by the method described in the description; and
wherein one of the one or more strains of *Lactobacillus mucosae* is *L. mucosae* DPC6426.

7. One or more soluble dietary fibers for use in a method of reducing weight gain in the mammal, treating or preventing cardiovascular disease in the mammal, treating or preventing heart failure in the mammal, reducing left ventricle wall thickness in the mammal, reducing left atrial area in the mammal, reducing systemic inflammation in the mammal, treating or preventing non-alcoholic fatty liver disease (NAFLD/NASH) in the mammal, reducing liver fibrosis in the mammal, reducing fat deposition in the of the mammal, treating or preventing metabolic syndrome in the mammal, reducing TNF-alpha, increasing FGF19, reducing hsCRP, reducing IL1beta, reducing PAI1, reducing IFN-gamma, reducing macrophage infiltration marker CD163, increasing IL17A, reducing NF-Kb, reducing TLR4, or reducing aSMA in a mammal, or of reducing macrophage infiltration in a tissue of mammal, the method comprising administering to the mammal effective amounts of one or more strains of *Lactobacillus mucosae* and the one or more soluble dietary fibers, wherein the soluble dietary fiber has a linkage pattern comprising:
29-45% terminally-linked glucopyranosyl residues;
10-22% 6-linked glucopyranosyl residues;
13-27% 4-linked glucopyranosyl residues;
2-11% 3-linked glucopyranosyl residues;
3-13% 4,6-linked glucopyranosyl residues;
1-5% 3,6-linked glucopyranosyl residues;
0.5-4% 2,4-linked glucopyranosyl residues;
wherein the soluble dietary fiber has a fiber content of at least 70%, and wherein the linkage pattern is determined by the method described in the description; and
wherein one of the one or more strains of *Lactobacillus mucosae* is *L. mucosae* DPC6426.

8. One or more soluble dietary fibres for use in a method of increasing the abundance of Parabacteroides in the gut microbiome of a mammal, the method comprising administering to the mammal effective amounts of one or more strains of *Lactobacillus mucosae* and the one or more soluble dietary fibers, wherein the soluble dietary fiber has a linkage pattern comprising:
29-45% terminally-linked glucopyranosyl residues;
10-22% 6-linked glucopyranosyl residues;
13-27% 4-linked glucopyranosyl residues;
2-11% 3-linked glucopyranosyl residues;
3-13% 4,6-linked glucopyranosyl residues;
1-5% 3,6-linked glucopyranosyl residues;
0.5-4% 2,4-linked glucopyranosyl residues;
wherein the soluble dietary fiber has a fiber content of at least 70%, and wherein the linkage pattern is determined by the method described in the description; and
wherein one of the one or more strains of *Lactobacillus mucosae* is *L. mucosae* DPC6426.

9. A non-therapeutic method of reducing weight gain in a mammal, the method comprising administering to the mammal effective amounts of one or more strains of *Lactobacillus mucosae* and one or more soluble dietary fibers, wherein the soluble dietary fiber has a linkage pattern comprising:
29-45% terminally-linked glucopyranosyl residues;
10-22% 6-linked glucopyranosyl residues;
13-27% 4-linked glucopyranosyl residues;
2-11% 3-linked glucopyranosyl residues;
3-13% 4,6-linked glucopyranosyl residues;
1-5% 3,6-linked glucopyranosyl residues;
0.5-4% 2,4-linked glucopyranosyl residues;
wherein the soluble dietary fiber has a fiber content of at least 70%, and wherein the linkage pattern is determined by the method described in the description; and
wherein one of the one or more strains of *Lactobacillus mucosae* is *L. mucosae* DPC6426.

10. The effective amount of one or more strains of *Lactobacillus mucosae* and one or more soluble dietary fibers for use of claim 6, or the one or more soluble dietary fibers for use of claims 7-8, or the method of claim 9,
wherein the one or more strains of *Lactobacillus mucosae* are administered in an amount of 10⁶ to 10¹⁶ cfu/day, and/or
wherein the one or more soluble dietary fibers are administered in an amount of 5 g/day to 50 g/day.

11. The effective amount of one or more strains of *Lactobacillus mucosae* and one or more soluble dietary fibers for use, the one or more soluble dietary fibers for use, or method of any of claims 6-10, wherein the one or more soluble dietary fibers and the one or more strains of *L*. *mucosae* are administered at least once every seven days.

12. The effective amount of one or more strains of *Lactobacillus mucosae* and one or more soluble dietary fibers for use, the one or more soluble dietary fibers for use, or method of any of claims 6-11, wherein the soluble dietary fiber is made by a process comprising:
providing a saccharide feed comprising at least 95 wt% (e.g., at least 97 wt%, at least 98 wt% or at least 99 wt%) on a dry solids basis of dextrose and/or dextrose oligomers;
reacting the saccharide feed in the presence of water and in the substantial absence of sugar alcohols at a total solids concentration of at least about 80% by weight and a temperature of at least about 120 °C with at least one acid catalyst that accelerates the rate of cleavage and formation of glucosyl bonds for a time sufficient to produce a product composition having a fiber content of at least 60% fiber.

## Patentansprüche

1. Zusammensetzung, die einen oder mehrere Stämme von *Lactobacillus mucosae* und eine oder mehrere lösliche Nahrungsfasern umfasst, wobei die lösliche Nahrungsfaser ein Bindungsmuster aufweist, welches umfasst:
29-45 % terminal gebundene Glucopyranosylreste;
10-22 % 6-gebundene Glucopyranosylreste;
13-27 % 4-gebundene Glucopyranosylreste;
2-11 % 3-gebundene Glucopyranosylreste;
3-13 % 4,6-gebundene Glucopyranosylreste;
1-5 % 3,6-gebundene Glucopyranosylreste;
0,5-4 % 2,4-gebundene Glucopyranosylreste;
wobei die lösliche Nahrungsfaser einen Fasergehalt von mindestens 70 % aufweist und wobei das Bindungsmuster durch das in der Beschreibung beschriebene Verfahren bestimmt ist; und
wobei einer des einen oder der mehreren Stämme von *Lactobacillus mucosae L. mucosae* DPC6426 ist.

2. Zusammensetzung nach Anspruch 1, wobei mindestens einer des einen oder der mehreren Stämme des *L. mucosae* ein Exopolysaccharid exprimiert, das Monosaccharidreste von Xylose, Fucose, Mannose, Glucose, Galactose, N-Acetylglucosamin und N-Acetylmannosamin umfasst, wobei ein Verhältnis zwischen der Anzahl von Xylose-, Fucose-, Mannose-, Glucose-, Galactose-, N-Acetylglucosamin- und N-Acetylmannosamin-Monosaccharidresten etwa 1,3:0,5:10,0:4,8:3,3:1,5:0,2 beträgt.

3. Zusammensetzung nach Anspruch 1, wobei der eine oder die mehreren Stämme von L. mucosae in einer Menge von 10⁵ bis 10¹⁵ koloniebildenden Einheiten (KBE)/g vorhanden sind, und/oder wobei die lösliche Nahrungsfaser ein gewichtsmittleres Molekulargewicht im Bereich von 1000 g/mol bis 2500 g/mol aufweist.

4. Zusammensetzung nach einem der Ansprüche 1-3, wobei die lösliche Nahrungsfaser durch einen Prozess hergestellt wird, welcher umfasst:
Bereitstellen eines Saccharidausgangsmaterials, das mindestens 95 Gew.-% (z. B. mindestens 97 Gew.-%, mindestens 98 Gew.-% oder mindestens 99 Gew.-%), auf Trockenfeststoffbasis, Dextrose und/oder Dextrose-Oligomere umfasst;
Umsetzen des Saccharidausgangsmaterials in Gegenwart von Wasser und im Wesentlichen in Abwesenheit von Zuckeralkoholen bei einer Gesamtfeststoffkonzentration von mindestens etwa 80 Gew.-% und einer Temperatur von mindestens etwa 120 °C mit mindestens einem Säurekatalysator, der die Spaltungsrate und Bildung von Glucosylbindungen beschleunigt, über eine ausreichend lange Zeit, um eine Produktzusammensetzung zu erzeugen, die einen Fasergehalt von mindestens 60 % Faser aufweist.

5. Zusammensetzung nach einem der Ansprüche 1-4, wobei die Zusammensetzung in Form eines funktionellen Nahrungsmittels oder Getränks (z. B. eines medizinischen Nahrungsmittels oder Getränks) vorliegt.

6. Wirksame Menge eines oder mehrerer Stämme von *Lactobacillus mucosae* und einer oder mehrerer löslicher Nahrungsfasern zur Verwendung beim Reduzieren von Gewichtszunahme bei dem Säugetier, Behandeln oder Vorbeugen von Herz-Kreislauf-Erkrankungen bei dem Säugetier, Behandeln oder Vorbeugen von Herzinsuffizienz bei dem Säugetier, Reduzieren der Wanddicke des linken Ventrikels bei dem Säugetier, Reduzieren der Fläche des linken Vorhofs bei dem Säugetier, Reduzieren von systemischer Entzündung bei dem Säugetier, Behandeln oder Vorbeugen von nichtalkoholischer Fettlebererkrankung (NAFLD/NASH) bei dem Säugetier, Reduzieren von Leberfibrose bei dem Säugetier, Reduzieren von Fettablagerung in der bei dem Säugetier, oder Behandeln oder Vorbeugen von metabolischem Syndrom bei dem Säugetier, Reduzieren von **TNF-**alpha, Erhöhen von FGF19, Reduzieren von hsCRP, Reduzieren von IL1beta, Reduzieren von PAI1, Reduzieren von IFN-gamma, Reduzieren von Makrophagen-Infiltrationsmarker CD163, Erhöhen von IL17A, Reduzieren von NF-Kb, Reduzieren von TLR4, Reduzieren von aSMA bei einem Säugetier, Reduzieren von Makrophagen-Infiltration in einem Säugetiergewebe, oder Erhöhen der Menge von Parabacteroides im Darmmikrobiom eines Säugetiers, wobei die lösliche Nahrungsfaser ein Bindungsmuster aufweist, welches umfasst:
29-45 % terminal gebundene Glucopyranosylreste;
10-22 % 6-gebundene Glucopyranosylreste;
13-27 % 4-gebundene Glucopyranosylreste;
2-11 % 3-gebundene Glucopyranosylreste;
3-13 % 4,6-gebundene Glucopyranosylreste;
1-5 % 3,6-gebundene Glucopyranosylreste;
0,5-4 % 2,4-gebundene Glucopyranosylreste;
wobei die lösliche Nahrungsfaser einen Fasergehalt von mindestens 70 % aufweist und wobei das Bindungsmuster durch das in der Beschreibung beschriebene Verfahren bestimmt ist; und
wobei einer des einen oder der mehreren Stämme von *Lactobacillus mucosae L. mucosae* DPC6426 ist.

7. Eine oder mehrere lösliche Nahrungsfasern zur Verwendung in einem Verfahren zum Reduzieren von Gewichtszunahme bei dem Säugetier, Behandeln oder Vorbeugen von Herz-Kreislauf-Erkrankungen bei dem Säugetier, Behandeln oder Vorbeugen von Herzinsuffizienz bei dem Säugetier, Reduzieren der Wanddicke des linken Ventrikels bei dem Säugetier, Reduzieren der Fläche des linken Vorhofs bei dem Säugetier, Reduzieren von systemischer Entzündung bei dem Säugetier, Behandeln oder Vorbeugen von nichtalkoholischer Fettlebererkrankung (NAFLD/NASH) bei dem Säugetier, Reduzieren von Leberfibrose bei dem Säugetier, Reduzieren von Fettablagerung in der bei dem Säugetier, Behandeln oder Vorbeugen von metabolischem Syndrom bei dem Säugetier, Reduzieren von TNF-alpha, Erhöhen von FGF19, Reduzieren von hsCRP, Reduzieren von IL1beta, Reduzieren von PAI1, Reduzieren von IFN-gamma, Reduzieren von Makrophagen-Infiltrationsmarker CD163, Erhöhen von IL17A, Reduzieren von NF-Kb, Reduzieren von TLR4, oder Reduzieren von aSMA bei einem Säugetier, oder zum Reduzieren von Makrophagen-Infiltration in einem Säugetiergewebe, wobei das Verfahren das Verabreichen wirksamer Mengen eines oder mehrerer Stämme von *Lactobacillus mucosae* und der einen oder der mehreren löslichen Nahrungsfasern an das Säugetier umfasst, wobei die lösliche Nahrungsfaser ein Bindungsmuster aufweist, welches umfasst:
29-45 % terminal gebundene Glucopyranosylreste;
10-22 % 6-gebundene Glucopyranosylreste;
13-27 % 4-gebundene Glucopyranosylreste;
2-11 % 3-gebundene Glucopyranosylreste;
3-13 % 4,6-gebundene Glucopyranosylreste;
1-5 % 3,6-gebundene Glucopyranosylreste;
0,5-4 % 2,4-gebundene Glucopyranosylreste;
wobei die lösliche Nahrungsfaser einen Fasergehalt von mindestens 70 % aufweist und wobei das Bindungsmuster durch das in der Beschreibung beschriebene Verfahren bestimmt ist; und
wobei einer des einen oder der mehreren Stämme von *Lactobacillus mucosae L. mucosae* DPC6426 ist.

8. Eine oder mehrere lösliche Nahrungsfasern zur Verwendung in einem Verfahren zum Erhöhen der Menge von Parabacteroides im Darmmikrobiom eines Säugetiers, wobei das Verfahren das Verabreichen wirksamer Mengen eines oder mehrerer Stämme von *Lactobacillus mucosae* und der einen oder der mehreren löslichen Nahrungsfasern an das Säugetier umfasst, wobei die lösliche Nahrungsfaser ein Bindungsmuster aufweist, welches umfasst:
29-45 % terminal gebundene Glucopyranosylreste;
10-22 % 6-gebundene Glucopyranosylreste;
13-27 % 4-gebundene Glucopyranosylreste;
2-11 % 3-gebundene Glucopyranosylreste;
3-13 % 4,6-gebundene Glucopyranosylreste;
1-5 % 3,6-gebundene Glucopyranosylreste;
0,5-4 % 2,4-gebundene Glucopyranosylreste;
wobei die lösliche Nahrungsfaser einen Fasergehalt von mindestens 70 % aufweist und wobei das Bindungsmuster durch das in der Beschreibung beschriebene Verfahren bestimmt ist; und
wobei einer des einen oder der mehreren Stämme von *Lactobacillus mucosae L. mucosae* DPC6426 ist.

9. Nicht-therapeutisches Verfahren zum Reduzieren von Gewichtszunahme bei einem Säugetier, wobei das Verfahren das Verabreichen wirksamer Mengen eines oder mehrerer Stämme von *Lactobacillus mucosae* und einer oder mehrerer löslicher Nahrungsfasern an das Säugetier umfasst, wobei die lösliche Nahrungsfaser ein Bindungsmuster aufweist, welches umfasst:
29-45 % terminal gebundene Glucopyranosylreste;
10-22 % 6-gebundene Glucopyranosylreste;
13-27 % 4-gebundene Glucopyranosylreste;
2-11 % 3-gebundene Glucopyranosylreste;
3-13 % 4,6-gebundene Glucopyranosylreste;
1-5 % 3,6-gebundene Glucopyranosylreste;
0,5-4 % 2,4-gebundene Glucopyranosylreste;
wobei die lösliche Nahrungsfaser einen Fasergehalt von mindestens 70 % aufweist und wobei das Bindungsmuster durch das in der Beschreibung beschriebene Verfahren bestimmt ist; und
wobei einer des einen oder der mehreren Stämme von *Lactobacillus mucosae L. mucosae* DPC6426 ist.

10. Wirksame Menge eines oder mehrerer Stämme von *Lactobacillus mucosae* und einer oder mehrerer löslicher Nahrungsfasern zur Verwendung nach Anspruch 6, oder eine oder mehrere lösliche Nahrungsfasern zur Verwendung nach den Ansprüchen 7-8, oder Verfahren nach Anspruch 9,
wobei der eine oder die mehreren Stämme von *Lactobacillus mucosae* in einer Menge von 10⁶ bis 10¹⁶ KBE/Tag verabreicht werden, und/oder
wobei die eine oder die mehreren löslichen Nahrungsfasern in einer Menge von 5 g/Tag bis 50 g/Tag verabreicht werden.

11. Wirksame Menge eines oder mehrerer Stämme von *Lactobacillus mucosae* und einer oder mehrerer löslicher Nahrungsfasern zur Verwendung, eine oder mehrere lösliche Nahrungsfasern zur Verwendung, oder Verfahren nach einem der Ansprüche 6-10, wobei die eine oder die mehreren löslichen Nahrungsfasern und der eine oder die mehreren Stämme von L. mucosae mindestens einmal alle sieben Tage verabreicht werden.

12. Wirksame Menge eines oder mehrerer Stämme von *Lactobacillus mucosae* und einer oder mehrerer löslicher Nahrungsfasern zur Verwendung, eine oder mehrere lösliche Nahrungsfasern zur Verwendung, oder Verfahren nach einem der Ansprüche 6-11, wobei die lösliche Nahrungsfaser durch einen Prozess hergestellt wird, welcher umfasst:
Bereitstellen eines Saccharidausgangsmaterials, das mindestens 95 Gew.-% (z. B. mindestens 97 Gew.-%, mindestens 98 Gew.-% oder mindestens 99 Gew.-%), auf Trockenfeststoffbasis, Dextrose und/oder Dextrose-Oligomere umfasst;
Umsetzen des Saccharidausgangsmaterials in Gegenwart von Wasser und im Wesentlichen in Abwesenheit von Zuckeralkoholen bei einer Gesamtfeststoffkonzentration von mindestens etwa 80 Gew.-% und einer Temperatur von mindestens etwa 120 °C mit mindestens einem Säurekatalysator, der die Spaltungsrate und Bildung von Glucosylbindungen beschleunigt, über eine ausreichend lange Zeit, um eine Produktzusammensetzung zu erzeugen, die einen Fasergehalt von mindestens 60 % Faser aufweist.

## Revendications

1. Composition comprenant une ou plusieurs souches de *Lactobacillus mucosae* et une ou plusieurs fibres alimentaires solubles, dans laquelle la fibre alimentaire soluble présente un motif de liaison comprenant :
29 à 45 % de résidus glucopyranosyle liés en position terminale ;
10 à 22 % de résidus glucopyranosyle liés en 6 ;
13 à 27 % de résidus glucopyranosyle liés en 4 ;
2 à 11% de résidus glucopyranosyle liés en 3 ;
3 à 13 % de résidus glucopyranosyle liés en 4,6 ;
1 à 5 % de résidus glucopyranosyle liés en 3,6 ;
0,5 à 4 % de résidus glucopyranosyle liés en 2,4 ;
dans laquelle la fibre alimentaire soluble présente une teneur en fibres d'au moins 70 %, et dans laquelle le motif de liaison est déterminé par le procédé décrit dans la description ; et
dans laquelle l'une des une ou plusieurs souches de *Lactobacillus mucosae* est *L. mucosae* DPC6426.

2. Composition selon la revendication 1, dans laquelle au moins une des une ou plusieurs souches de *L. mucosae* exprime un exopolysaccharide comprenant des résidus monosaccharides xylose, fucose, mannose, glucose, galactose, N-acétylglucosamine et N-acétylmannosamine, dans laquelle un rapport entre le nombre de résidus monosaccharides xylose, fucose, mannose, glucose, galactose, N-acétylglucosamine et N-acétylmannosamine est d'environ 1,3:0,5:10,0:4,8:3,3:1,5:0,2.

3. Composition selon la revendication 1, dans laquelle les une ou plusieurs souches de L. mucosae sont présentes en une quantité de 10⁵ à 10¹⁵ unités formant colonie (cfu)/g, et/ou dans laquelle la fibre alimentaire soluble présente une masse moléculaire moyenne en poids dans la plage de 1000 g/mole à 2 500 g/mole.

4. Composition selon l'une quelconque des revendications 1-3, dans laquelle la fibre alimentaire soluble est fabriquée par un processus comprenant :
la fourniture d'un aliment saccharidique comprenant au moins 95 % en poids (par exemple, au moins 97 % en poids, au moins 98 % en poids ou au moins 99 % en poids) sur une base de solides secs de dextrose et/ou d'oligomères de dextrose ;
la mise à réagir de l'aliment saccharidique en présence d'eau et en l'absence substantielle d'alcools de sucre à une concentration totale en solides d'au moins environ 80 % en poids et à une température d'au moins environ 120°C avec au moins un catalyseur acide qui accélère la vitesse de clivage et de formation de liaisons glucosyle pendant une durée suffisante pour produire une composition de produit présentant une teneur en fibres d'au moins 60 % de fibres.

5. Composition selon l'une quelconque des revendications 1-4, dans laquelle la composition se présente sous la forme d'un aliment fonctionnel ou d'une boisson fonctionnelle (par exemple un aliment médical ou une boisson médicale).

6. Quantité efficace d'une ou plusieurs souches de *Lactobacillus mucosae* et d'une ou plusieurs fibres alimentaires solubles destinées à être utilisées pour réduire la prise de poids chez le mammifère, traiter ou prévenir les maladies cardiovasculaires chez le mammifère, traiter ou prévenir l'insuffisance cardiaque chez le mammifère, réduire l'épaisseur de la paroi du ventricule gauche chez le mammifère, réduire la zone auriculaire gauche chez le mammifère, réduire l'inflammation systémique chez le mammifère, traiter ou prévenir la stéatose hépatique non alcoolique (NAFLD/NASH) chez le mammifère, réduire la fibrose hépatique chez le mammifère, réduire le dépôt de graisse chez le mammifère, ou traiter ou prévenir le syndrome métabolique chez le mammifère, réduire le TNF-alpha, augmenter le FGF19, réduire la hsCRP, réduire l'IL1bêta, réduire le PAI1, réduire l'IFN-gamma, réduire le marqueur d'infiltration des macrophages CD163, augmenter l'IL17A, réduire le NF-Kb, réduire le TLR4, réduire l'aSMA chez un mammifère, réduire l'infiltration des macrophages dans un tissu de mammifère, ou augmenter l'abondance de Parabacteroides dans le microbiome intestinal d'un mammifère, dans laquelle la fibre alimentaire soluble présente un motif de liaison comprenant :
29 à 45 % de résidus glucopyranosyle liés en position terminale ;
10 à 22 % de résidus glucopyranosyle liés en 6 ;
13 à 27 % de résidus glucopyranosyle liés en 4 ;
2 à 11 % de résidus glucopyranosyle liés en 3 ;
3 à 13 % de résidus glucopyranosyle liés en 4,6 ;
1 à 5 % de résidus glucopyranosyle liés en 3,6 ;
0,5 à 4 % de résidus glucopyranosyle liés en 2,4 ;
dans laquelle la fibre alimentaire soluble présente une teneur en fibres d'au moins 70 %, et dans laquelle le motif de liaison est déterminé par le procédé décrit dans la description ; et
dans laquelle l'une des une ou plusieurs souches de *Lactobacillus mucosae* est *L. mucosae* DPC6426.

7. Une ou plusieurs fibres alimentaires solubles destinées à être utilisées dans un procédé de réduction de la prise de poids chez le mammifère, de traitement ou de prévention des maladies cardiovasculaires chez le mammifère, de traitement ou de prévention de l'insuffisance cardiaque chez le mammifère, de réduction de l'épaisseur de la paroi du ventricule gauche chez le mammifère, de réduction de la zone auriculaire gauche chez le mammifère, de réduction de l'inflammation systémique chez le mammifère, de traitement ou de prévention de la stéatose hépatique non alcoolique (NAFLD/NASH) chez le mammifère, de réduction de la fibrose hépatique chez le mammifère, de réduction du dépôt de graisse chez le mammifère, de traitement ou de prévention du syndrome métabolique chez le mammifère, de réduction du TNF-alpha, d'augmentation du FGF19, de réduction de la hsCRP, de réduction de l'IL1bêta, de réduction du PAI1, de réduction de l'IFN-gamma, de réduction du marqueur d'infiltration des macrophages CD163, d'augmentation de l'IL17A, de réduction du NF-Kb, de réduction du TLR4 ou de réduction de l'aSMA chez un mammifère ou de réduction de l'infiltration des macrophages dans un tissu de mammifère, le procédé comprenant l'administration au mammifère de quantités efficaces d'une ou plusieurs souches de *Lactobacillus mucosae* et des une ou plusieurs fibres alimentaires solubles, dans laquelle la fibre alimentaire soluble présente un motif de liaison comprenant :
29 à 45 % de résidus glucopyranosyle liés en position terminale ;
10 à 22 % de résidus glucopyranosyle liés en 6 ;
13 à 27 % de résidus glucopyranosyle liés en 4 ;
2 à 11 % de résidus glucopyranosyle liés en 3 ;
3 à 13 % de résidus glucopyranosyle liés en 4,6 ;
1 à 5 % de résidus glucopyranosyle liés en 3,6 ;
0,5 à 4 % de résidus glucopyranosyle liés en 2,4 ;
dans laquelle la fibre alimentaire soluble présente une teneur en fibres d'au moins 70 %, et dans laquelle le motif de liaison est déterminé par le procédé décrit dans la description ; et
dans laquelle l'une des une ou plusieurs souches de *Lactobacillus mucosae* est *L. mucosae* DPC6426.

8. Une ou plusieurs fibres alimentaires solubles destinées à être utilisées dans un procédé d'augmentation de l'abondance de Parabacteroides dans le microbiome intestinal d'un mammifère, le procédé comprenant l'administration au mammifère de quantités efficaces d'une ou plusieurs souches de *Lactobacillus mucosae* et des une ou plusieurs fibres alimentaires solubles, dans laquelle la fibre alimentaire soluble présente un motif de liaison comprenant :
29 à 45 % de résidus glucopyranosyle liés en position terminale ;
10 à 22 % de résidus glucopyranosyle liés en 6 ;
13 à 27 % de résidus glucopyranosyle liés en 4 ;
2 à 11 % de résidus glucopyranosyle liés en 3 ;
3 à 13 % de résidus glucopyranosyle liés en 4,6 ;
1 à 5 % de résidus glucopyranosyle liés en 3,6 ;
0,5 à 4 % de résidus glucopyranosyle liés en 2,4 ;
dans laquelle la fibre alimentaire soluble présente une teneur en fibres d'au moins 70 %, et dans laquelle le motif de liaison est déterminé par le procédé décrit dans la description ; et
dans laquelle l'une des une ou plusieurs souches de *Lactobacillus mucosae* est *L. mucosae* DPC6426.

9. Procédé non thérapeutique de réduction de la prise de poids chez un mammifère, le procédé comprenant l'administration au mammifère de quantités efficaces d'une ou plusieurs souches de *Lactobacillus mucosae* et d'une ou plusieurs fibres alimentaires solubles, dans lequel la fibre alimentaire soluble présente un motif de liaison comprenant :
29 à 45 % de résidus glucopyranosyle liés en position terminale ;
10 à 22 % de résidus glucopyranosyle liés en 6 ;
13 à 27 % de résidus glucopyranosyle liés en 4 ;
2 à 11 % de résidus glucopyranosyle liés en 3 ;
3 à 13 % de résidus glucopyranosyle liés en 4,6 ;
1 à 5 % de résidus glucopyranosyle liés en 3,6 ;
0,5 à 4 % de résidus glucopyranosyle liés en 2,4 ;
dans lequel la fibre alimentaire soluble présente une teneur en fibres d'au moins 70 %, et dans lequel le motif de liaison est déterminé par le procédé décrit dans la description ; et
dans lequel l'une des une ou plusieurs souches de *Lactobacillus mucosae* est *L. mucosae* DPC6426.

10. Quantité efficace d'une ou plusieurs souches de *Lactobacillus mucosae* et d'une ou plusieurs fibres alimentaires solubles destinées à être utilisées selon la revendication 6, ou des une ou plusieurs fibres alimentaires solubles destinées à être utilisées selon les revendications 7-8, ou procédé selon la revendication 9,
dans laquelle les une ou plusieurs souches de *Lactobacillus mucosae* sont administrées en une quantité de 10⁶ à 10¹⁶ cfu/jour, et/ou
dans laquelle les une ou plusieurs fibres alimentaires solubles sont administrées en une quantité de 5 g/jour à 50 g/jour.

11. Quantité efficace d'une ou plusieurs souches de *Lactobacillus mucosae* et d'une ou plusieurs fibres alimentaires solubles destinées à être utilisées, les une ou plusieurs fibres alimentaires solubles destinées à être utilisées, ou procédé selon l'une quelconque des revendications 6-10, dans laquelle les une ou plusieurs fibres alimentaires solubles et les une ou plusieurs souches de L. mucosae sont administrées au moins une fois tous les sept jours.

12. Quantité efficace d'une ou plusieurs souches de *Lactobacillus mucosae* et d'une ou plusieurs fibres alimentaires solubles destinées à être utilisées, les une ou plusieurs fibres alimentaires solubles destinées à être utilisées, ou procédé selon l'une quelconque des revendications 6-11, dans laquelle la fibre alimentaire soluble est fabriquée par un processus comprenant :
la fourniture d'un aliment saccharidique comprenant au moins 95 % en poids (par exemple, au moins 97 % en poids, au moins 98 % en poids ou au moins 99 % en poids) sur une base de solides secs de dextrose et/ou d'oligomères de dextrose ;
la mise à réagir de l'aliment saccharidique en présence d'eau et en l'absence substantielle d'alcools de sucre à une concentration totale en solides d'au moins environ 80 % en poids et à une température d'au moins environ 120°C avec au moins un catalyseur acide qui accélère la vitesse de clivage et de formation de liaisons glucosyle pendant une durée suffisante pour produire une composition de produit présentant une teneur en fibres d'au moins 60 % de fibres.
